# EUROPEAN PATENT APPLICATION

(11) **EP 1 932 853 A1**
(43) Date of publication of application: **18.06.2008**
(21) Application number: 07023159.2
(22) Date of filing: 06.08.2002
(51) Int. Cl.: C07K 7/50

(54) **Novel depsipeptides and process for preparing same**

(30) Priority: 06.08.2001 US 310313 P
(62) Divisional of application: 02761255.5
(71) Applicant: Cubist Pharmaceutical Inc., Lexington, MA 02421 (US)
(72) Inventor: Finn, John, Encinitas CA 92024 (US); Morytko, Michael, Framingham MA 01701 (US); Parr, Ian Barrie, Medford 02155 (US); Jung, Michael, Los Angeles CA 90064 (US)
(74) Representative: Vossius & Partner

(57) **Abstract**

The present invention relates to novel depsipeptide compounds. The invention also relates to pharmaceutical compositions of these compounds and methods of using these compounds as antibacterial compounds. The invention also relates to methods of producing these novel depsipeptide compounds and imntermediates used in producing these compounds.

## Description

### CROSS REFERENCE TO RELATED APPLICATIONS

This application claims the benefit of United States Application No. 60/310,313 filed August 6, 2001, which is hereby incorporated by reference in its entirety.

### FIELD OF THE INVENTION

The present invention relates to novel depsipeptides compounds. The invention also relates to pharmaceutical compositions of these compounds and methods of using these compounds as antibacterial agents. The invention also relates to methods of producing these novel depsipeptide compounds and intermediates used in producing these compounds.

### BACKGROUND OF THE INVENTION

The rapid increase in the incidence of gram-positive infections - including those caused by resistant bacteria - has sparked renewed interest in the development of novel classes of antibiotics. A class of compounds that have shown potential as useful antibiotic agents is the cyclic depsipeptides. A notable member of the cyclic depsipeptides is the A-21978C lipopeptides described in, for example, United States Patents RE 32,333; RE 32,455; RE 32,311; RE 32,310; 4,482,487; 4,537,717; and 5,912,226 and International Patent Applications WO01/44272; WO01/44274; and WO01/44271. Additionally, the A54145 class of compounds described in United States Patents 4,994,270; 5,039,789; and 5,028,590 have also been shown to possess antibiotic activity.

Daptomycin, also known as LY 146032, is comprised of an n-decanoyl side chain linked to the N-terminal tryptophan of a three-amino acid chain linked to a cyclic 10-amino acid peptide. Daptomycin has potent bactericidal activity *in vitro* and *in vivo* against clinically relevant gram-positive bacteria that cause serious and life-threatening diseases. These bacteria include resistant pathogens, such as vancomycin-resistant enterococci (VRE), methicillin-resistant *Staphylococcus aureus* (MRSA), glycopeptide intermediate susceptible *Staphylococcus aureus* (GISA), vancomycin-resistant *Staphylococcus aureus* (VRSA), *coagulase*-negative staphylococci (CNS), and penicillin-resistant *Streptococcus pneumoniae* (PRSP), for which there are few therapeutic alternatives. See, e.g., Tally et al., 1999, Exp. Opin. Invest. Drugs 8:1223-1238.

Despite the promise that existing antibacterial agents have shown, the need for novel antibiotics continues. Many pathogens have been repeatedly exposed to commonly used antibiotics. This exposure has led to the selection of variant antibacterial strains resistant to a broad spectrum of antibiotics. The loss of potency and effectiveness of an antibiotic caused by resistant mechanisms renders the antibiotic ineffective and consequently can lead to some life-threatening infections that are virtually untreatable. As new antibiotics come to market pathogens may develop resistance or intermediate resistance to these new drugs, effectively creating a need for a stream of new antibacterial agents to combat these emerging strains. In addition compounds that exhibit bactericidal activity offer advantages over present bacteriostatic compounds. Thus, novel antibacterial agents would be expected to be useful to treat not only "natural" pathogens, but also intermediate drug resistant and drug resistant pathogens because the pathogen has never been exposed to the novel antibacterial agent New antibacterial agents may exhibit differential effectiveness against different types of pathogens.

### SUMMARY OF THE INVENTION

The present invention provides novel compounds that have antibacterial activity against a broad spectrum of bacteria, including drug-resistant bacteria, and processes for making these compounds.

The present invention provides, in one aspect, compounds of Formula I: 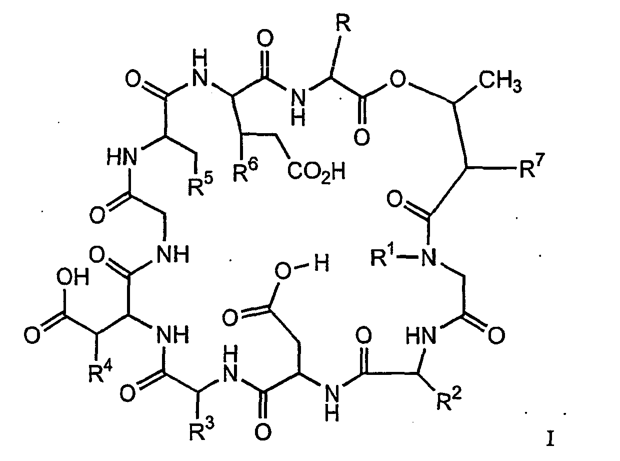 and salts thereof; wherein:
(a) R is 2-butyl, isopropyl or 2-(2'-aminophenacyl);
(b) each R¹ and R⁶ is independently hydrido or methyl;
(c) R² is methyl or -CH₂CH₂CH₂R⁸ ;
(d) R³ is methyl or -CH₂CH₂CH₂CH₂R⁹ ;
(e) R⁴ is hydrido or methoxy;
(f) R⁵ is hydroxy or carboxyamino;
(g) each of R⁷, R⁸ and R⁹ is independently amino, monosubstituted amino, disubstituted amino, acylamino, ureido, guanidino, carbamoyl, sulfonamino, thioacylamino, thioureido, iminoamino, or phosphonamino;
(h) provided that
   (1) when R² is -CH₂CH₂CH₂R⁸, R⁷ is other than 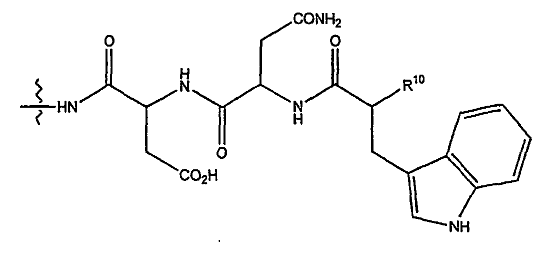 wherein R¹⁰ is amino, monosubstituted amino, disubstituted amino, acylamino, ureido, guanidino, carbamoyl, sulfonamino, thioacylamino, iminoamino, or phosphonamino;
   (2) when R² is methyl, R⁷ is other than 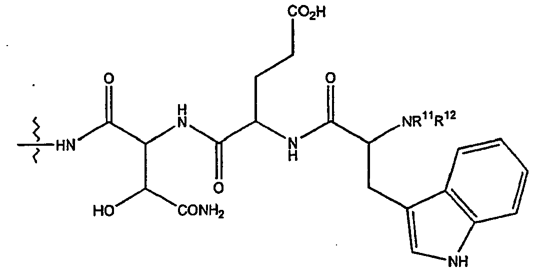
wherein each of R¹¹ and R¹² is hydrido, C₆-C₁₈ unsubstituted alkanoyl, C₈-C₁₈ unsubstituted alkenoyl, C₈-C₁₈ unsubstituted alkyl, or C₈-C₁₈ select substituted alkyl; or alternatively, R¹¹ and R¹² together are C₈-C₁₈ alkylidenyl.

In another aspect, the present invention also provides pharmaceutical compositions including compounds of Formula I and methods of use thereof.

In a further aspect the present invention provides a process for preparing the compounds of Formula I.

In yet a further aspect, the invention provides compounds useful as intermediates for the preparation of the compounds of Fomula I.

### DETAILED DESCRIPTION OF THE INVENTION

### Definitions

The term "activating group" denotes a group that when adjacent to a carbonyl group activates the carbonyl group to attack by a nucleophilic amine, resulting in the loss of the activating group and the formation of an amide bond. Examples of activating groups are aryloxy, acyloxy, imidazolyl

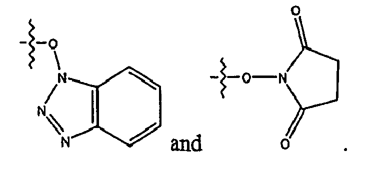

Preferred activating groups are aryloxy groups. The most preferred activating group is pentafluorophenoxy.

The term "acyl" denotes a carbonyl radical attached to an alkyl, alkenyl, alkynyl, cycloalkyl, heterocycyl, aryl or heteroaryl group, examples including, without limitation, such radicals as acetyl and benzoyl. Subsets of the term acyl are (1) "unsubstituted alkanoyl" which is defined as carbonyl radical attached to an unsubstituted alkyl group and (2) "unsubstituted alkenoyl" which is defined as carbonyl radical attached to an unsubsituted alkenyl group.

The term "acylamino" is defined as a nitrogen radical adjacent to an acyl group.

The term "acyloxy" denotes an oxygen radical adjacent to an acyl group.

The term "alkenyl" is defined as linear or branched radicals having two to about twenty carbon atoms, preferably three to about ten carbon atoms, and containing at least one carbon-carbon double bond. One or more hydrogen atoms can also be replaced by a substituent group selected from acyl, acylamino, acyloxy, alkenyl, alkoxy, alkyl, alkynyl, amino, aryl, aryloxy, carbamoyl, carboalkoxy, carboxy, carboxyamido, carboxyamino, cyano, disubstituted amino, formyl, guanidino, halo, heteroaryl, heterocyclyl, hydroxy, iminoamino, monosubstituted amino, nitro, oxo, phosphonamino, sulfinyl, sulfonamino, sulfonyl, thio, thioacylamino, thioureido, or ureido. The double bond portion(s) of the unsaturated hydrocarbon chain may be either in the cis or trans configuration. Examples of alkenyl groups include, without limitation, ethylenyl or phenyl ethylenyl. A subset of term alkenyl is "unsubstituted alkenyl" which is defined as an alkenyl group that bears no substituent groups.

The term "alkoxy" denotes oxygen radical substituted with an alkyl, cycloalkyl or heterocyclyl group. Examples include, without limitation, methoxy, *tert*-butoxy, benzyloxy and cyclohexyloxy.

The term "alkyl" is defined as a linear or branched, saturated radical having one to about twenty carbon atoms unless otherwise specified. The term "lower alkyl" is defined as an alkyl group containing 1-4 carbon atoms. One or more hydrogen atoms can also be replaced by a substitutent group selected from acyl, acylamino, acyloxy, alkenyl, alkoxy, alkyl, alkynyl, amino, aryl, aryloxy, carbamoyl, carboalkoxy, carboxy, carboxyamido, carboxyamino, cyano, disubstituted amino, formyl, guanidino, halo, heteroaryl, heterocyclyl, hydroxy, iminoamino, monosubstituted amino, nitro, oxo, phosphonamino, sulfinyl, sulfonamino, sulfonyl, thio, thioacylamino, thioureido, or ureido. Examples of alkyl groups include, without limitation, methyl, butyl, *tert*-butyl, isopropyl, trifluoromethyl, nonyl, undecyl, octyl, dodecyl, methoxymethyl, 2-(2'-aminophenacyl), 3-indolyhnethyl, benzyl, and carboxymethyl. Subsets of the term alkyl are (1) "unsubstituted alkyl" which is defined as an alkyl group that bears no substituent groups (2) "substituted alkyl" which denotes an alkyl radical in which one or more hydrogen atoms is replaced by a substitutent group selected from acyl, acylamino, acyloxy, alkenyl, alkoxy, alkyl, alkynyl, amino, aryl, aryloxy, carbamoyl, carboalkoxy, carboxy, carboxyamido, carboxyamino, cyano, disubstituted amino, formyl, guanidino, halo, heteroaryl, heterocyclyl, hydroxy, iminoamino, monosubstituted amino, nitro, oxo, phosphonamino, sulfinyl, sulfonamino, sulfonyl, thio, thioacylamino, thioureido, or ureido and (3) "selected substituted alkyl" which denotes an alkyl radical in which (a) one proton is replaced by a group selected from hydroxy, carboxy, C₁-C₈ alkoxy, or (b) one to three protons is replaced by a halo substituent

The term "alkylidenyl" is defined as a carbon radical of the formula 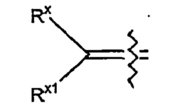 wherein R^{x} and R^{x1} are independently selected from hydrido or C₇-C₁₇ unsubstituted alkyl, wherein the total number of carbons from R^{x} and R^{x1} does not exceed 17.

The term "alkynyl" denotes linear or branched radicals having from two to about ten carbon atoms, and containing at least one carbon-carbon triple bond. One or more hydrogen atoms can also be replaced by a substituent group selected from acyl, acylamino, acyloxy, alkenyl, alkoxy, alkyl, alkynyl, amino, aryl, aryloxy, carbamoyl, carboalkoxy, carboxy, carboxyamido, carboxyamino, cyano, disubstituted amino, formyl, guanidino, halo, heteroaryl, heterocyclyl, hydroxy, iminoamino, monosubstituted amino, nitro, oxo, phosphonamino, sulfinyl, sulfonamino, sulfonyl, thio, thioacylamino, thioureido, or ureido. An example of alkynyl group includes, without limitation, propynyl.

The term "amino" is defined as an NH₂ radical.

The term "amino acid residue" denotes a compound of the formula 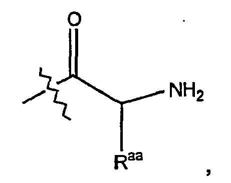 wherein R^{aa} is an amino acid side chain.

The term "amino acid side chain" denotes any side chain (R group) from a naturally-occurring or synthetic amino acid. For example, 3-indolylmethyl could also be called a tryptophan side chain.

The term "2-(2'-aminophenacyl)" refers to a radical of the formula

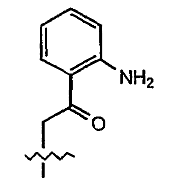

The term "amino protecting group" refers to any chemical compound that may be used to prevent an amino group on a molecule from undergoing a chemical reaction while chemical change occurs elsewhere in the molecule. Numerous amino protecting groups are known to those skilled in the art and examples can be found in "Protective Groups in Organic Synthesis" by Theodora W. Greene, John Wiley and Sons, New York, 1981. Examples of amino protecting groups include phthalimido, trichloroacetyl, STA-base, benzyloxycarbonyl, t-butoxycarbonyl, t-amyloxycarbonyl, isobornyloxycarbonyl, adamantyloxycarbonyl, chlorobenzyloxycarbonyl, nitrobenzyloxycarbonyl or the like. Preferred amino protecting groups are "carbamate amino protecting groups" which are defined as an amino protecting group that when bound to an amino group forms a carbamate. Preferred amino carbamate protecting groups are allyloxycarbonyl (alloc), carbobenzyloxy (CBZ), and *tert*-butoxycarbonyl protecting groups.

The term "aryl" or "aryl ring" is defined as an aromatic radical in a single or fused carbocyclic ring system, having from five to fourteen ring members. In a preferred embodiment, the ring system has from six to ten ring members. One or more hydrogen atoms may also be replaced by a substituent group selected from acyl, acylamino, acyloxy, alkenyl, alkoxy, alkyl, alkynyl, amino, aryl, aryloxy, azido, carbamoyl, carboalkoxy, carboxy, carboxyamido, carboxyamino, cyano, disubstituted amino, formyl, guanidino, halo, heteroaryl, heterocyclyl, hydroxy, iminoamino, monosubstituted amino, nitro, oxo, phosphonamino, sulfinyl, sulfonamino, sulfonyl, thio, thioacylamino, thioureido, or ureido. Examples of aryl groups include, without limitation, phenyl, naphthyl, biphenyl, terphenyl.

The term "aryloxy" denotes oxy-containing radicals substituted with an aryl or heteroaryl group. Examples include, without limitation, phenoxy.

The term "carbamoyl" denotes a nitrogen radical of the formula 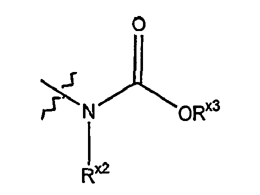 wherein R^{x2} is selected from hydrido, alkyl, aryl, cycloalkyl, heteroaryl or heterocyclyl and R^{x3} is selected from alkyl, aryl, cycloalkyl, heteroaryl or heterocyclyl

The term "carboalkoxy" is defined as a carbonyl radical adjacent to an alkoxy or aryloxy group.

The term "carboxy" denotes a COOH radical.

The term "carboxyamino" denotes a CONH₂ radical.

The term "carboxyamido" is defined as a carbonyl radical adjacent to a monosubstituted amino or disubstituted amino group.

The term "α-carboxy amino acid side chain" is defined as a carbon radical of the formula 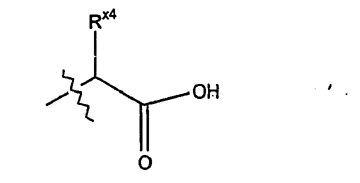 wherein R^{x4} is defined as an amino acid side chain.

The term "carboxymethyl" denotes a CH₂CO₂H radical.

The term "cycloalkyl" or "cycloalkyl ring" denotes a saturated or partially unsaturated carbocyclic ring in a single or fused carbocyclic ring system having from three to twelve ring members. In a preferred embodiment, a cycloalkyl is a ring system having three to seven ring members. One or more hydrogen atoms may also be replaced by a substituent group selected from acyl, acylamino, acyloxy, alkenyl, alkoxy, alkyl, alkynyl, amino, aryl, aryloxy, carbamoyl, carboalkoxy, carboxy, carboxyamido, carboxyamino, cyano, disubstituted amino, formyl, guanidino, halo, heteroaryl, heterocyclyl, hydroxy, iminoamino, monosubstituted amino, nitro, oxo, phosphonamino, sulfinyl, sulfonamino, sulfonyl, thio, thioacylamino, thioureido, or ureido. Examples of a cycloalkyl group include, without limitation, cyclopropyl, cyclobutyl, cyclohexyl, and cycloheptyl.

The term "disubstituted amino" is defined as a nitrogen radical containing two substituent groups independently selected from, alkyl, cycloalkyl, heterocyclyl, aryl, or heteroaryl. Preferred disubstituted amino radicals are "lower disubstituted amino" radicals, whereby the substituent groups are lower alkyl. Also preferred disubstituted amino radicals are amino radicals wherein one substituent is a lower alkyl group and the other substituent is an α-carboxy amino acid side chain.

The group "Fmoc" is a 9-fluorenylmethoxycarbonyl group.

The term "guanidino" is defined as a nitrogen radical of the Formula 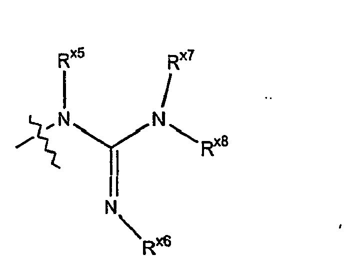 wherein each of R^{x5}, R^{x7} and R^{x8} is independently selected from hydrido, alkyl, aryl, cycloalkyl, heteroaryl or heterocyclyl group; and R^{x6} is selected from alkyl, aryl, cycloalkyl, heteroaryl or heterocyclyl group.

The term "halo" denotes a bromo, chloro, fluoro or iodo radical.

"Heteroaryl" or "heteroaryl ring" is defined as an aromatic radical which contain one to four hetero atoms or hetero groups selected from O, N, S, or SO in a single or fused heterocyclic ring system, having from five to fifteen ring members. In a preferred embodiment, the heteroaryl ring system has from six to ten ring members. One or more hydrogen atoms may also be replaced by a substituent group selected from acyl, acylamino, acyloxy, alkenyl, alkoxy, alkyl, alkynyl, amino, aryl, aryloxy, carbamoyl, carboalkoxy, carboxy, carboxyamido, carboxyamino, cyano, disubstituted amino, formyl, guanidino, halo, heteroaryl, heterocyclyl, hydroxy, iminoamino, monosubstituted amino, nitro, oxo, phosphonamino, sulfinyl, sulfonamino, sulfonyl, thio, thioacylamino, thioureido, or ureido. Examples of heteroaryl groups include, without limitation, pyridinyl, thiazolyl, thiadiazoyl, isoquinolinyl, pyrazolyl, oxazolyl, oxadiazoyl, triazolyl, and pyrrolyl groups.

The term "heterocyclyl," "heterocyclic" or "heterocyclyl ring" denotes a saturated or partially unsaturated ring containing one to four hetero atoms or hetero groups selected from O, N, NH, N(lower alkyl), S, SO or SO₂, in a single or fused heterocyclic ring system having from three to twelve ring members. In a preferred embodiment, a heterocyclyl is a ring system having three to seven ring members. One or more hydrogen atoms may also be replaced by a substituent group selected from acyl, acylamino, acyloxy, alkenyl, alkoxy, alkyl, alkynyl, amino, aryl, aryloxy, carbamoyl, carboalkoxy, carboxy, carboxyamido, carboxyamino, cyano, disubstituted amino, formyl, guanidino, halo, heteroaryl, heterocyclyl, hydroxy, iminoamino, monosubstituted amino, nitro, oxo, phosphonamino, sulfinyl, sulfonamino, sulfonyl, thio, thioacylamino, thioureido, or ureido. Examples of a heterocyclyl group include, without limitation, morpholinyl, piperidinyl, and pyrrolidinyl.

The term "hydrido" is defined as a single hydrogen atom (H).

The term "iminoamino" denotes a nitrogen radical of the formula: 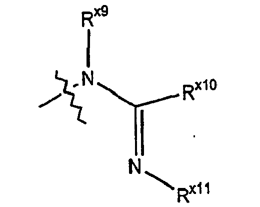 wherein each of R^{x9} and R^{x11} is independently selected from a hydrido, alkyl, cycloalkyl, aryl, heteroaryl or heterocyclyl group; and R^{x10} is selected from an alkyl, cycloalkyl, aryl, heteroaryl or heterocyclyl group.

The term "modifying agent" is defined as (a) a nucleophilic acceptor or (b) an aldehyde or ketone that reacts with an amine under reductive conditions to form an alkylated amine.

The term "monosubstituted amino" denotes a nitrogen radical containing a hydrido group and a substituent group selected from alkyl, cycloalkyl, heterocyclyl, aryl, or heteroaryl. Preferred monosubstituted amino radicals are "lower monosubstituted amino" radicals, whereby the substituent group is a lower alkyl group. More preferred monosubstituted amino radicals are amino radicals containing an α-carboxy amino acid side chain.

The term "nucleophilic acceptor" is defined as a compound that is susceptible to nucleophilic attack by a primary or secondary amine. Examples of nucleophilic acceptors include, without limitation, isocyanates, isothiocyanates, activated esters, acid chlorides, sulfonyl chlorides, activated sulfonamides, activated heterocycles, activated heteroaryls, chloroformates, cyanoformates, thioesters, phosphoryl chlorides, phosphoramidates, imidates, and lactones.

The term "phosphonamino" is defined as a nitrogen radical of the Formula: 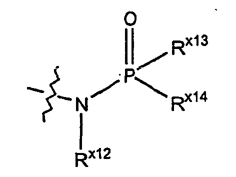 wherein R^{x12} is selected from hydrido, alkyl, aryl, cycloalkyl, heteroaryl or heterocyclyl; wherein each of R^{x13} and R^{x14} is independently selected from alkyl, alkoxy, aryl, aryloxy, cycloalkyl, heteroaryl and heterocyclyl.

The term "sulfinyl" denotes a tetravalent sulfur radical substituted with an oxo substituent and a second substituent selected from the group consisting of alkyl, cycloalkyl, heterocyclyl, aryl, or heteroaryl group.

The term sulfonamino is defined as an amino radical of the formula: 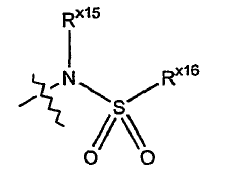 wherein R^{x15} is selected from a hydrido, alkyl, cycloalkyl, aryl, heteroaryl or heterocyclyl group; and R^{x16} is selected from alkyl, cycloalkyl, aryl, heteroaryl or heterocyclyl group.

The term "sulfonyl" denotes a hexavalent sulfur radical substituted with two oxo substituents and a third substituent selected from alkyl, cycloalkyl, heterocyclyl aryl, or heteroaryl.

The term "thio" is defined as a radical containing a substituent group independently selected from hydrido, alkyl, cycloalkyl, heterocyclyl, aryl and heteroaryl, attached to a divalent sulfur atom, such as, methylthio and phenylthio.

The term "thioacylamino" denotes an amino radical of the formula 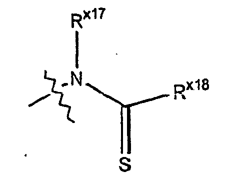 wherein R^{x17} is selected from a hydrido, alkyl, aryl, cycloalkyl, heteroaryl or heterocyclyl group; and wherein R^{x18} is selected from an alkyl, aryl, cycloalkyl, heteroaryl or heterocyclyl group.

The term "thioureido" is defined as a sulfur radical of the formula 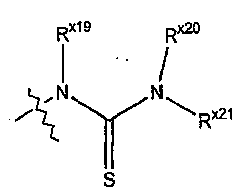 wherein each of R^{x19} and R^{x20} is independently selected from hydrido, alkyl, aryl, cycloalkyl, heteroaryl or heterocyclyl group; and R^{x21} is selected from an alkyl, aryl, cycloalkyl, heteroaryl or heterocyclyl group.

The group trityl is a triphenylmethyl group.

The term "ureido" is defined as a nitrogen radical of the formula 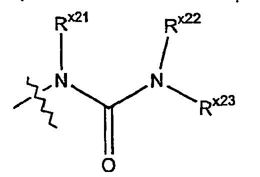 wherein each of R^{x21} and R^{x22} is independently selected from hydrido, alkyl, aryl, cycloalkyl, heteroaryl or heterocyclyl group; and R^{x23} is selected from an alkyl, aryl, cycloalkyl, heteroaryl or heterocyclyl group.

The salts of the compounds of the invention include acid addition salts and base addition salts. In a preferred embodiment, the salt is a pharmaceutically acceptable salt of the compound of Formula I. The term "pharmaceutically acceptable salts" embraces salts commonly used to form alkali metal salts and to form addition salts of free acids or free bases. The nature of the salt is not critical, provided that it is pharmaceutically-acceptable. Suitable pharmaceutically acceptable acid addition salts of the compounds of the invention may be prepared from an inorganic acid or an organic acid. Examples of such inorganic acids include, without limitation, hydrochloric, hydrobromic, hydroiodic, nitric, carbonic, sulfuric and phosphoric acid. Appropriate organic acids may be selected from aliphatic, cycloaliphatic, aromatic, arylaliphatic, heterocyclic, carboxylic and sulfonic classes of organic acids, examples of which include, without limitation, formic, acetic, propionic, succinic, glycolic, gluconic, maleic, embonic (pamoic), methanesulfonic, ethanesulfonic, 2-hydroxyethanesulfonic, pantothenic, benzenesulfonic, toluenesulfonic, sulfanilic, mesylic, cyclohexylaminosulfonic, stearic, algenic, β-hydroxybutyric, malonic, galactic, and galacturonic acid. Suitable pharmaceutically-acceptable base addition salts of compounds of the invention include, but are not limited to, metallic salts made from aluminum, calcium, lithium, magnesium, potassium, sodium and zinc or organic salts made from N,N'-dibenzylethylenediamine, chloroprocaine, choline, diethanolamine, ethylenediamine, N-methylglucamine, lysine and procaine. All of these salts may be prepared by conventional means from the corresponding compound of the invention by treating, for example, the compound of the invention with the appropriate acid or base.

The compounds of the invention can possess one or more asymmetric carbon atoms and are thus capable of existing in the form of optical isomers as well as in the form of racemic or non-racemic mixtures thereof. The compounds of the invention can be utilized in the present invention as a single isomer or as a mixture of stereochemical isomeric forms. Diastereoisomers, i.e., nonsuperimposable stereochemical isomers, can be separated by conventional means such as chromatography, distillation, crystallization or sublimation. The optical isomers can be obtained by resolution of the racemic mixtures according to conventional processes, for example by formation of diastereoisomeric salts by treatment with an optically active acid or base. Examples of appropriate acids include, without limitation, tartaric, diacetyltartaric, dibenzoyltartaric, ditoluoyltartaric and camphorsulfonic acid. The mixture of diastereomers can be separated by crystallization followed by liberation of the optically active bases from the optically active salts. An alternative process for separation of optical isomers includes the use of a chiral chromatography column optimally chosen to maximize the separation of the enantiomers. Still another method involves synthesis of covalent diastereoisomeric molecules by reacting compounds of the invention with an optically pure acid in an activated form or an optically pure isocyanate. The synthesized diastereoisomers can be separated by conventional means such as chromatography, distillation, crystallization or sublimation, and then hydrolyzed to obtain the enantiomerically pure compound. The optically active compounds of the invention can likewise be obtained by utilizing optically active starting materials. These isomers may be in the form of a free acid, a free base, an ester or a salt

The invention also embraces isolated compounds. An isolated compound refers to a compound which represents at least 10%, preferably at least 20%, more preferably at least 50% and most preferably at least 80% of the compound present in the mixture. In a preferred embodiment, the compound, a pharmaceutically acceptable salt thereof or a pharmaceutical composition comprising the compound exhibits a detectable (i.e. statistically significant) antimicrobial activity when tested in conventional biological assays such as those described herein.

### Depsipeptide Compounds

In one aspect, the invention provides compounds of Formula I 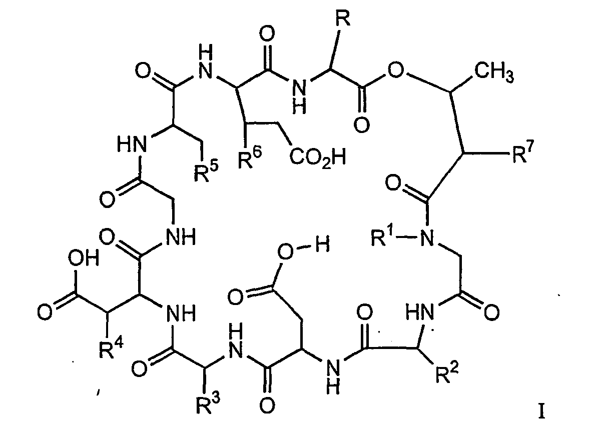 and salts thereof; wherein:
(a) R is 2-butyl, isopropyl or 2-(2'-aminophenacyl);
(b) each R¹ and R⁶ is independently hydrido or methyl;
(c) R² is methyl or -CH₂CH₂CH₂R⁸;
(d) R³ is methyl or -CH₂CH₂CH₂CH₂R⁹;
(e) R⁴ is hydrido or methoxy;
(f) R⁵ is hydroxy or carboxyamino;
(g) each of R⁷, R⁸ and R⁹ is independently amino, monosubstituted amino, disubstituted amino, acylamino, ureido, guanidino, carbamoyl, sulfonamino, thioacylamino, thioureido, iminoamino, or phosphonamino ;
(h) provided that
   (1) when R² is -CH₂CH₂CH₂R⁸, R⁷ is other than 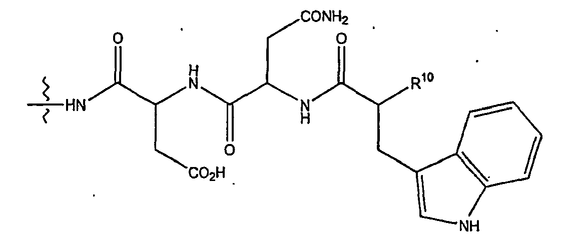 wherein R¹⁰ is amino, monosubstituted amino, disubstituted amino, acylamino, ureido, guanidino, carbamoyl, sulfonamino, thioacylamino, thioureido, iminoamino, and phosphonamino;
   (2) when R² is methyl, R⁷ is other than 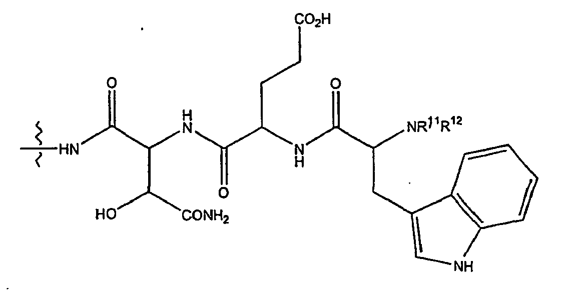
wherein each of R¹¹ and R¹² is hydrido, C₆-C₁₈ unsubstituted alkanoyl, C₈-C₁₈ unsubstituted alkenoyl, C₈-C₁₈ unsubstituted alkyl, or C₈-C₁₈ select substituted alkyl; or alternatively, R¹¹ and R¹² together are C₈-C₁₈ alkylidenyl.

Preferably R⁷ is 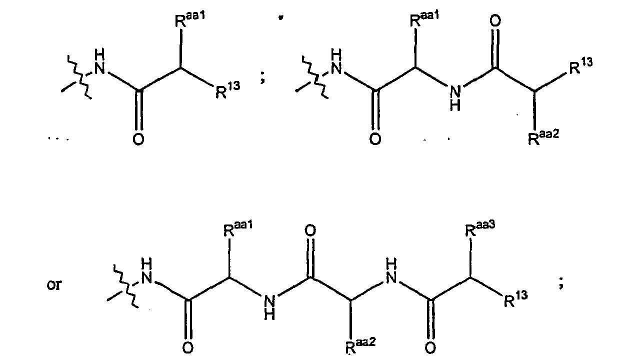 wherein each of R^{aa}, R^{aa2} and R^{aa3} is independently an amino acid side chain and wherein R¹³ is amino, monosubstituted amino, disubstituted amino, acylamino, ureido, guanidino, carbamoyl, sulfonamino, thioacylamino, thioureido, iminoamino, or phosphonamino.

In one embodiment of the invention, R is 2-(2'-aminophenacyl); each of R¹ and R⁴ is hydrido; R² is -CH₂CH₂CH₂R⁸ ; each of R³ and R⁶ is methyl; and R⁵ is hydroxyl. This embodiment provides a compound of Formula II.

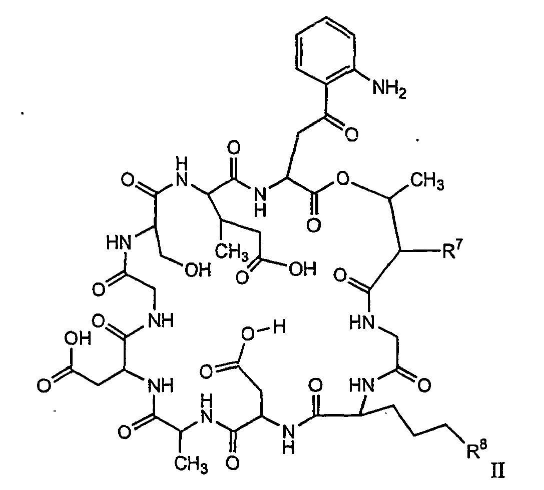

In another embodiment of the invention, R is isopropyl or 2-butyl; each of R¹ and R² is methyl; R³ is-CH₂CH₂CH₂CH₂R⁹; R⁴ is methoxy, and R⁵ is carboxyamino. This embodiment gives a compound of Formula III. 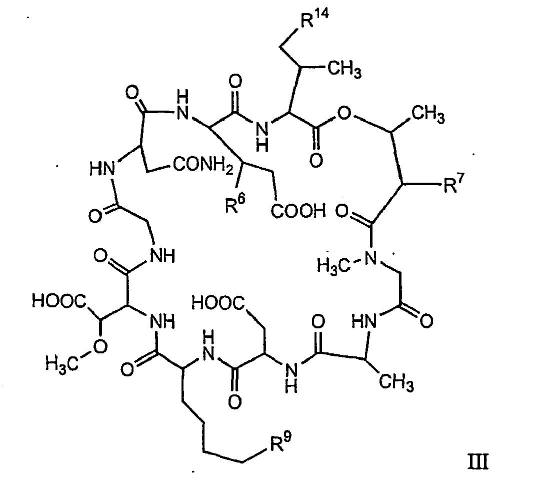 wherein R¹⁴ is hydrido or methyl.

Table I provides exemplary compounds of Formula II.

**Table I**

| *Compounds of Formula II* | |
|---|---|
| R⁷ | R⁸ |
| 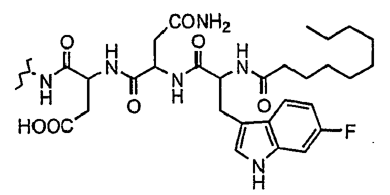 | NH₂ |
| 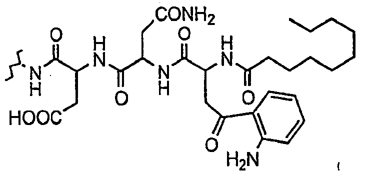 | NH₂ |
| 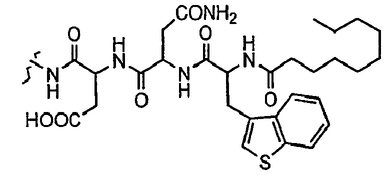 | NH₂ |
| 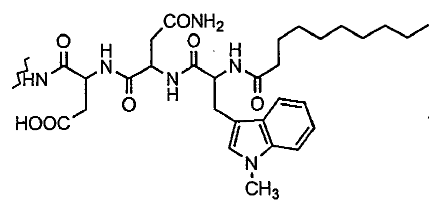 | NH₂ |
| 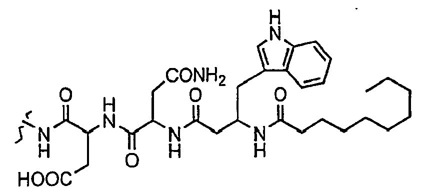 | NH₂ |
| 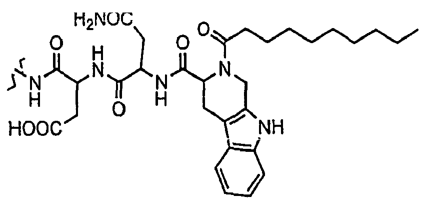 | NH₂ |
| 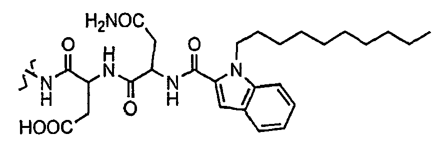 | NH₂ |
| 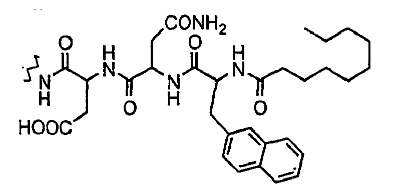 | NH₂ |
| 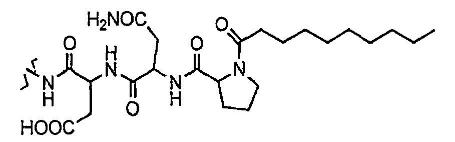 | NH₂ |
| 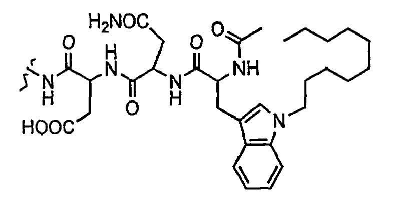 | NH₂ |
| 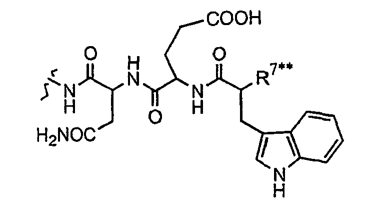 | NH₂ |
| 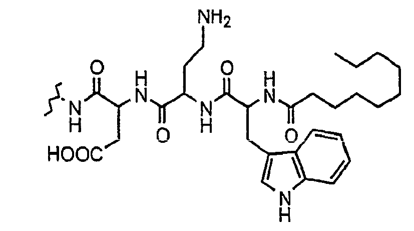 | NH₂ |
| 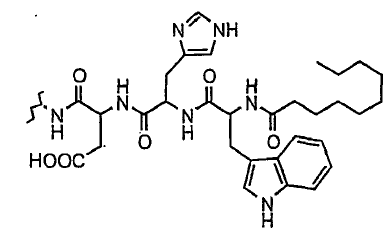 | NH₂ |
| 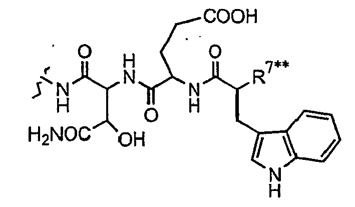 | NH₂ |
| 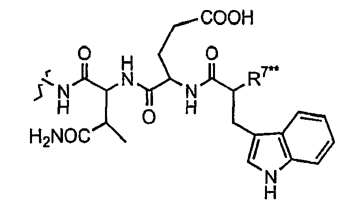 | NH₂ |
| 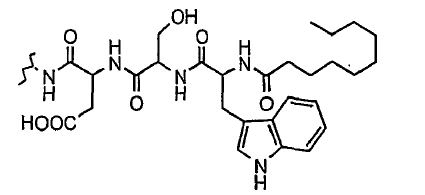 | NH₂ |
| 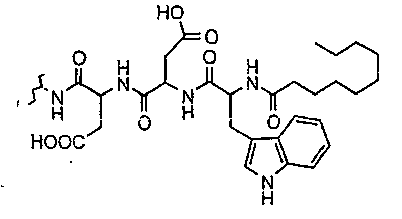 | NH₂ |
| 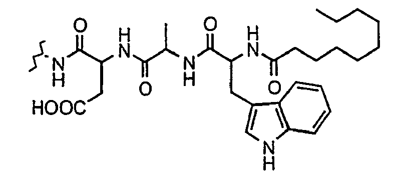 | NH₂ |
| 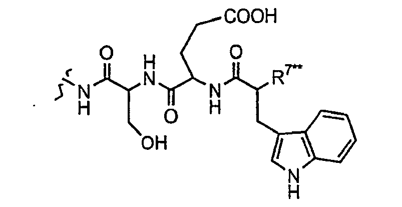 | NH₂ |
| 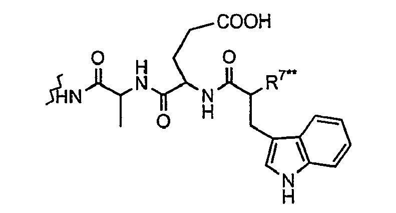 | NH₂ |
| 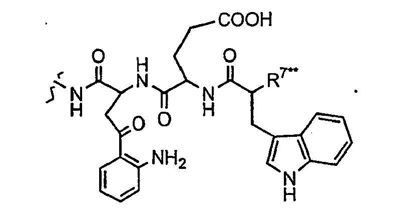 | NH₂ |
| 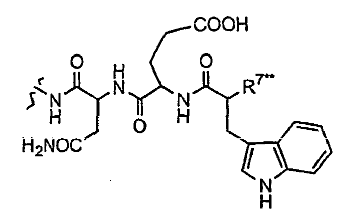 | NH₂ |
| 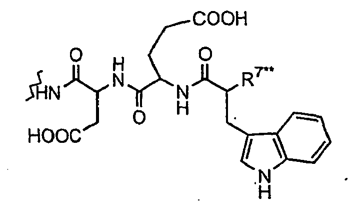 | NH₂ |
| 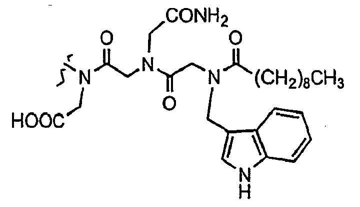 | NH₂ |
| 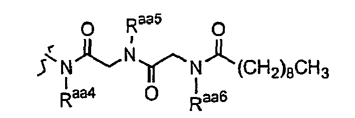 | NH₂ |

Wherein R^{7**} is an amino, monosubstituted amino, disubstituted amino, acylamino, ureido, guanidino, carbamoyl, sulfonamino, thioacylamino, thioureido, iminoamino, or phosphonamino and each of R^{aa4}, R^{aa5}, and R^{aa6} is independently an amino acid side chain.

Table II provides exemplary compounds of Formula III.

**Table II**

| *Compounds of Formula III* | |
|---|---|
| R⁷ | R⁹ |
| 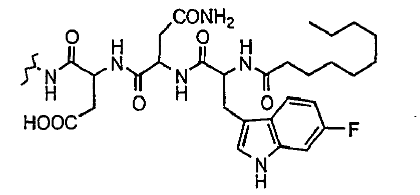 | NH₂ |
| 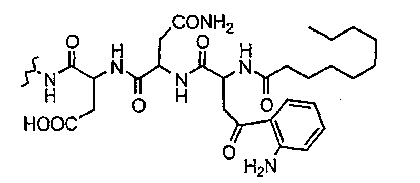 | NH₂ |
| 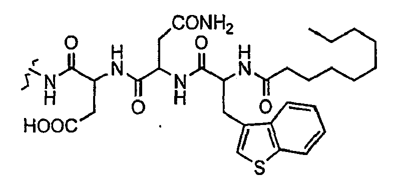 | NH₂ |
| 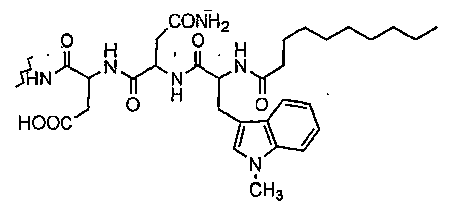 | NH₂ |
| 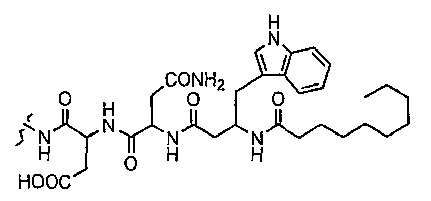 | NH₂ |
| 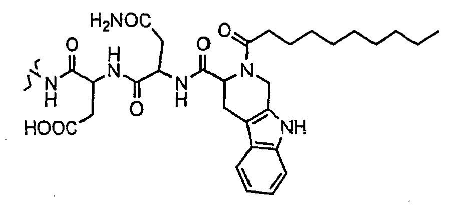 | NH₂ |
| 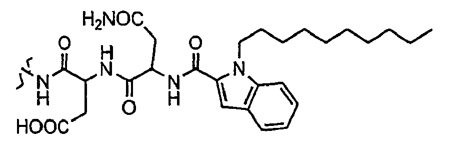 | NH₂ |
| 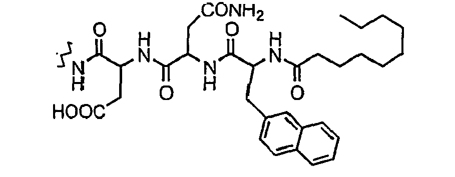 | NH₂ |
| 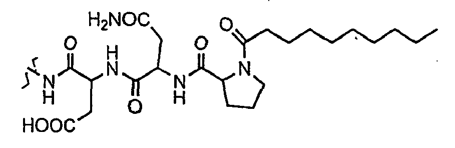 | NH₂ |
| 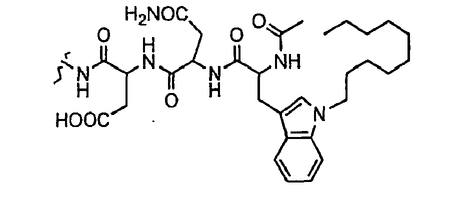 | NH₂ |
| 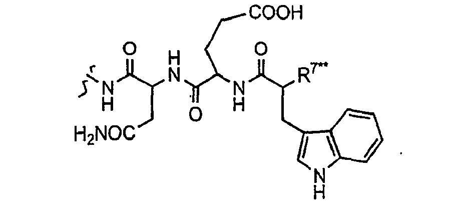 | NH₂ |
| 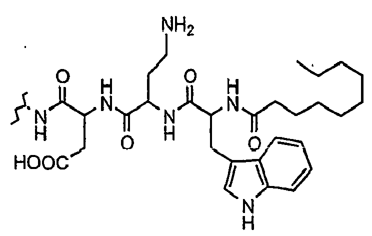 | NH₂ |
| 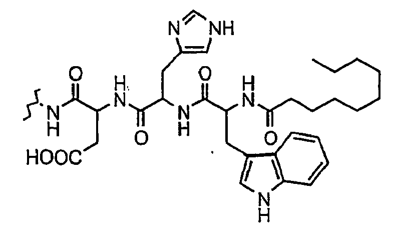 | NH₂ |
| 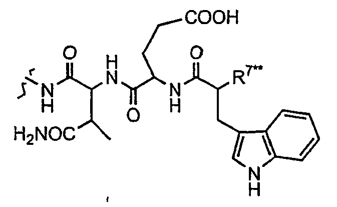 | NH₂ |
| 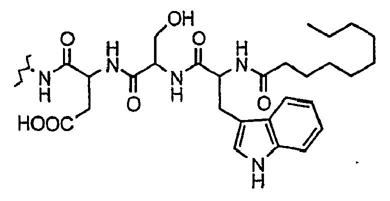 | NH₂ |
| 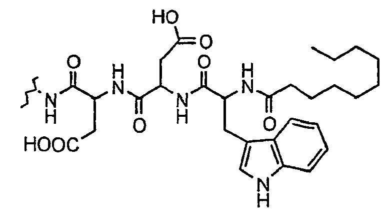 | NH₂ |
| 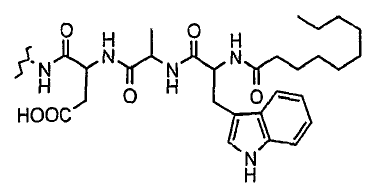 | NH₂ |
| 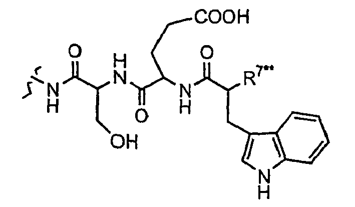 | NH₂ |
| 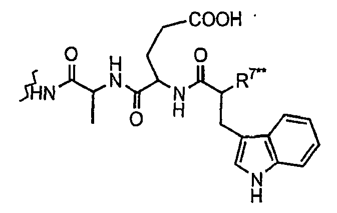 | NH₂ |
| 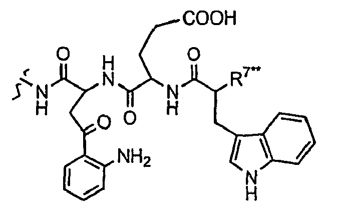 | NH₂ |
| 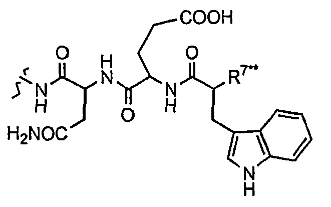 | NH₂ |
| 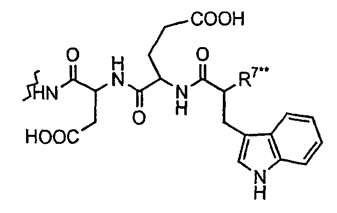 | NH₂ |
| 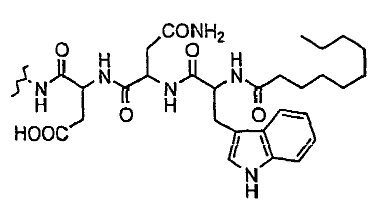 | NH₂ |
| 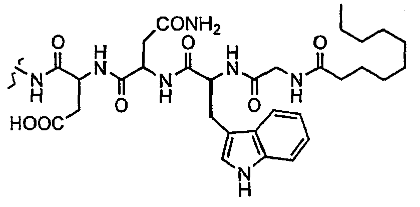 | NH₂ |
| 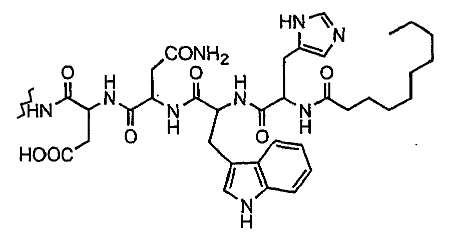 | NH₂ |
| 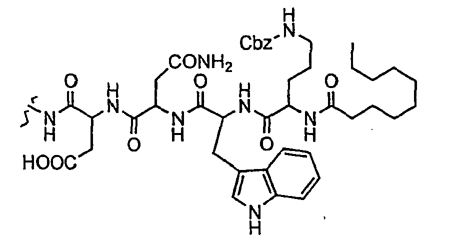 | NH₂ |
| 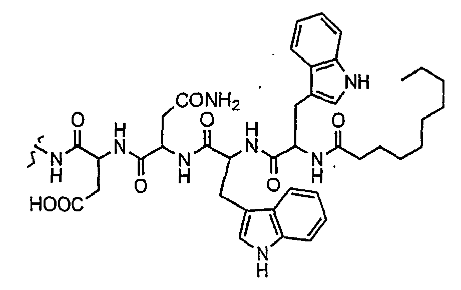 | NH₂ |
| 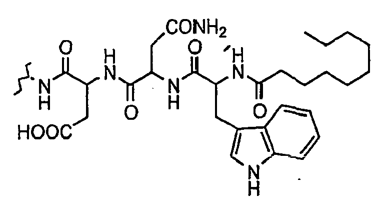 | NH₂ |
| 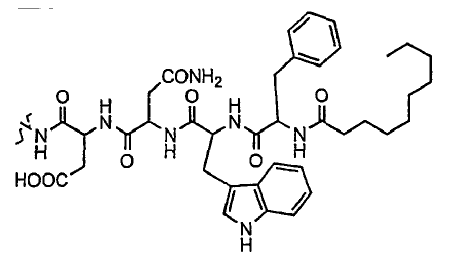 | NH₂ |
| 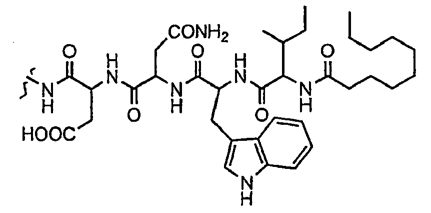 | NH₂ |
| 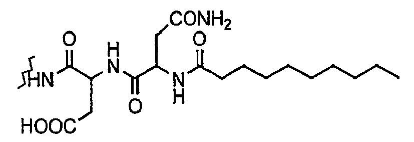 | NH₂ |
| 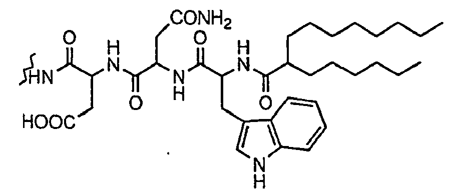 | NH₂ |
| 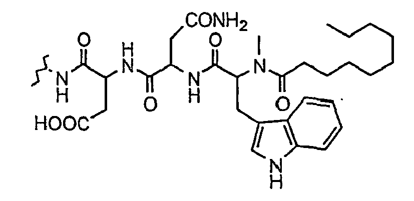 | NH₂ |
| 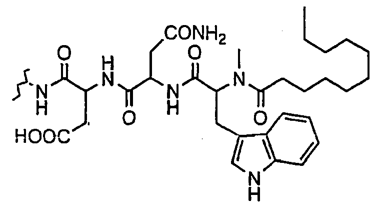 | NH₂ |
| 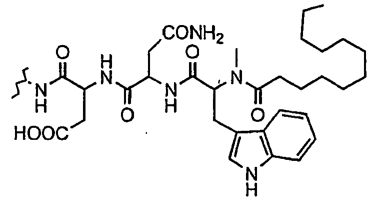 | NH₂ |
| 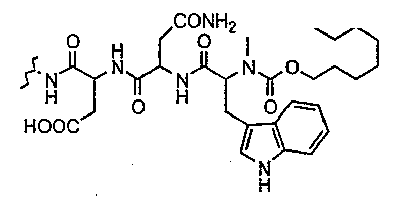 | NH₂ |
| 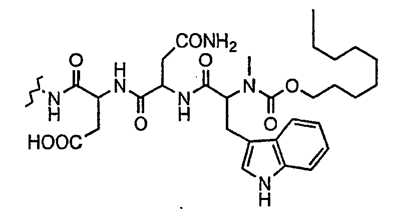 | NH₂ |
| 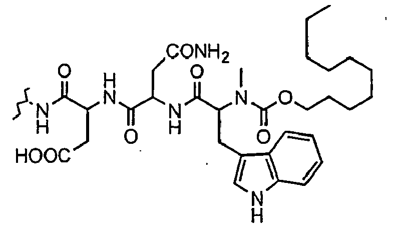 | NH₂ |
| 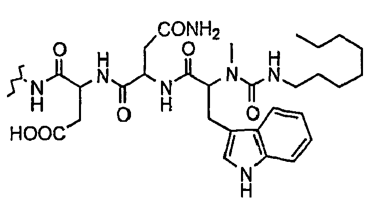 | NH₂ |
| 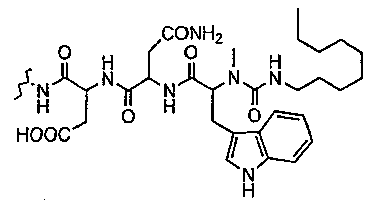 | NH₂ |
| 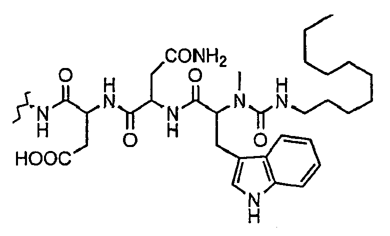 | NH₂ |
| 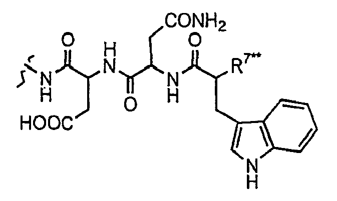 | NH₂ |
| 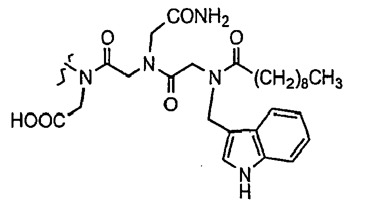 | NH₂ |
| 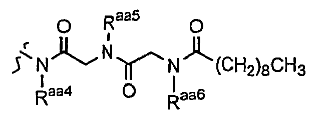 | NH₂ |

Wherein R^{7**} is an amino, monosubstituted amino, disubstituted amino, acylamino, ureido, guanidino, carbamoyl, sulfonamino, thioacylamino, thioureido, iminoamino, or phosphonamino and each of R^{aa4}, R^{aa5}, and R^{aa6} is independently an amino acid side chain.

### Intermediates

The.present invention also provides compounds of the Formula IV that are particularly useful as intermediates for the preparation of the compounds of Formula I. 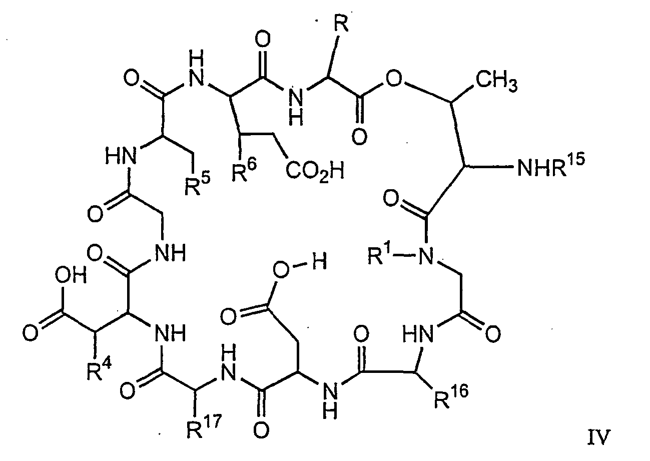 and salts thereof ; wherein:
(a) R is 2-butyl, isopropyl or 2-(2'-aminophenacyl) ;
(b) each R¹ and R⁶ is independently hydrido or methyl;
(c) R⁴ is hydrido or methoxy;
(d) R⁵ is hydroxy or carboxyamino;
(e) R¹⁵ is hydrido, 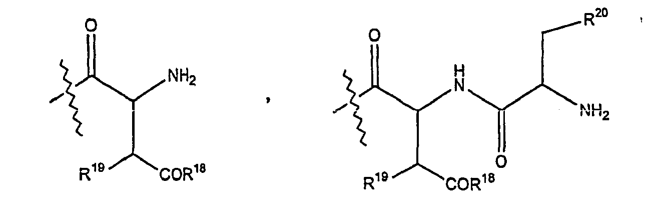 or 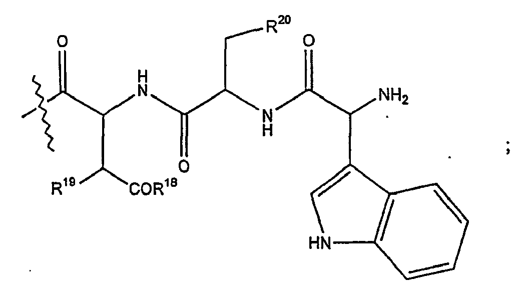 wherein: R¹⁸ is amino or hydroxy; R¹⁹ is hydrido or hydroxy; and R²⁰ is carboxyamino or carboxymethyl
(f) R¹⁶ is methyl or-CH₂CH₂CH₂R²¹;
(g) R¹⁷ is methyl or-CH₂CH₂CH₂CH₂R²²;
wherein each of R²¹ and R²² is independently amino, monosubstituted amino, disubstituted amino, acylamino, ureido, guanidino, carbamoyl, sulfonamino, thioacylamino, thioureido, iminoamino, or phosphonamino.

In a preferred embodiment of the invention, each of R²¹ and R²² is-NHR²³, wherein R²³ is an amino protecting group. In a more preferred embodiment of the invention, R²³ is a carbamate amino protecting group selected from allyloxycarbonyl, carbobenzyloxycarbonyl and *tert*-butoxycarbonyl. In the most preferred embodiment R²³ is allyloxycarbonyl.

In a more preferred embodiment, the present invention provides intermediates of the Formulas V, VI, VII, VIII, IX and X that are particularly useful as intermediates for the preparation of the compounds of Formula I. 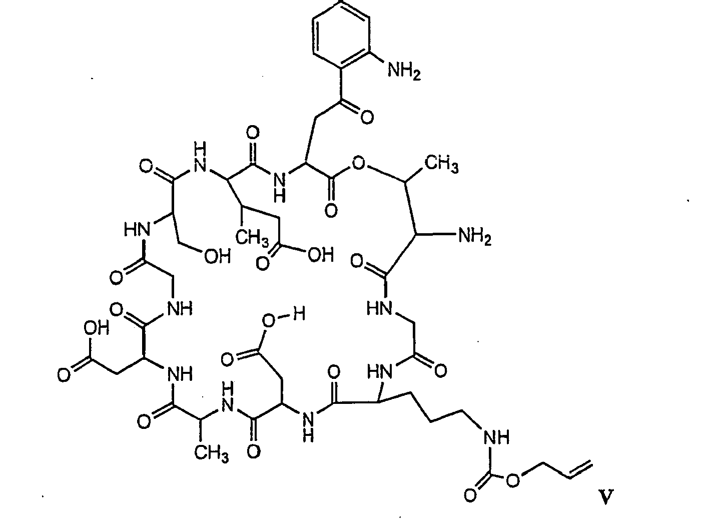 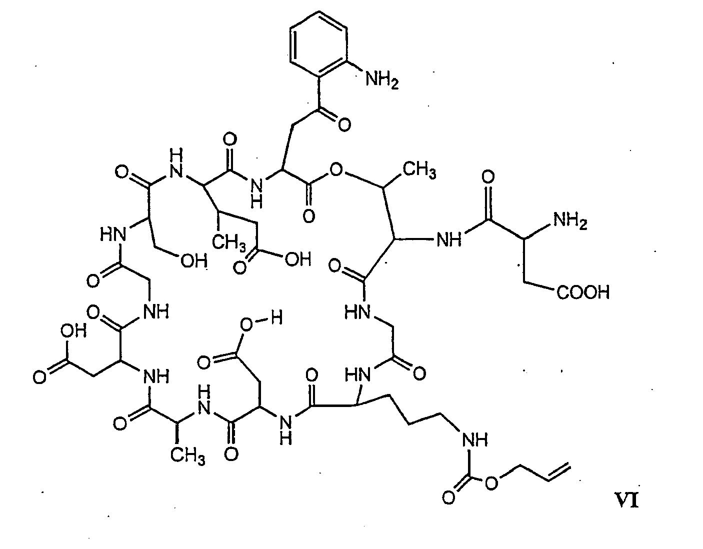 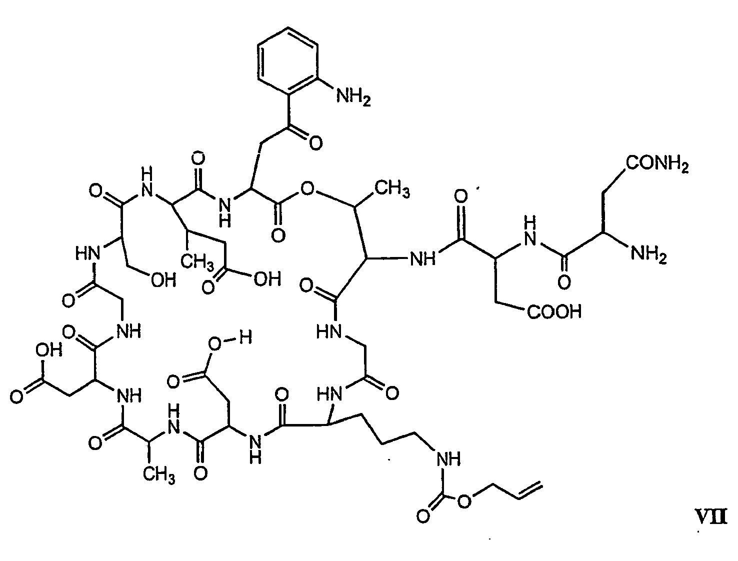 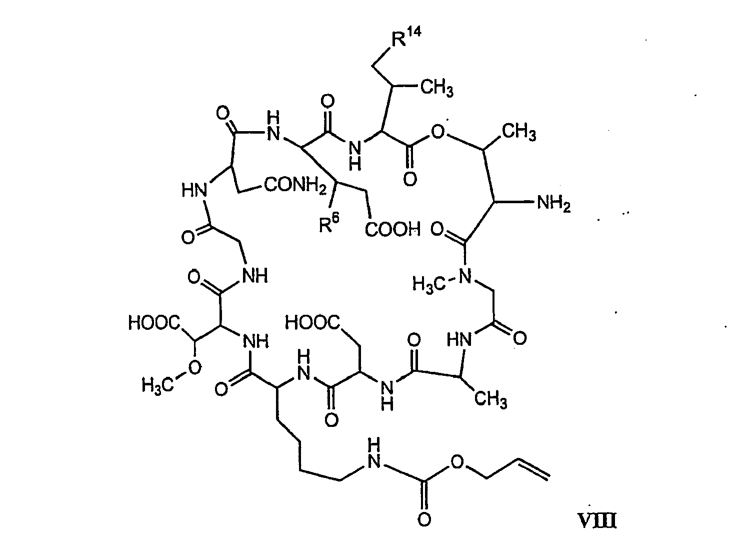 wherein R⁶ and R¹⁴ are as previously defined. 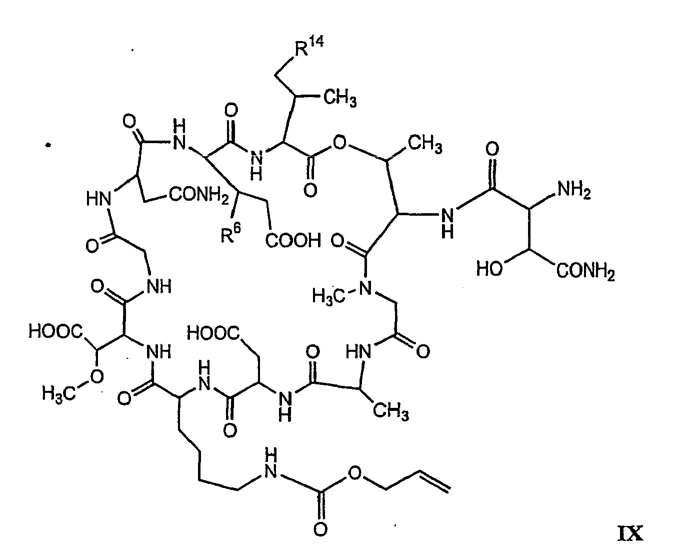 wherein R⁶ and R¹⁴ are as previously defined. 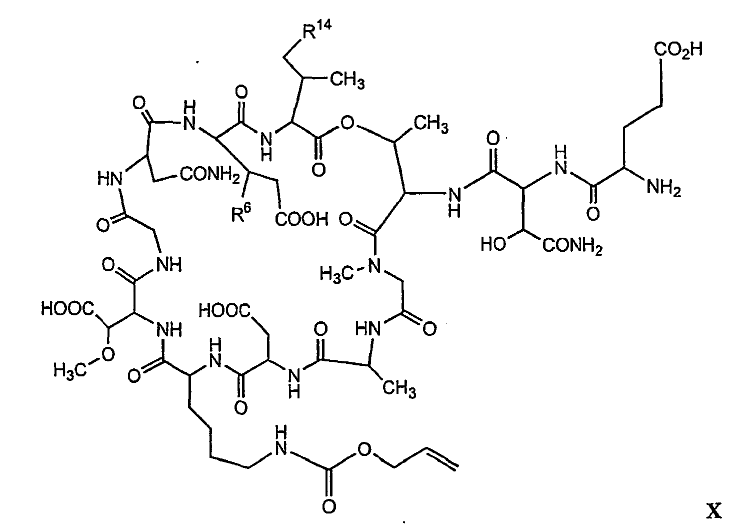 wherein R⁶ and R¹⁴ are as previously defined.

### Pharmaceutical Compositions and Methods of Use Thereof

The instant invention provides pharmaceutical compositions or formulations comprising compounds of Formula I, or salts thereof.

Compounds of the present invention, preferably compounds of Formula I, or pharmaceutically acceptable salts thereof, can be formulated for oral, intravenous, intramuscular, subcutaneous or parenteral administration for the therapeutic or prophylactic treatment of diseases, particularly bacterial infections. For oral or parenteral administration, compounds of the present invention can be mixed with conventional pharmaceutical carriers and excipients and used in the form of tablets, capsules, elixirs, suspensions, syrups, wafers and the like. The compositions comprising a compound of this invention will contain from about 0.1 to about 99% by weight of the active compound, and more generally from about 10 to about 30%.

The pharmaceutical preparations disclosed herein are prepared in accordance with standard procedures and are administered at dosages that are selected to reduce, prevent or eliminate the infection (See, e. g., Remington's Pharmaceutical Sciences, Mack Publishing Company, Easton, PA and Goodman and Gilman's The Pharmaceutical Basis of Therapeutics, Pergamon Press, New York, NY, the contents of which are incorporated herein by reference, for a general description of the methods for administering various antimicrobial agents for human therapy). The compositions of the present invention, preferably compounds of Formula I, can be delivered using controlled (e.g., capsules) or sustained release delivery systems (e.g., bioerodable matrices). Exemplary delayed release delivery systems for drug delivery that are suitable for administration of the compositions of the invention, preferably of Formula I, are described in U.S. Patent Nos. 4,452,775 (issued to Kent), 5,239,660 (issued to Leonard), 3,854,480 (issued to Zaffaroni).

The pharmaceutically-acceptable compositions of the present invention comprise one or more compounds of the invention, preferably compounds of Formula I, in association with one or more nontoxic, pharmaceutically-acceptable carriers and/or diluents and/or adjuvants and/or excipients, collectively referred to herein as "carrier" materials, and if desired other active ingredients. The compositions may contain common carriers and excipients, such as corn starch or gelatin, lactose, sucrose, microcrystalline cellulose, kaolin, mannitol, dicalcium phosphate, sodium chloride and alginic acid. The compositions may contain croscarmellose sodium, microcrystalline cellulose, corn starch, sodium starch glycolate and alginic acid.

Tablet binders that can be included are acacia, methylcellulose, sodium carboxymethylcellulose, polyvinylpyrrolidone (Povidone), hydroxypropyl methylcellulose, sucrose, starch and ethylcellulose.

Lubricants that can be used include magnesium stearate or other metallic stearates, stearic acid, silicone fluid, talc, waxes, oils and colloidal silica.

Flavoring agents such as peppermint, oil of wintergreen, cherry flavoring or the like can also be used. It may also be desirable to add a coloring agent to make the dosage form more aesthetic in appearance or to help identify the product

For oral use, solid formulations such as tablets and capsules are particularly useful. Sustained release or enterically coated preparations may also be devised. For pediatric and geriatric applications, suspensions, syrups and chewable tablets are especially suitable. For oral administration, the pharmaceutical compositions are in the form of, for example, a tablet, capsule, suspension or liquid. The pharmaceutical composition is preferably made in the form of a dosage unit containing a therapeutically-effective amount of the active ingredient. Examples of such dosage units are tablets and capsules. For therapeutic purposes, the tablets and capsules which can contain, in addition to the active ingredient, conventional carriers such as binding agents, for example, acacia gum, gelatin, polyvinylpyrrolidone, sorbitol, or tragacanth; fillers, for example, calcium phosphate, glycine, lactose, maize-starch, sorbitol, or sucrose; lubricants, for example, magnesium stearate, polyethylene glycol, silica, or talc; disintegrants, for example, potato starch, flavoring or coloring agents, or acceptable wetting agents. Oral liquid preparations generally are in the form of aqueous or oily solutions, suspensions, emulsions, syrups or elixirs may contain conventional additives such as suspending agents, emulsifying agents, non-aqueous agents, preservatives, coloring agents and flavoring agents. Examples of additives for liquid preparations include acacia, almond oil, ethyl alcohol, fractionated coconut oil, gelatin, glucose syrup, glycerin, hydrogenated edible fats, lecithin, methyl cellulose, methyl or propyl para-hydroxybenzoate, propylene glycol, sorbitol, or sorbic acid.

For intravenous (IV) use, a compound of the present invention can be dissolved or suspended in any of the commonly used intravenous fluids and administered by infusion. Intravenous fluids include, without limitation, physiological saline or Ringer's solution. Intravenous administration may be accomplished by using, without limitation, syringe, minipump or intravenous line.

Formulations for parenteral administration can be in the form of aqueous or non-aqueous isotonic sterile injection solutions or suspensions. These solutions or suspensions can be prepared from sterile powders or granules having one or more of the carriers mentioned for use in the formulations for oral administration. The compounds can be dissolved in polyethylene glycol, propylene glycol, ethanol, corn oil, benzyl alcohol, sodium chloride, and/or various buffers.

For intramuscular preparations, a sterile formulation of a compound of the present invention, or a suitable soluble salt form of the compound, for example the hydrochloride salt, can be dissolved and administered in a pharmaceutical diluent such as Water-for-Injection (WFI), physiological saline or 5% glucose. A suitable insoluble form of the compound may be prepared and administered as a suspension in an aqueous base or a pharmaceutically acceptable oil base, e.g., an ester of a long chain fatty acid such as ethyl oleate.

A dose of an intravenous, intramuscular or parental formulation of a compound of the present invention may be adminstered as a bolus or by slow infusion. A bolus is a dose that is administered in less than 30 minutes. In a preferred embodiment, a bolus is administered in less than 15 or less than 10 minutes. In a more preferred embodiment, a bolus is administered in less than 5 minutes. In an even more preferred embodiment, a bolus is administered in one minute or less. An infusion is a dose that is administered at a rate of 30 minutes or greater. In a preferred embodiment, the infusion is one hour or greater. In another embodiment, the infusion is substantially constant.

For topical use the compounds of the present invention, preferably compounds of Formula I, can also be prepared in suitable forms to be applied to the skin, or mucus membranes of the nose and throat, and can take the form of creams, ointments, liquid sprays or inhalants, lozenges, or throat paints. Such topical formulations further can include chemical compounds such as dimethylsulfoxide (DMSO) to facilitate surface penetration of the active ingredient

For application to the eyes or ears, the compounds of the present invention, preferably compounds of Formula I, can be presented in liquid or semiliquid form formulated in hydrophobic or hydrophilic bases as ointments, creams, lotions, paints or powders.

For rectal administration the compounds of the present invention, preferably compounds of Formula I, can be administered in the form of suppositories admixed with conventional carriers such as cocoa butter, wax or other glyceride.

Alternatively, the compounds of the present invention, preferably compounds of Formula I, can be in powder form for reconstitution in the appropriate pharmaceutically acceptable carrier at the time of delivery. In another embodiment, the unit dosage form of the compound can be a solution of the compound or preferably a salt thereof in a suitable diluent in sterile, hermetically sealed ampoules or sterile syringes. The concentration of the compound in the unit dosage may vary, e.g. from about 1 percent to about 50 percent, depending on the compound used and its solubility and the dose desired by the physician. If the compositions contain dosage units, each dosage unit preferably contains from 1-500 mg of the active material. For adult human treatment, the dosage employed preferably ranges from 5 mg to 10 g, per day, depending on the route and frequency of administration.

In another aspect, the invention provides a method for inhibiting the growth of microorganisms, preferably bacteria, comprising contacting said organisms with a compound of the present invention under conditions which permit contact of the compound with said organism and with said microorganism. Such conditions are known to one skilled in the art and are exemplified in the Examples. This method involves contacting a microbial cell with a therapeutically-effective amount of compound(s) of the invention, preferably compound(s) of Formula I, *in vivo* or *in vitro.*

According to this aspect of the invention, the novel compositions disclosed herein are placed in a pharmaceutically acceptable carrier and are delivered to a recipient subject (preferably a human) in accordance with known methods of drug delivery. In general, the methods of the invention for delivering the compositions of the invention *in vivo* utilize art-recognized protocols for delivering the agent with the only substantial procedural modification being the substitution of the compounds of the present invention, preferably compounds of Formula I, for the drugs in the art-recognized protocols. Likewise, the methods for using the claimed composition for treating cells in culture, for example, to eliminate or reduce the level of bacterial contamination of a cell culture, utilize art-recognized protocols for treating cell cultures with antibacterial agent(s) with the only substantial procedural modification being the substitution of the compounds of the invention, preferably compounds of Formula I, for the agents used in the art-recognized protocols.

In one embodiment, the invention provides a method for treating an infection, especially those caused by gram-positive bacteria, in a subject with a therapeutically-effective amount of a compound of the invention. Exemplary procedures for delivering an antibacterial agent are described in U.S. Patent No. 5,041,567, and PCT patent application number EP94/02552 (publication no. WO 95/05384), the entire contents of which documents are incorporated in their entirety herein by reference. As used herein, the phrase "therapeutically-effective amount" means an amount of a compound of the present invention that prevents the onset, alleviates the symptoms, or stops the progression of a bacterial infection. The term "treating" is defined as administering, to a subject, a therapeutically-effective amount of a compound of the invention both to prevent the occurrence of an infection and to control or eliminate an infection. The term "subject," as described herein, is defined as a mammal, a plant or a cell culture. In a preferred embodiment, a subject is a human or other animal patient in need of antibacterial treatment.

The method comprises administering to the subject an effective dose of a compound of the present invention. An effective dose is generally between about 0.1 and about 100 mg/kg of a compound of the invention or a pharmaceutically acceptable salt thereof. A preferred dose is from about 0.1 to about 50 mg/kg of a compound of the invention or a pharmaceutically acceptable salt thereof. A more preferred dose is from about 1 to 25 mg/kg of a compound of the invention or a pharmaceutically acceptable salt thereof. An effective dose for cell culture is usually between 0.1 and 1000 µg/mL, more preferably between 0.1 and 200 µg/mL.

Compositions containing the compounds of the invention can be administered as a single daily dose or in multiple doses per day. The treatment regime may require administration over extended periods of time, e.g., for several days or for from two to four weeks. The amount per administered dose or the total amount administered will depend on such factors as the nature and severity of the infection, the age and general health of the patient, the tolerance of the patient to the compound and the microorganism or microorganisms involved in the infection. A method of administration to a patient of daptomycin, another member of the depsipeptide compound class, is disclosed in United States Serial No. 09/406,568, filed September 24, 1999, which claims the benefit of U.S. Provisional Application Nos. 60/101,828, filed September 25, 1998, and 60/125,750, filed March 24, 1999, the contents of which are herein incorporated by reference.

A compound of the present invention may also be administered in the diet or feed of a patient or animal. If administered as part of a total dietary intake, the amount of compound employed can be less than 1% by weight of the diet and preferably no more than 0.5% by weight. The diet for animals can be normal foodstuffs to which the compound can be added or it can be added to a premix.

The present invention also provides methods of administering a compound of Formula I or a pharmaceutical composition thereof to a subject in need thereof in an amount that is efficacious in reducing or eliminating the bacterial infection. The compound may be administered orally, parenterally, by inhalation, topically, rectally, nasally, buccally, vaginally, or by an implanted reservoir, external pump or catheter. The compound may be prepared for opthalmic or aerosolized uses. The compounds of the present invention can be administered as an aerosol for the treatment of pneumonia or other lung-based infections. A preferred aerosol delivery vehicle is an anhydrous or dry powder inhaler. Compounds of Formula I or a pharmaceutical composition thereof may also be directly injected or administered into an abscess, ventricle or joint. Parenteral administration includes subcutaneous, intravenous, intramuscular, intra-articular, intra-synovial, cisternal, intrathecal, intrahepatic, intralesional and intracranial injection or infusion. In a preferred embodiment, the compounds of the present invention are administered intravenously, subcutaneously or orally. In a preferred embodiment for administering a compound according to Formula I to a cell culture, the compound may be administered in a nutrient medium.

The method of the instant invention may be used to treat a subject having a bacterial infection in which the infection is caused or exacerbated by any type of bacteria, particularly gram-positive bacteria. In one embodiment, a compound of the present invention or a pharmaceutical composition thereof is administered to a patient according to the methods of this invention. In a preferred embodiment, the bacterial infection may be caused or exacerbated by gram-positive bacteria. These gram-positive bacteria include, but are not limited to, methicillin-susceptible and methicillin-resistant staphylococci (including *Staphylococcus aureus, S. epidermidis, S. haemolyticus, S. hominis, S. saprophyticus,* and coagulase-negative staphylococci), glycopeptide intermediary- susceptible *S. aureus* (GISA), vancomycin-resistant *Staphylococcus aureus* (VRSA), penicillin-susceptlible and penicillin-resistant streptococci (including *Streptococcus pneumoniae, S. pyogenes, S. agalactiae, S. avium, S. bovis, S. lactis, S. sangius* and *Streptococci* Group C, *Streptococci* Group G and viridans streptococci), enterococci (including vancomycin-susceptible and vancomycin-resistant strains such as *Enterococcus faecalis* and *E. faecium*), *Clostridium difficile, C. clostridiiforme, C. innocuum, C. perfringens, C. ramosum, Haemophilus influenzae, Listeria monocytogenes, Corynebacterium jeikeium, Bifidobacterium* spp., *Eubacterium aerofaciens, E. lentum, Lactobacillus acidophilus, L. casei, L. plantarum, Lactococcus* spp., *Leuconostoc* spp., *Pediococcus, Peptostreptococcus anaerobius, P. asaccarolyticus, P. magnus, P. micros, P. prevotii, P. productus, Propionibacterium acnes, Actinomyces* spp., *Moraxella* spp. (including *M. catarrhalis*) and *Escherichia* spp. (including *E. coli*).

In a preferred embodiment, the antibacterial activity of compounds of Formula I against classically "resistant" strains is comparable to that against classically "susceptible" strains in *in vitro* experiments. In another preferred embodiment, the minimum inhibitory concentration (MIC) value for compounds according to this invention against susceptible strains is typically the same or lower than that of vancomycin. Thus, in a preferred embodiment, a compound of this invention or a pharmaceutical composition thereof is administered according to the methods of this invention to a patient who exhibits a bacterial infection that is resistant to other compounds, including vancomycin or daptomycin. In addition, unlike glycopeptide antibiotics, depsipeptide compounds such as those disclosed in the present invention, exhibit rapid, concentration-dependent bactericidal activity against gram-positive organisms. Thus, in a preferred embodiment, a compound according to this invention or a pharmaceutical composition thereof is administered according to the methods of this invention to a patient in need of rapidly acting antibiotic therapy.

The method of the instant invention may be used for any bacterial infection of any organ or tissue in the body. In a preferred embodiment, the bacterial infection is caused by gram-positive bacteria. These organs or tissue include, without limitation, skeletal muscle, skin, bloodstream, kidneys, heart, lung and bone. The method of the invention may be used to treat, without limitation, skin and soft tissue infections, bacteremia and urinary tract infections. The method of the invention may be used to treat community acquired respiratory infections, including, without limitation, otitis media, sinusitis, chronic bronchitis and pneumonia, including pneumonia caused by drug-resistant *S. pneumoniae* or *H. influenzae.* The method of the invention also may be used to treat mixed infections that comprise different types of gram-positive bacteria, or which comprise both gram-positive and gram-negative bacteria. These types of infections include intra-abdominal infections and obstetrical/gynecological infections. The method of the invention also may be used to treat an infection including, without limitation, endocarditis, nephritis, septic arthritis, intra-abdominal sepsis, bone and joint infections. and osteomyelitis. In a preferred embodiment, any of the above-described diseases may be treated using compounds according to this invention or pharmaceutical compositions thereof.

The method of the present invention may also be practiced while concurrently administering one or more other antimicrobial agents, such as antibacterial agents (antibiotics) or antifungal agents. In one aspect, the method may be practiced by administering more than one compound according to this invention. In another embodiment, the method may be practiced by administering a compound according to this invention with a lipopeptide compound, such as daptomycin or the lipopeptide compounds described, for example in International Patent Applications WO01/44272; WO01/44274; and WO01/44271.

Antibacterial agents and classes thereof that may be co-administered with a compound according to the invention include, without limitation, penicillins and related drugs, carbapenems, cephalosporins and related drugs, aminoglycosides, bacitracin, gramicidin, mupirocin, chloramphenicol, thiamphenicol, fusidate sodium, lincomycin, clindamycin, macrolides, novobiocin, polymyxins, rifamycins, spectinomycin, tetracyclines, vancomycin, teicoplanin, streptogramins, anti-folate agents including sulfonamides, trimethoprim and its combinations and pyrimethamine, synthetic antibacterials including nitrofurans, methenamine mandelate and methenamine hippurate, nitroimidazoles, quinolones, fluoroquinolones, isoniazid, ethambutol, pyrazinamide, para-aminosalicylic acid (PAS), cycloserine, capreomycin, ethionamide, prothionamide, thiacetazone, viomycin, eveminomycin, glycopeptide, glycylcylcline, ketolides, oxazolidinone; imipenen, amikacin, netilmicin, fosfomycin, gentamicin, ceftriaxone, Ziracin, LY 333328, CL 331002, HMR 3647, Zyvox®, Synercid®, Aztreonam, and Metronidazole, Epiroprim, OCA-983, GV-143253, Sanfetrinem sodium, CS-834, Biapenem, A-99058.1, A-165600, A-179796, KA 159, Dynemicin A, DX8739, DU 6681; Cefluprenam, ER 35786, Cefoselis, Sanfetrinem celexetil, HGP-31, Cefpirome, HMR-3647, RU-59863, Mersacidin, KP 736, Rifalazil; AM 1732, MEN 10700, Lenapenem, BO 2502A, NE-1530, PR 39, K130, OPC 20000, OPC 2045, Veneprim, PD 138312, PD 140248, CP 111905, Sulopenem, ritipenam acoxyl, RO-65-5788, Cyclothialidine, Sch-40832, SEP-132613, micacocidin A, SB-275833, SR-15402, SUN A0026, TOC 39, carumonam, Cefozopran, Cefetamet pivoxil, and T 3811.

Antifungal agents that may be co-administered with a compound according to the invention include, without limitation, Caspofungen, Voriconazole, Sertaconazole, IB-367, FK-463, LY-303366, Sch-56592, Sitafloxacin, DB-289 polyenes, such as Amphotericin, Nystatin, Primaricin; azoles, such as Fluconazole, Itraconazole, and Ketoconazole; allylamines, such as Naftifine and Terbinafine; and anti-metabolites such as Flucytosine. Other antifungal agents include without limitation, those disclosed in Fostel et al., Drug Discovery Today 5:25-32 (2000), herein incorporated by reference. Fostel et al. discloses antifungal compounds including Corynecandin, Mer-WF3010, Fusacandins, Artrichitin/LL 15G256, Sordarins, Cispentacin, Azoxybacillin, Aureobasidin and Khafrefungin.

A compound according to this invention may be administered according to this method until the bacterial infection is eradicated or reduced. In one embodiment, a compound of Formula I is administered for a period of time from 2 days to 6 months. In a preferred embodiment, a compound of Formula I is administered for 7 to 56 days. In a more preferred embodiment a compound of Formula I is administered for 7 to 28 days. In an even more preferred embodiment, a compound of Formula I is administered for 7 to 14 days. A compound of Formula I may be administered for a longer or shorter time period if it is so desired.

### Preparation of Novel Depsipeptides

### 1. Semisynthetic Process

### Process for the preparation of compounds of Formula I wherein at least one of R² and R³ is other than methyl and each of R⁸ and R⁹ is independently NH₂.

### Procedure A

For compounds of Formula I wherein at least one of R² and R³ is other than methyl and each of R⁸ and R⁹ is independently NH₂, the process, in accordance with one aspect of the invention, comprises the steps of:
(a) providing a depsipeptide derivative of the Formula XI 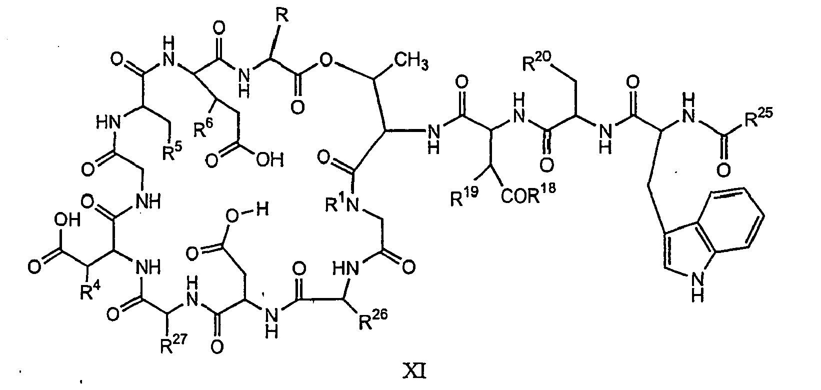 wherein: R, R¹, R⁴ R⁵, R⁶, R¹⁸, R¹⁹, and R²⁰ are as previously described; R²⁵ is an alkyl group; R²⁶ is methyl or -CH₂CH₂CH₂NH₂; and R²⁷ is methyl or -CH₂CH₂CH₂CH₂NH₂; or a salt thereof
(b) protecting the free amino group(s) of the compound of Formula XI with a protecting group to obtain a protected depsipeptide compound.
(c) treating the protected depsipeptide compound obtained in (b) with a deacylating agent to obtain a terminal amino compound;
(d) removing the tryptophan amino acid residue of the terminal amino compound obtained in (c) to obtain a destryptophan compound;
(e) removing the terminal amino acid residue of the destryptophan compound obtained in (d) to obtain a desdipeptide compound;
(f) removing the terminal amino acid residue of the desdipeptide compound obtained in (e) to obtain a depsipeptide core compound;
(g) treating the depsipeptide core compound of (f) with a modifying agent; and
(h) removing the protecting group from the protected Formula I compound to obtain Formula Ia compound.

Procedure A is illustrated in Scheme I.

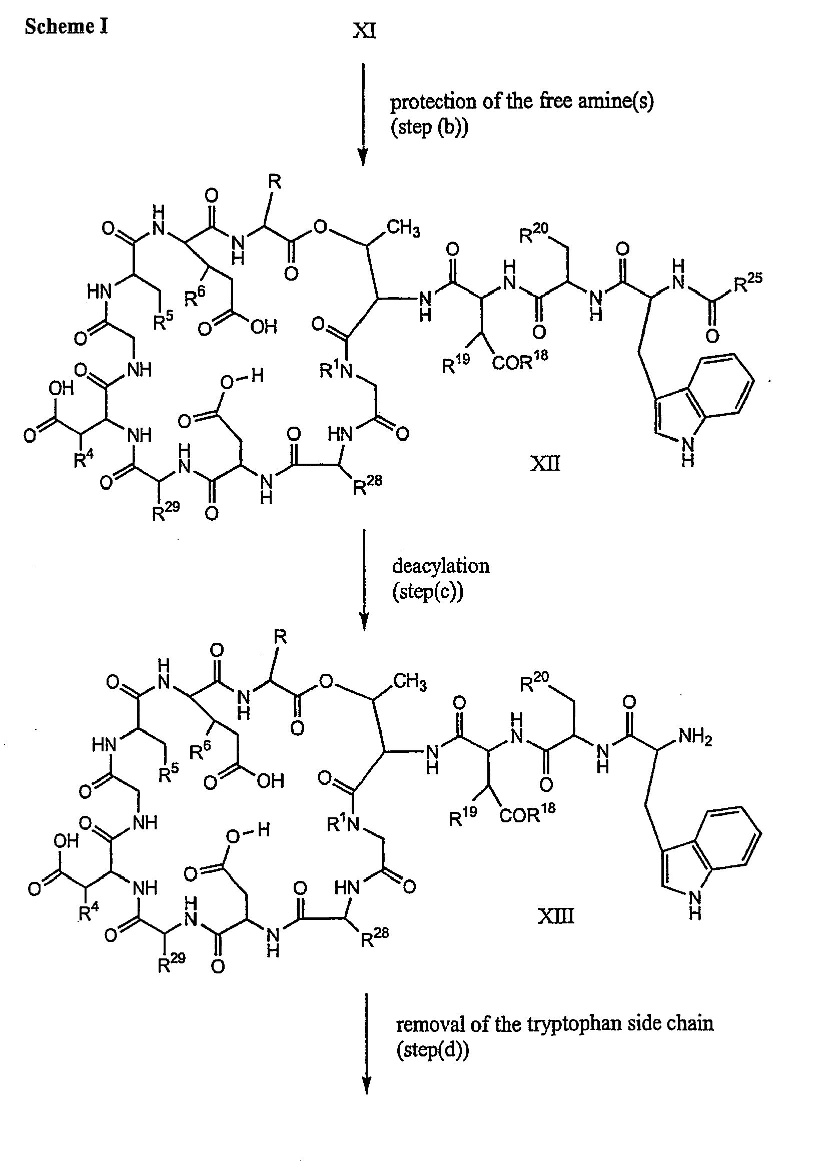

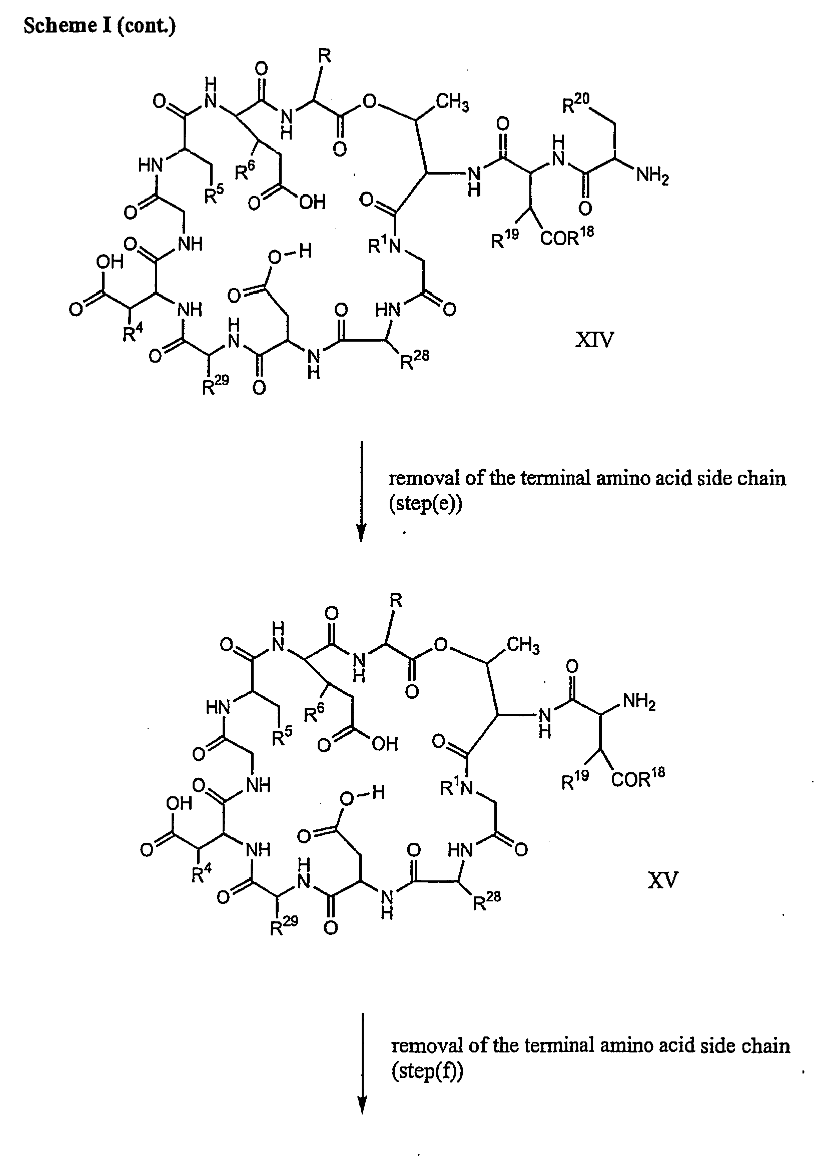

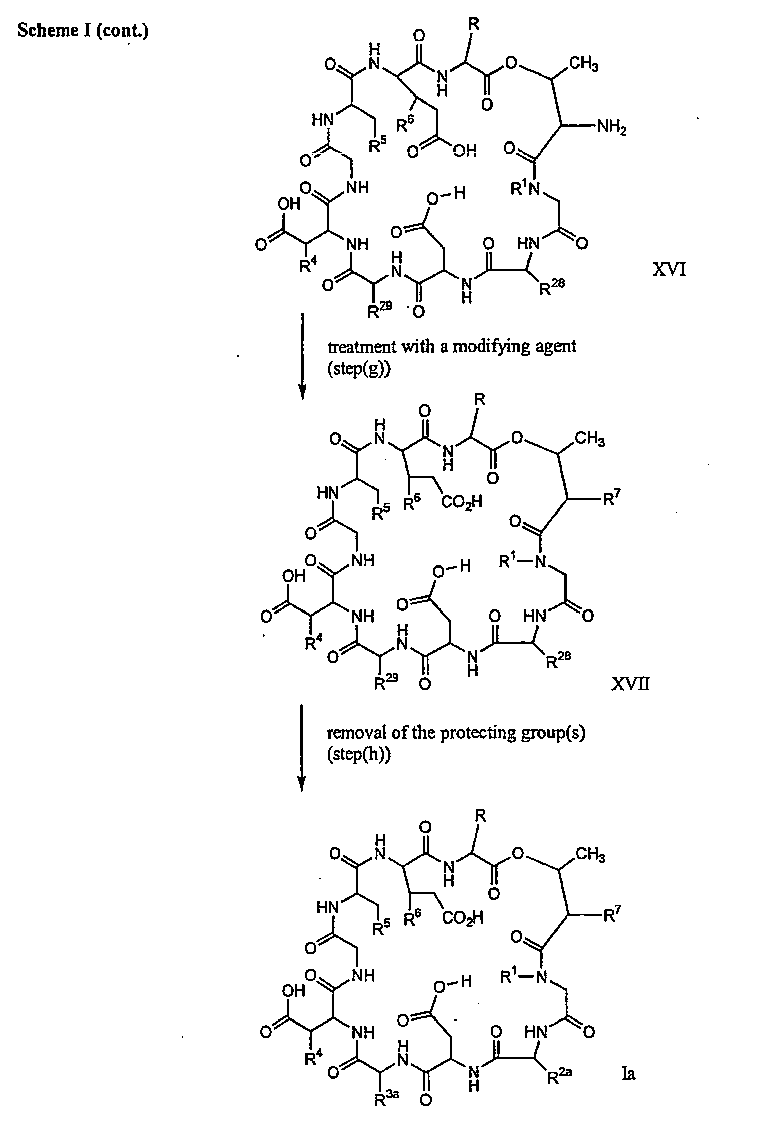

The semisynthetic process, in accordance with one aspect of the invention comprises providing a compound of the Formula XI (step(a)). Compounds of Formula XI can be obtained by methods disclosed in United States Patents RE 32,333; RE 32,455; RE 32,311; 4,482,487; 4,537,717; 4,800,157, 4,874,843; 4,885,243; 5,912,226; 4,994,270; 5,039,789; and 5,028,590; International Patent Application Serial Numbers WO01/44272, WO01/44274, WO01/44271, WO01/53330, and WO02/059,322 each of which is incorporated herein by reference.

In a preferred embodiment, the compound of Formula XI is one in which, each of R¹ and R⁴ is hydrido; each of R³ and R⁶ is methyl; R⁵ is hydroxyl; R²⁵ is 7-methylnonyl, 9-methyldecyl, 9-methylundecyl, nonyl, decyl or mixtures thereof and R²⁶ is -CH₂CH₂CH₂NH₂.

In another preferred embodiment, the compound of Formula XI is one in which R is isopropyl or 2-butyl; each of R¹ and R² is methyl; R⁴ is methoxy, R⁵ is carboxyamino; R²⁵ is 8-methylnonanoyl, n-decanoyl, or 8-methyldecanoyl; and R²⁷ is -CH₂CH₂CH₂CH₂NH₂;.

The free amine of the compound of Formula XI is treated with a protecting group to give a protected depsipeptide compound of Formula XII(step (b)), wherein: R, R¹, R⁴, R⁵, R⁶, R¹⁸, R¹⁹, R²⁰, and R²⁵ are as described previously; R²⁸ is methyl or-CH₂CH₂CH₂NHP; R²⁹ is methyl or-CH₂CH₂CH₂CH₂NHP; wherein P is an amino protecting group or a salt thereof.

Examples of amino protecting groups and methods for protecting amines with said groups can be found in Protective Groups in Organic Synthesis by Theodora W. Greene, (New York: John Wiley and Sons, Inc.), 1981, hereafter "Greene", incorporated herein by reference. Preferred amino protecting groups are carbamate amino protecting groups. More preferred amino groups are allyloxycarbonyl (alloc), carbobenzyloxy (CBZ), and *tert*-butoxycarbonyl protecting groups. The most preferred carbamate amino protecting group is allylyoxycarbonyl. Methods of protecting the amine of daptomycin, A54145 and related lipopeptides can be found in United States Patents RE 32,310; RE 32,311; 4,482,487; 4,524,135, 4,537,717; 5,039,789; and 5,028590; International Patent Application Serial Numbers WO01/44272, WO01/44274, and WO01/44271.

The protected depsipeptide compound is then treated with a deacylating agent to form the terminal amino compound of Formula XIII (step(c)). Deacylating agents suitable for the invention are enzymatic deacylating agents. An enzyme which is useful for deacylation of a compound of Formula XII is produced by certain microorganism of the family Actinoplanaceae. Some of these known species and varieties of this family include *Actinoplanes philippinensis, Actinoplanes armeniacus, Actinoplanes utahensis, Actinoplanes missouriensis, Spirillospora albida, Streptosporiangium roseum, Streptosporangium vulgare, Streptosporangium roseum* var *hollandensi, Streptosporangium album, Streptosporangium viridialbum, Amorphosporangium auranticolor, Ampullariella regularis, Ampullariella campanulata, Ampullariella lobata, Ampullariella digitata, Pilimelia terevasa, Pimelia anulata, Planomonospora parontospora, Planomonospora venezuelensis, Planobispora longispora, Planobispora rosea, Dactylosporangium aurantiacum, and Dactylosporangium thailandende.*

All natural and artificial variants and mutants which are obtained from the Actinoplanacea and which produce the enzyme may be used in this invention.

Preferred sources of the deacylation enzyme are *Actinoplanes utahensi :* NRRL 12052; *Actinoplanes missouriensis* NRRL 12053; *Actinoplanes sp.:* NRRL8122, *Actinoplanes sp.:* NRRL 12065, *Streptosporsngium roseum* var *hollandensis :* NRRL 12064, *Actinoplanes utahenis* ATCC 14539 and *Actinoplanes missouriensis* ATCC 14538. The more preferred source of deacylation enzyme is the species *Actinoplanes utahensi.* The most preferred source of deacylation enzyme is one produced from recombinant *Streptomyces lividans,* which expresses the *Actinoplanes utahensis* deacylation enzyme as described in J. Ind. Microbiol. Biotechnol. 2000, 24(3) 173-180. This enzyme is also known as echinocandin B deacylase or ECB deacylase.

Suitable methods for enzymatic deacylation of compounds of Formula XII can be found in United States Patent 4,524,135; 4,537,717; 4,482,487; RE 32,310, RE 32,311 5,039,789 and 5,028590; International Patent Application Serial Numbers WO01/44272, WO01/44274, and WO01/44271; each herein incorporated by reference.

Removal of the tryptophan amino acid residue from the terminal amino compound of Formula XIII leads to the formation of the compound of Formula XIV (step(d)). Methods for removal of the tryptophan amino acid residue are known to those skilled in the art. A preferred method for removal of the tryptophan amino acid residue is under Edman degradation conditions.

The Edman degradation is a well-established reaction known to those skilled in the art (see, for example, P. Edman, 1950, Acta Chem. Scan. 4: 283-93 and P. Edman, 1956, Acta Chem Scan 10: 761-768). In this reaction the terminal NH₂ group of a peptide reacts with an isothiocycanate to form a thiourea derivative of the peptide. Upon treatment with acid or base, the thiourea peptide undergoes a cyclization reaction, giving a thiohydantoin and a shorter peptide (see Scheme II). 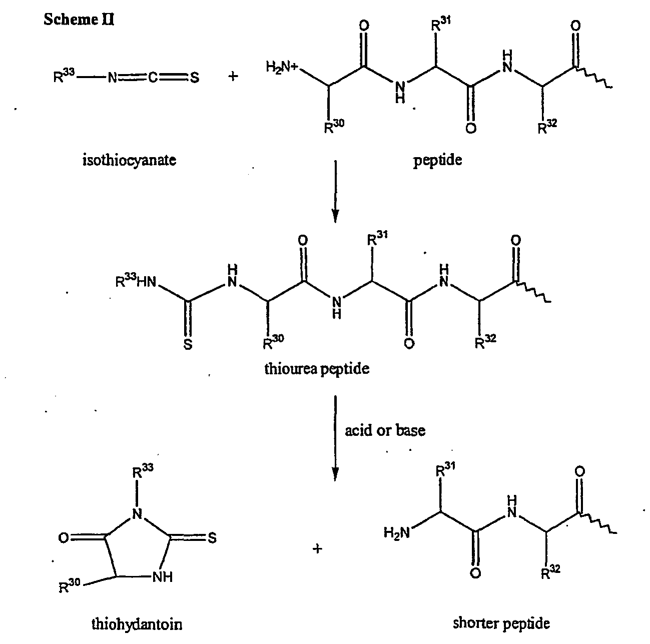 wherein R³⁰, R³¹, and R³², are each an amino acid side chain; and R³³ is an aryl or alkyl group.

The Edman degradation can be carried out under a variety of conditions. In the first step of the Edman degradation sequence the isothiocyanate reacts with the amine under neutral to mildly basic (pH <9.5) conditions in solvents such as tetrahydrofuran, N, N'-dimethylformamide, dichloromethane, dioxane or ethanol. A variety of isothiocyanates can be used (see K. K. Han et al. Biochemie 1977, 59: 557-576.

Subsequent cyclization and cleavage can be accomplished under a variety of conditions. Typically, anhydrous trifluoroacetic acid, heptafluorobutyric acid (see, for example, W. F. Brandt et al., 1976, Z. Physiol. Chem. 357: 1505-1508) or concentrated hydrochloric acid (see, for example, G. E. Tarr, 1977, Methods in Enzymology, 47: 335-337) are used. Mild basic conditions such as triethylamine or N, N-dimethylallyamine / acetic acid (pH ~9) can also be used (see G.C. Barrett et al., 1985, Tetrahedron Letters 26(36): 4375-4378): For a review of this reaction see K.K. Han, 1985, Int. J. Biochem 17(4): 429-445.

In a preferred embodiment, the thiourea peptide (the compound of Formula XVIII) formed upon reaction of the thioisocyanate with a compound of Formula XIII is treated under acidic conditions to provide a compound of Formula XIV. In a more preferred embodiment of the invention, a compound of Formula XVIII is treated with trifluoroacetic acid to give the compound of Formula XIV (Scheme III). 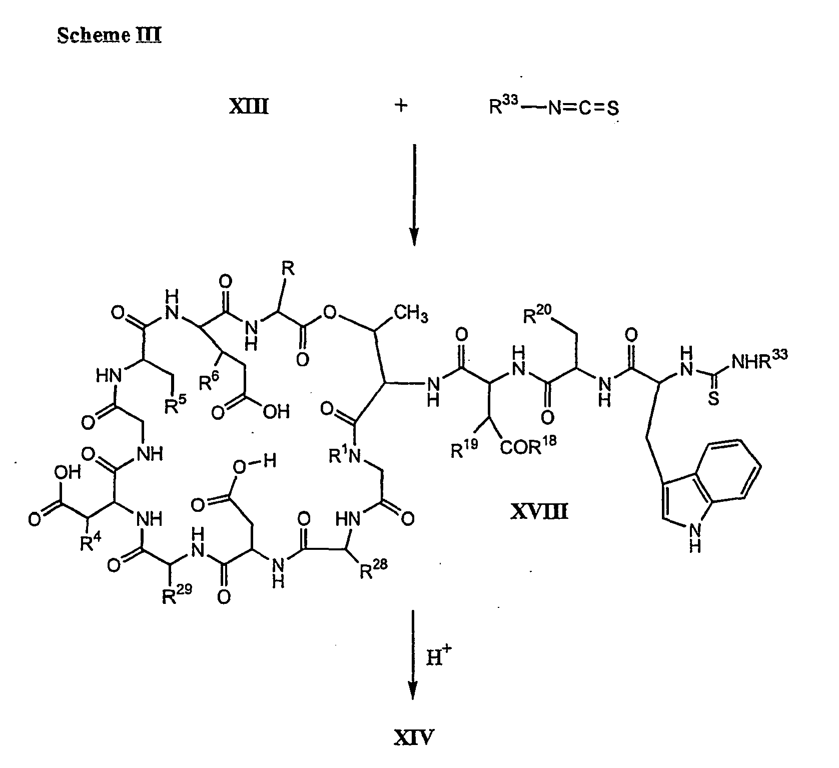 wherein each of R, R¹, R⁴, R⁵, R⁶, R¹⁸, R¹⁹, R²⁰, R²⁸, R²⁹ and R³³ is as described previously.

In a preferred embodiment, R³³ is phenyl, n-decyl, nonyl or octyl. In a more preferred embodiment, R³³ is n-decyl.

Removal of the terminal amino acid residue from the compound of Formula XIV leads to the formation of the desdipeptide compound of Formula XV (step(e)). Methods for removal of the terminal amino acid residue are known to those skilled in the art. A preferred method for removal of the terminal amino acid residue is under Edman degradation conditions *(vide supra).*

In a preferred embodiment, the thiourea peptide (the compound of Formula XIX) formed upon reaction of the thioisocyanate with a compound of Formula XIV, is treated under acidic conditions to provide desdipeptide compound of Formula XV. In a preferred embodiment of the invention, a compound of Formula XIX is treated with trifluoroacetic acid to give the compound of Formula XV (Scheme IV). 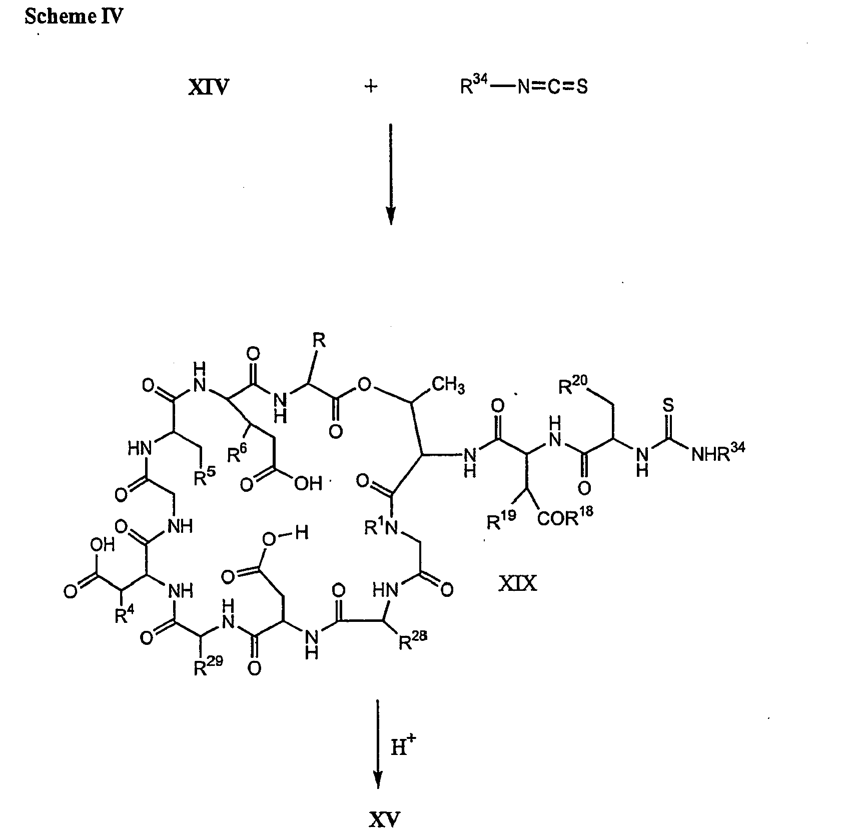 wherein each of R, R¹, R⁴, R⁵, R⁶, R¹⁸, R¹⁹, R²⁰, R²⁸, and R²⁹ is as described previously; and R³⁴ is alkyl or aryl.

Removal of the terminal amino acid residue from the compound of Formula XV leads to the formation of the depsipeptide core compound of Formula XVI (step(f)). Methods for removal of the terminal amino acid residue are known to those skilled in the art. A preferred method for removal of the terminal amino acid residue is under Edman degradation conditions (*vide supra*).

In a preferred embodiment, the thiourea peptide (the compound of Formula XX) formed upon reaction of the thioisocyanate with a compound of Formula XV, is treated under acidic conditions to provide the depsipeptide core compound of Formula XVI. In a preferred embodiment of the invention, a compound of Formula XX is treated with trifluoroacetic acid to give the compound of Formula XVI (Scheme IV A). 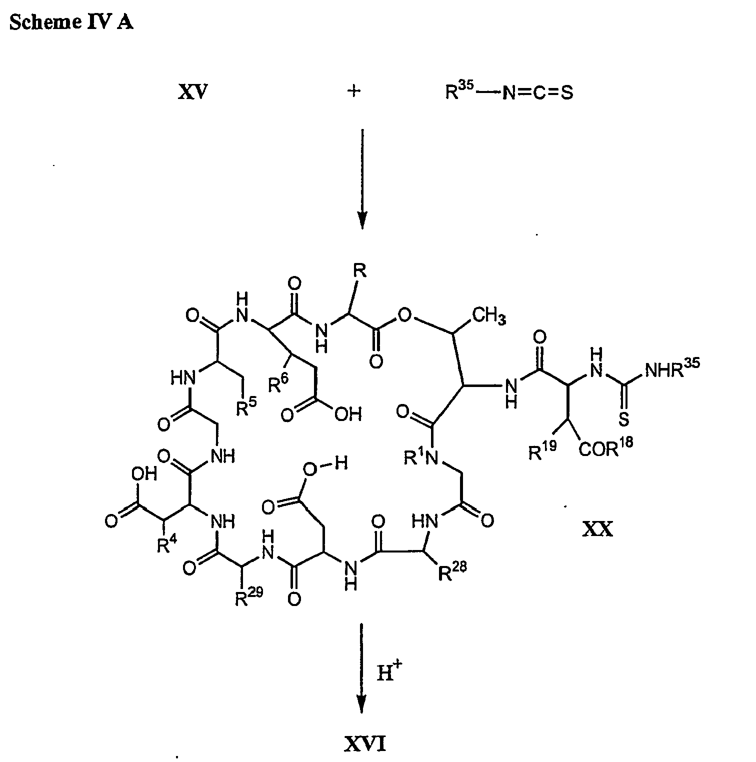 wherein each of R, R¹, R⁴, R⁵, R⁶, R¹⁸, R¹⁹, R²⁰, R²⁸, and R²⁹ is as described previously; and R³⁵ is alkyl or aryl.

Treatment of the depsipeptide core compound of Formula XVI with a modifying agent results in the formation of protected Formula I compound (the compound of Formula XVII, step(f)). The reaction of an amine with modifying agents, as defined herein, is well known to those skilled in the art. For example, treatment of a compound of Formula XVI with an isocyanate gives compounds of Formula XVII in which R⁷ is ureido. Similarly, treatment of a compound of Formula XVI with an activated ester, lactone or acid chloride yields compounds of Formula XVII in which R⁷ is acylamino. Treatment of a compound of Formula XVI with a sulfonyl chloride or activated sulfonamide results in Formula XVII in which R⁷ is a sulfonamino. Treatment of a compound of the Formula XVI with an activated heterocycle results in a compound of Formula XVII in which R⁷ is a heterocyclic amino. Treatment of a compound of the Formula XVI with an activated heteroaryl results in a compound of Formula XVII in which R⁷ is a heteroaryl amino. Treatment of a compound of Formula XVI with a carbonate, chloroformate, or cyanoformate gives compounds of Formula XVII in which R⁷ is a carbamate. Treatment of a compound of Formula XVI with a thioester gives compounds of Formula XVII in which R⁷ is thioacylamino. Treatment of a compound of Formula XVI with a phosphoryl chloride or phosphoramidate, gives compounds of Formula XVII in which R⁷ is phosphonamino. Treatment of a compound of Formula XVI with an imidate gives compounds of Formula XVII in which R⁷ is iminoamino. Treatment of a compound of Formula XVI with a thioisocyanate gives compounds of Formula XVII in which R⁷ is thioureido. Treatment of a compound of Formula XVI with an an aldehyde or ketone under reductive conditions gives compounds of Formula XVII in which R⁷ is a monosubstituted amino or a disubstituted amino group. Treatment of a compound of Formula XVI with an imidate gives compounds of Formula XVII in which R⁷ is iminoamino. Treatment of a compound of Formula XVI with an guanidinylating agent such as 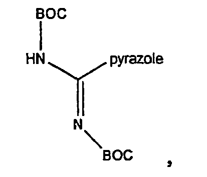 provides compounds of Formula XVII in which R⁷ is guanidino.

It will be understood by those skilled in the art that if a modifying agent contains substituents that are incompatible with the reaction conditions under which the compound of Formula XVII is formed, said substituents will have to be protected prior to use in the reaction. Suitable protecting groups and methods of making them can be found in Greene *(vide supra).*

The reaction of amines of complex molecules such as daptomycin and related depsipeptides can be found in United States Patents 4,399,067; 4,482,487; and 4,537,717; 5,039,789; and 5,028,590; and international Patent Application Serial Numbers WO01/44272, WO01/44274, and WO01/44271.

In a preferred embodiment of the reaction the modifying agent is an activated ester. In a more preferred embodiment of the reaction the modifying agent is 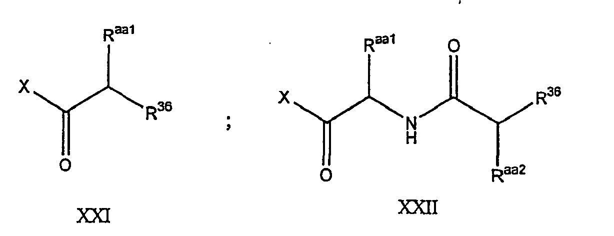 or 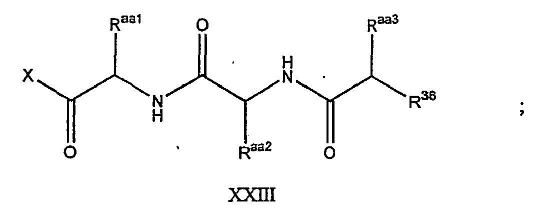 wherein each ofR^{aa1}, R^{aa2}, and R^{aa3} is an amino acid side chain or a protected form of an amino acid side chain; R³⁶ is amino, monosubstituted amino, disubstituted amino, acylamino, ureido, guanidino, carbamoyl, sulfonamino, thioacylamino, thioureido, iminoamino, or phosphonamino; and X is an activating group. In an even more preferred embodiment, X is an aryloxy group. In a still more preferred embodiment, X is pentafluorophenoxy.

Compounds of Formulas XXI, XXII and XXIII can be prepared from the corresponding peptide or amino acid upon treatment with an activating agent such as anhydrides, chloroformates, pentafluorophenol/dicyclohexylcarbodiimide, N',N'-carbonyldiimidazole, hydroxybenzotriazole or N-hydroxysuccinimide. The peptides can be prepared by any standard peptide procedure. For an overview of some standard peptide formation procedures see Vogel's Textbook of Practical Organic Chemistry. 5th Ed., eds. B.S. Furniss, A.J. Hannaford; P.W.G. Smith; A.R. Tatchell (New York: John Wiley and Sons, Inc.), 1989, pp750-763 and Introduction to Organic Chemistry, 2nd Ed. by A. Streitwieser, Jr. and C.H. Heathcock (New York: MacMillan Publishing Co., Inc.), pp 954-962. Other methods that are useful for the preparation of peptides of the present invention involve synthesis on a solid support. Specific examples of such procedures are detailed in the examples (*vide infra*).

Removal of the protecting group(s) from the protected Formula I compound (the compound of Formula XVII) results in the formation of the compound of Formula Ia (step(h)) wherein, R¹, R⁴, R⁵, R⁶, and R⁷ are as previously described ; R^{2a} is methyl or -CH₂CH₂CH₂NH₂, and R^{3a} is a methyl or -CH₂CH₂CH₂CH₂NH₂. Removal of the amino protecting group can be accomplished according to procedures described in Greene *(vide supra).* As those skilled in the art will recognize, the choice of amino protecting group employed in the first step of the process will dictate the reagents and procedures used in removing said amino protecting group.

When the modifying agent contains one or more protecting group(s), said protecting group(s) must also be removed. The choice of the protecting group(s) utilized on the modifying agent substituent(s) will dictate the reagents and procedures used in removing said protecting group(s). When the protecting group(s) utilized on the modifying agent substituent(s) and the protecting group utilized in step (b) are compatable, the protecting groups may be removed in a single step. However, when the protecting group(s) are incompatable multiple steps may be required to remove all of the protecting groups.

### Process for the preparation of compounds of Formula I wherein at least one of R² and R³ is other than methyl and each of R⁸ and R⁹ is other than NH₂.

### Procedure B

For compounds of Formula I wherein at least one of R² and R³ is other than methyl and each of R⁸ and R⁹ is other than NH₂, the process, in accordance with another aspect of the invention, comprises the additional steps of:
(i) treating the Formula I free amine compound of step(h) of Procedure A with a modifying agent to obtain a compound of Formula I.

Treatment of the Formula I free amine compound of Formula Ia with a modifying agent are well know to those skilled in the art and are described for step(g) of Procedure A (*vide supra*).

The reaction of free amines of complex molecules such as daptomycin and related depsipeptides can be found in United States Patents 4,399,067; 4,482,487; and 4,537,717; and International Patent Application Serial Numbers WO01/44272, WO01/44274, and WO01/44271.

It will be understood by those skilled in the art that if R⁷ or the modifying agent of step (i) contain substituents that are incompatible with the reaction conditions under which the compound of Formula I is formed, said substituents will have to be protected prior to step (i). Suitable protecting groups and methods of making them can be found in Greene *(vide supra*).

When R⁷, and the modifying agent of step(i) contain protecting group(s), said protecting group(s) may be removed. The choice of the protecting group(s) utilized on R⁷ and the modifying agent substituent(s) will dictate the reagents and procedures used in removing said protecting group(s). When the protecting group(s) utilized on R⁷, and the modifying agent substituent(s) are compatable, the protecting groups may be removed in a single step. However, when the protecting group(s) are incompatable multiple steps may be required to remove all of the protecting groups.

### Procedure C

An alternate procedure for preparing compounds of Formula I wherein at least one of R² and R³ is other than methyl and each of R⁸ and R⁹ is other than NH₂, comprises the steps of:
(a) providing a depsipeptide derivative of the Formula XI 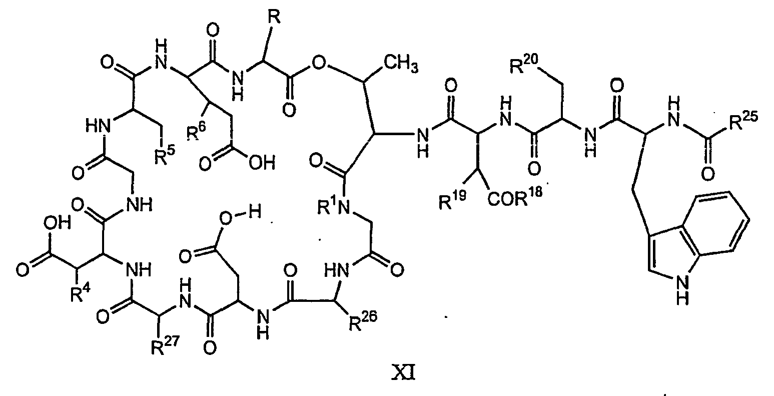 wherein: R, R¹, R⁴, R⁵, R⁶, R¹⁸, R¹⁹, R²⁰, R²⁵, R²⁶, and R²⁷ are as previously described;
(b) treating the free amino group(s) of the compound of Formula XI with a modifying agent to obtain a blocked depsipeptide compound wherein said modifying agent is selected such that the blocked depsipeptide compound formed is stable to step (c), (d), (e) (f) and (g);
(c) treating the blocked depsipeptide compound obtained in (b) with a deacylating agent to obtain a terminal amino compound;
(d) removing the tryptophan amino acid residue of the terminal amino compound obtained in (c) to obtain a destryptophan compound;
(e) removing the terminal amino acid residue of the destryptophan compound obtained in (d) to obtain a desdipeptide compound ;
(f) removing the terminal amino acid residue of the desdipeptide compound obtained in (e) to obtain a depsipeptide core compound;
(g) treating the depsipeptide core compound of (f) with a modifying agent Procedure C is described in Scheme V

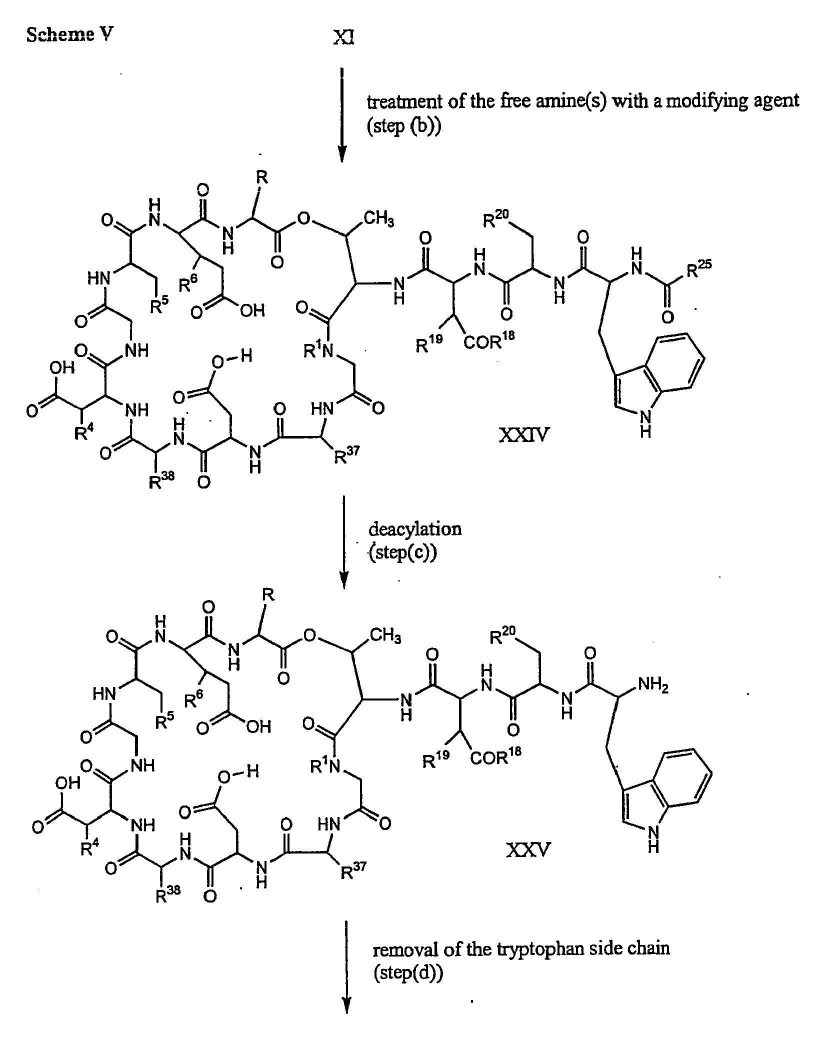

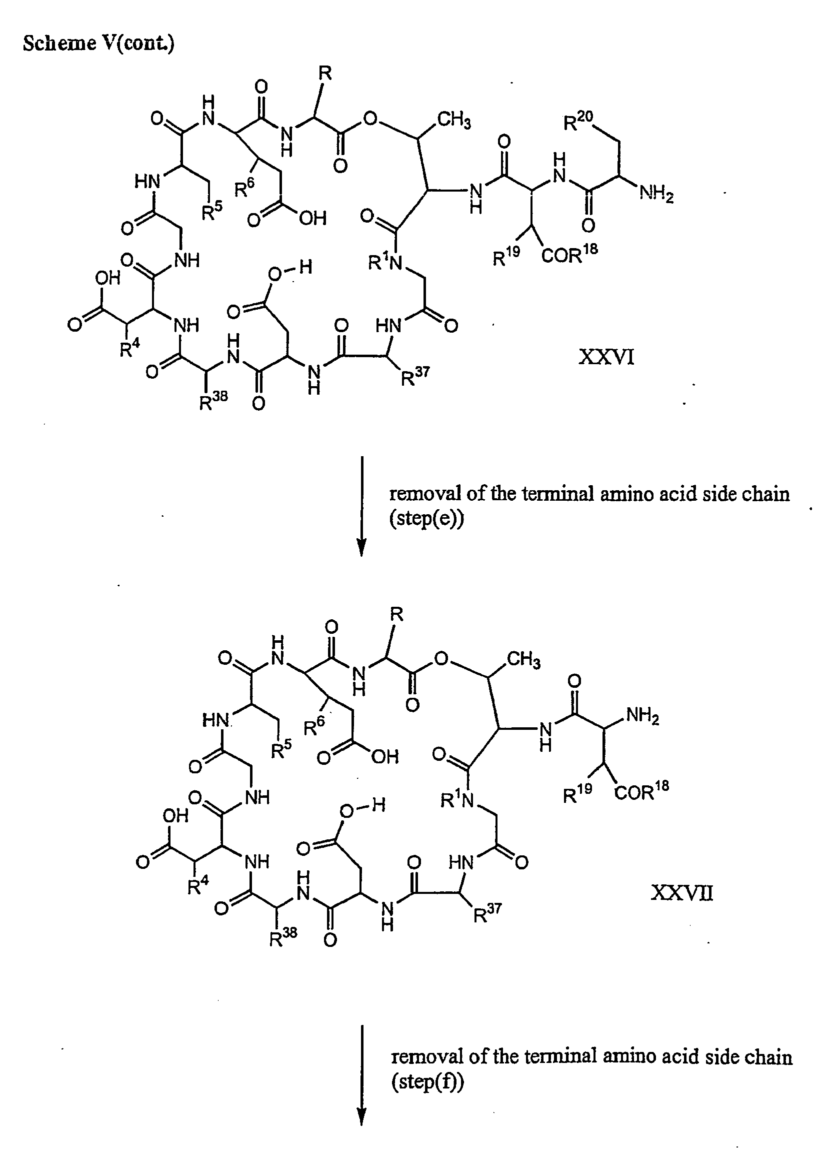

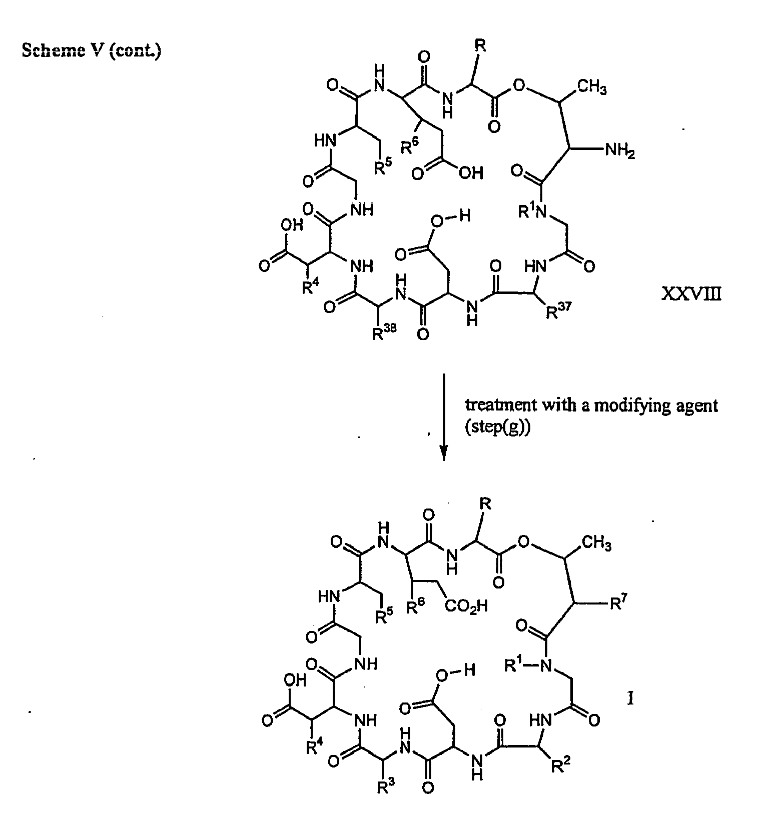

Treatment of the compound of Formula XII with a modifying agent results in the formation of blocked Formula I compound (the compound of Formula XXIV, step(b)) wherein R³⁷ is methyl or -CH₂CH₂CH₂R⁸; and R³⁸ is methyl or -CH₂CH₂CH₂CH₂R⁹; provided that at least one of R³⁷ and R³⁸ must be other than methyl and R⁸ and R⁹ must be other than amino.. The formation of the blocked Formula I compound is performed as previously described in step (g) of Procedure A (*vide supra*)

The deacylation of compound XXIV lead to the formation of the terminal amino compound XXV. Suitable agents for the deacylation of compounds of Formula XXIV are enzymatic deacylated agents (*vide supra*)*.*

Removal of the tryptophan amino acid residue from the terminal amino compound of Formula XXV leads to the formation of the compound of Formula XXVI (step(d)). Methods for removal of the tryptophan amino acid residue are known to those skilled in the art. A preferred method for removal of the tryptophan amino acid residue is under Edman degradation conditions *(vide supra).*

Removal of the terminal amino acid residue from the compound of Formula XXVI leads to the formation of the desdipeptide compound of Formula XXVII (step(e)). Methods for removal of the terminal amino acid residue are known to those skilled in the art. A preferred method for removal of the terminal amino acid residue is under Edman degradation conditions *(vide supra*).

Removal of the terminal amino acid residue from the compound of Formula XXVII leads to the formation of the depsipeptide core compound of Formula XXVIII (step(f)). Methods for removal of the terminal amino acid residue are known to those skilled in the art. A preferred method for removal of the terminal amino acid residue is under Edman degradation conditions *(vide supra).*

Treatment of the depsipeptide compound of Formula XXVIII with a modifying agent are well know to those skilled in the art and are described for step(g) of Procedure A *(vide supra).*

It will be understood by those skilled in the art that if the modifying agent, contain substituents that are incompatible with the reaction conditions under which the compound of Formula I is formed, said substituents must be protected prior to reaction with the compound of Formula XXVIII. Suitable protecting groups and methods of making them can be found in Greene *(vide supra*).

When desired, protecting groups may be removed. When the protecting group(s) are compatable, the protecting groups may be removed in a single step. However, when the protecting group(s) are incompatable multiple steps may be required to remove all of the protecting groups.

### Procedure D

Compounds of Formula I and compounds of Formula Ia in which R⁷ is 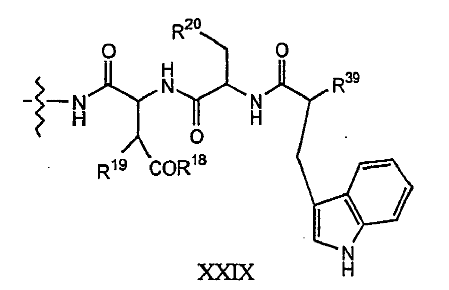 wherein each of R¹⁸, R¹⁹, and R²⁰ is as previously described and R³⁹ is amino, monosubstituted amino, disubstituted amino, acylamino, ureido, guanidino, carbamoyl, sulfonamino, thioacylamino, thioureido, iminoamino, or phosphonamino; can be prepared from compound XIII, as described in Scheme VI, or by compound XXV as described in Scheme VII.

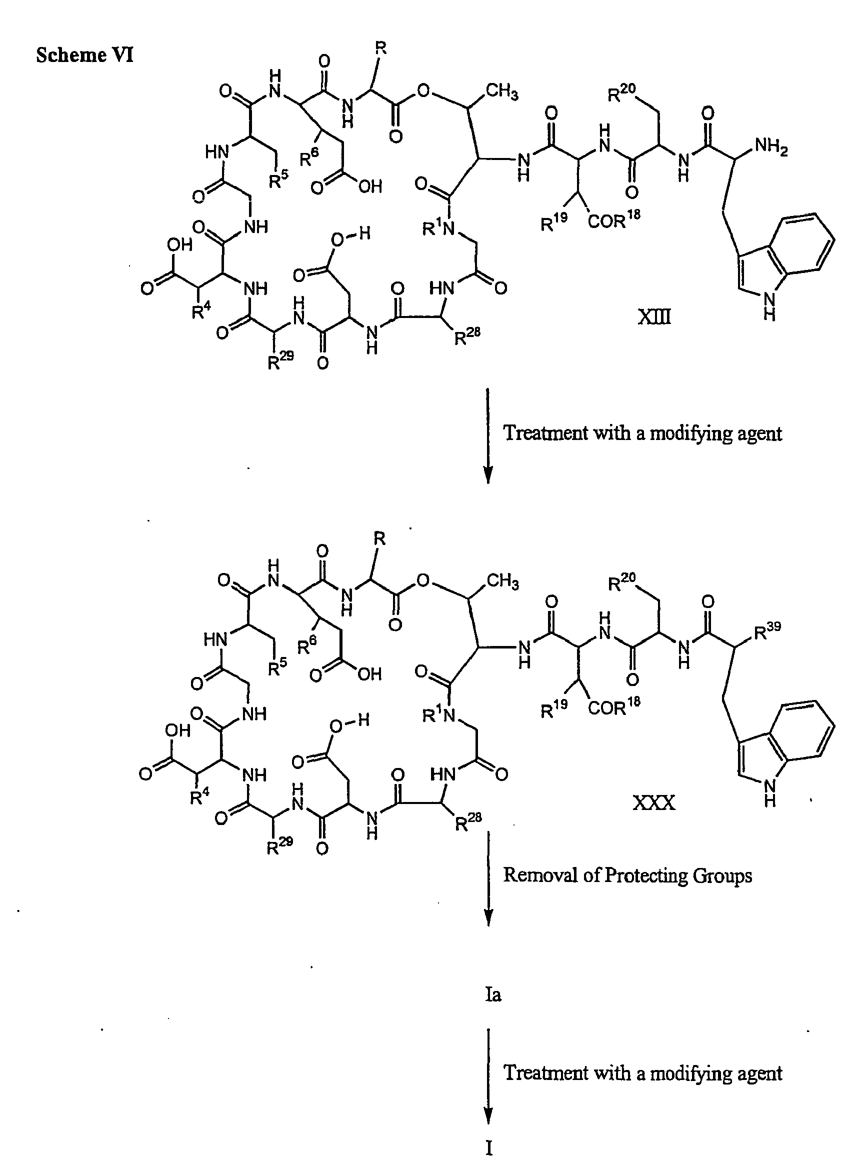

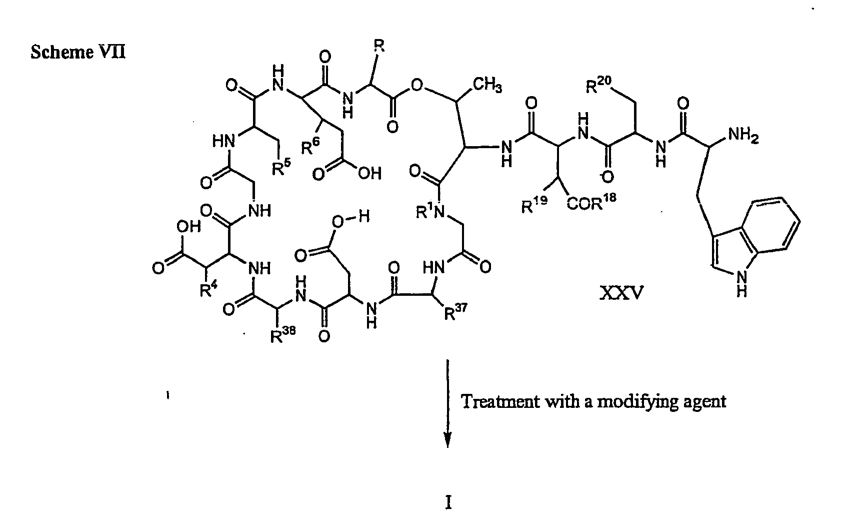

### Procedure E

Similarly compounds of Formula I and compounds of Formula Ia in which R⁷ is 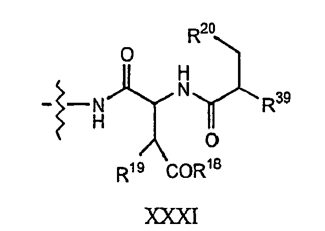 wherein each of R¹⁸, R¹⁹, and R²⁰ is as previously described and R³⁹ is amino, monosubstituted amino, disubstituted amino, acylamino, ureido, guanidino, carbamoyl, sulfonamino, thioacylamino, thioureido, iminoamino, or phosphonamino; can be prepared from compound XIV, as described Scheme VIII, or by compound XXVI as described in Scheme IX.

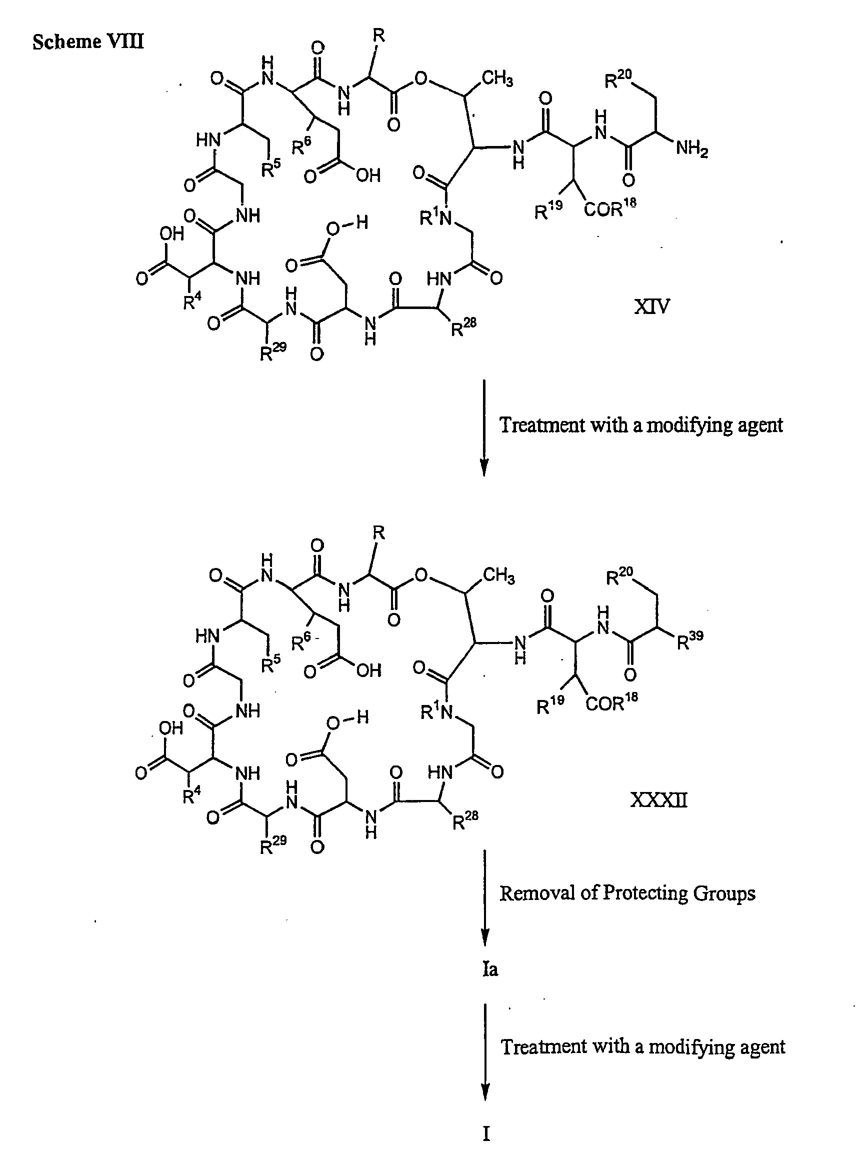

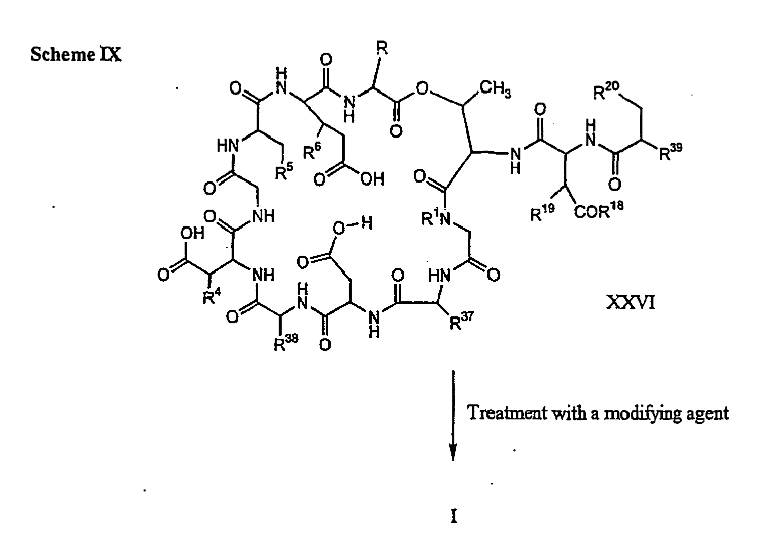

### Procedure F

Similarly compounds of Formula I and compounds of Formula Ia in which R⁷ is 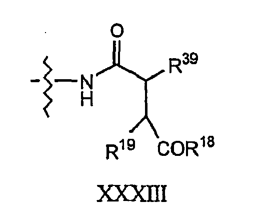 wherein each of R¹⁸, and R¹⁹ is as previously described and R³⁹ is amino, monosubstituted amino, disubstituted amino, acylamino, ureido, guanidino, carbamoyl, sulfonamino, thioacylamino, thioureido, iminoamino, or phosphonamino; can be prepared from compound XV, as described Scheme X, or by compound XXVII as described in Scheme XI.

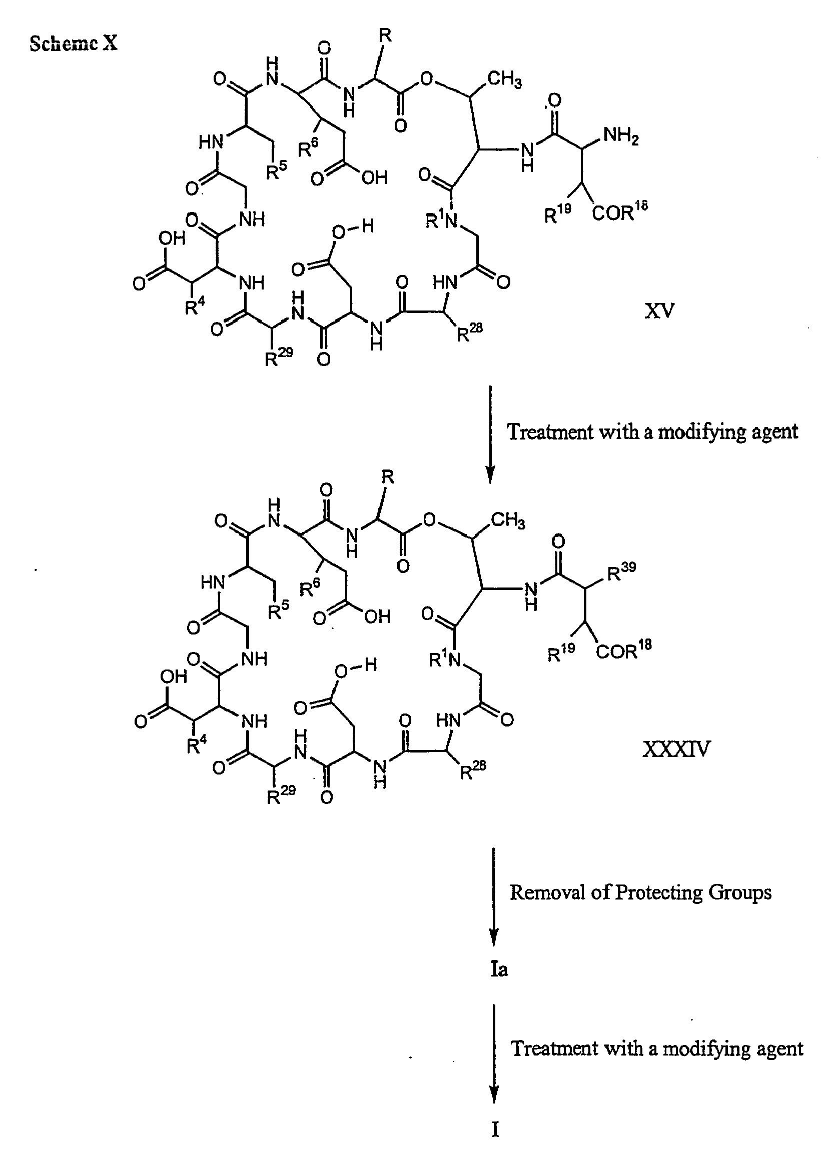

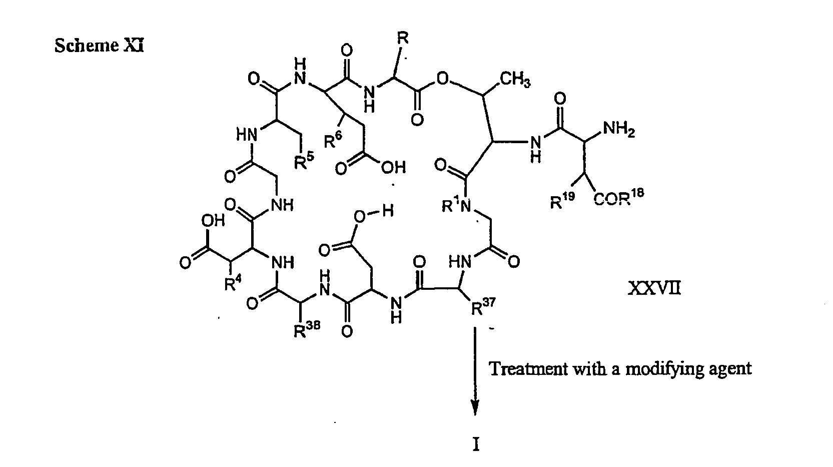

### 2. Synthetic Process

### Solid Support Synthesis of Depsipeptide Compounds

In an alternative embodiment of the invention, the depsipeptide compounds of Formula I may be synthesized on a solid support (Scheme XII, Scheme XIII and Scheme XV).

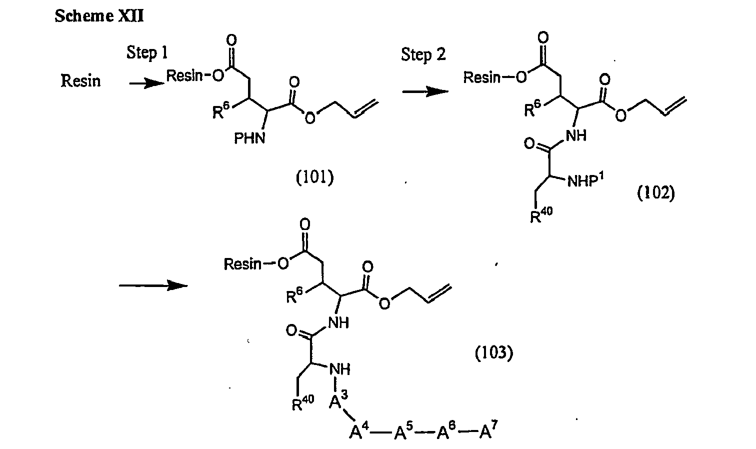

In step 1, an N-protected-β-methyl glutamic acid-O-allyl ester or a protected glutamic acid-O-allyl ester is coupled to a resin to give Compound 101, wherein R⁶ is as described previously. A resin or solid support, such as, but not limited to, Wang, HMPA, Safety Catch, Rink Acid, 2-chlorotrityl-chloride resin, trityl-chloride resin, 4-methyltrityl-chloride resin, 4-methoxytrityl-chloride resin or PAM resin may be used in this reaction.

Deprotection of Compound 101, followed by coupling of the free amino with a protected serine or protected asparagine affords Compound 102, wherein R⁴⁰ is -OP², or -CONHP³; and each ofP, P¹, P² and P³ is independently a protecting group. This peptide coupling process, i.e., deprotection of the alpha-amino group, followed by coupling to a protected amino acid, is repeated until the desired number of amino acids has been coupled to the resin. In Scheme XII, a total of seven amino acids have been coupled to give compound 103 wherein, A³ is 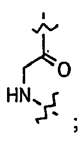 A⁴ is 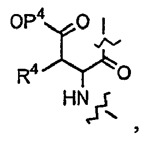 wherein P⁴ is a protecting group and R⁴ is as previously described ; A⁵
is 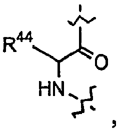 wherein R⁴⁴ is methyl or-CH₂CH₂CH₂CH₂NP⁵, wherein P⁵ is an
amino protecting group; A⁶ is 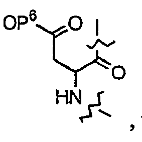 wherein P⁶ is a protecting group; and A⁷
is 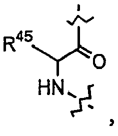 wherein R⁴⁵ is methyl or-CH₂CH₂CH₂NP⁷, wherein P⁷ is an amino protecting group. A second peptide is coupled to a resin in a similar fashion, as outlined in Scheme XIII.

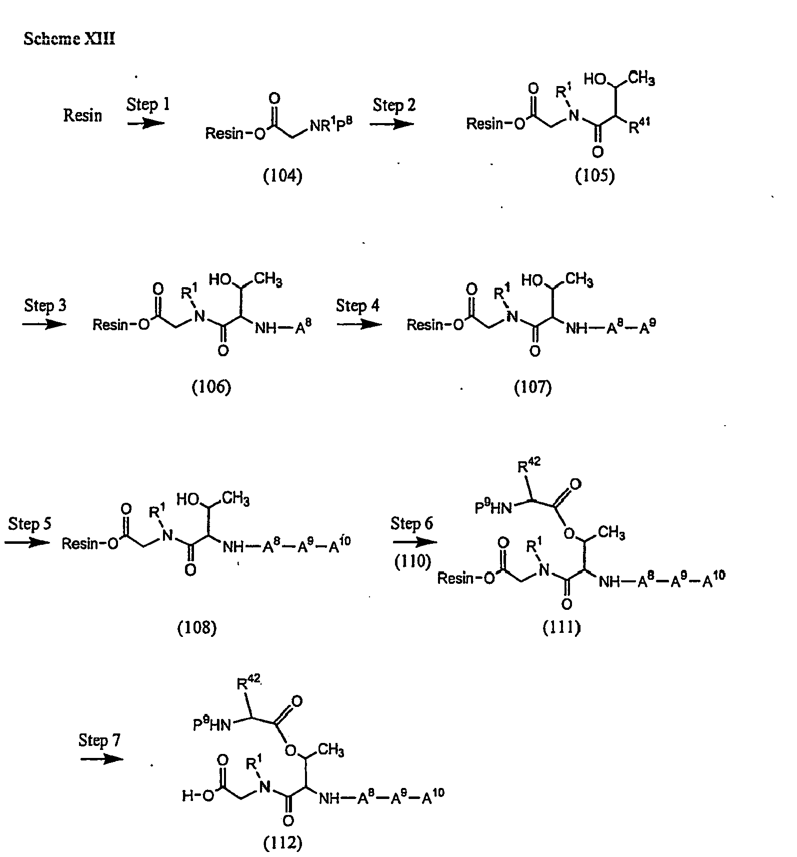

In step 1, a N-protected-glycine or a sarcosine is coupled to a resin to give Compound 104 wherein R¹ is as described previously and P⁸ is an amino protecting group. The choice of resin used in step 1 is dependent upon the nature of the amino acid that is coupled in steps 2-6. If the amino acid side chains contain protecting groups, a resin must be chosen such that the protecting groups remain intact when the resin is removed from the peptide in step 7. Resins that can be cleaved while preserving the protecting groups of peptides include, but are not limited to, Safety Catch, Rink Acid, 2-chlorotrityl-chloride resin, trityl-chloride resin, 4-methyltrityl-chloride resin, 4-methoxytrityl-chloride resin or PAM resin.

Deprotection of the protected amino of Compound 104, followed by coupling of the free amino with 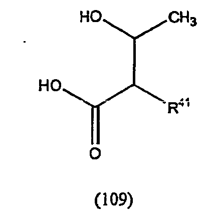 affords Compound 105, wherein R⁴¹ is amino, monosubstituted amino, disubstituted amino, acylamino, ureido, guanidino, carbamoyl, sulfonamino, thioacylamino, thioureido, iminoamino, or phosphonamino.

When R⁴¹ is amino, this peptide coupling process, i.e., deprotection of the alpha-amino group, followed by coupling to a protected amino acid, is repeated until the desired number of amino acids has been coupled to the resin. In Scheme XIII, five amino acids have been coupled to give Compound 108 wherein, A⁸ is 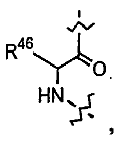 wherein R⁴⁶ is hydrido, an amino acid side chain or a protected amino acid side chain; A⁹ is 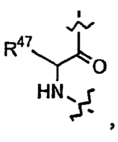 wherein R⁴⁷ is hydrido, an amino acid side chain or a protected amino acid side chain; A¹⁰ is 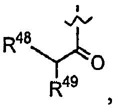 wherein R⁴⁸ is hydrido, an amino acid side chain or a protected amino acid side chain and R⁴⁹ is amino, monosubstituted amino, disubstituted amino, acylamino, ureido, guanidino, carbamoyl, sulfonamino, thioacylamino, thioureido, iminoamino, or phosphonamino. Compound 108 is coupled with 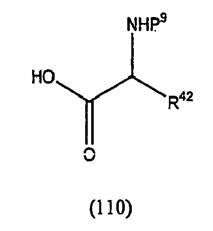 to give compound 111, wherein P⁹ is a protecting group and R⁴² is 2-butyl, isopropyl or 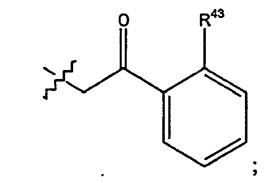 wherein R⁴³ is a group capable of being converted to an amino group. For example, R⁴³ may be azido, a protected amino phthalimido or nitro.

The peptide 111 is then removed from the resin to give compound 112.

Coupling of the peptide fragments 103 and 112 is outlined in Scheme XIV

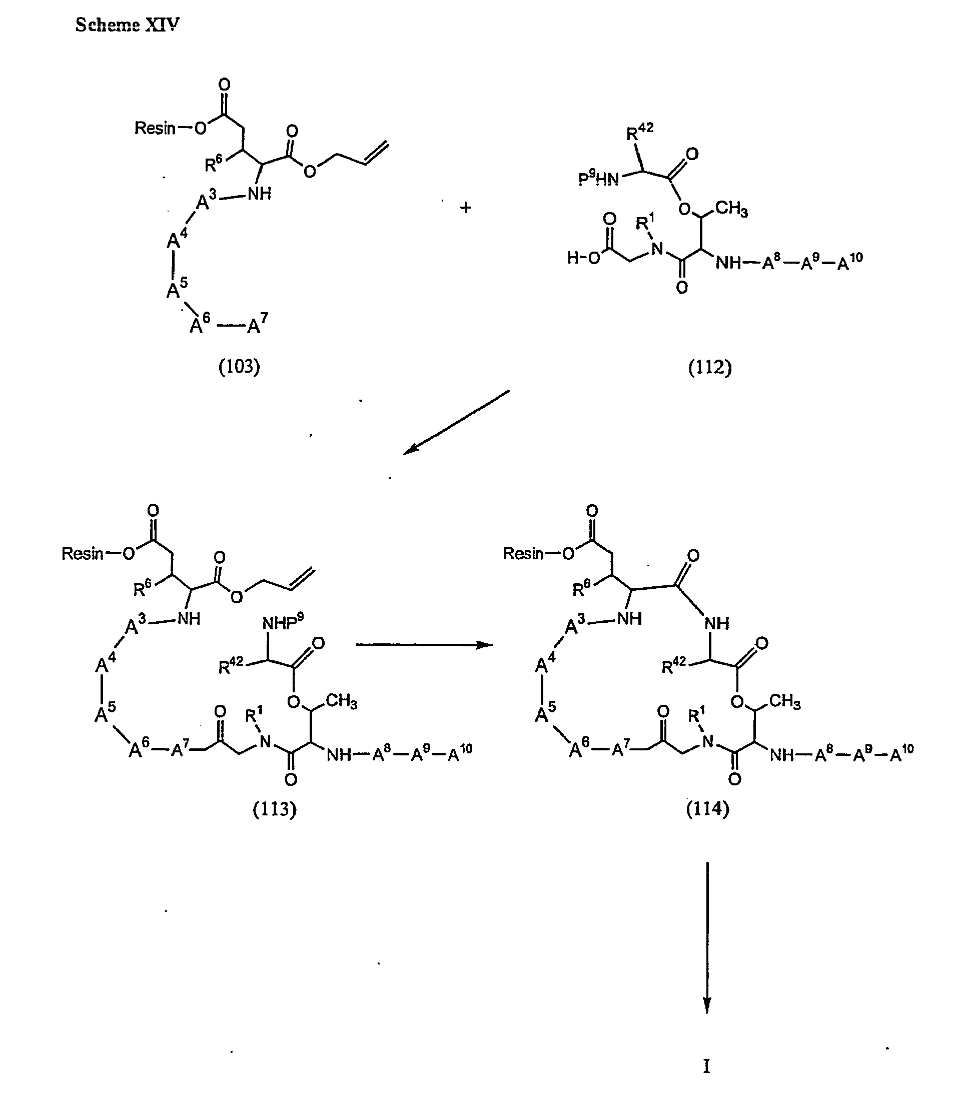

The peptide fragments 103 and 112 are coupled to yield the resin bound peptide 113. Deprotection of the P⁹ protecting group and O-allyl ester, followed by cyclization affords a resin-bound depsipeptide 114. Cleavage of the depsipeptide from the resin followed by deprotection of any remaining protecting groups yields compounds of Formula I (Scheme XVI).

When compounds of Formula I are desired in which there are no exocyclic amino acids, compound 105 can be used in place of compound 108 (Scheme XV). Compound 105 is coupled with Compound 110 to give Compound 115; removed from the resin to give Compound 116; coupled with Compound 103 to give Compound 117; then deprotected and cyclized to yield a resin bound depsipeptide (118) and cleaved from the resin as described previously in Schemes XIII and XIV (*vide supra*).

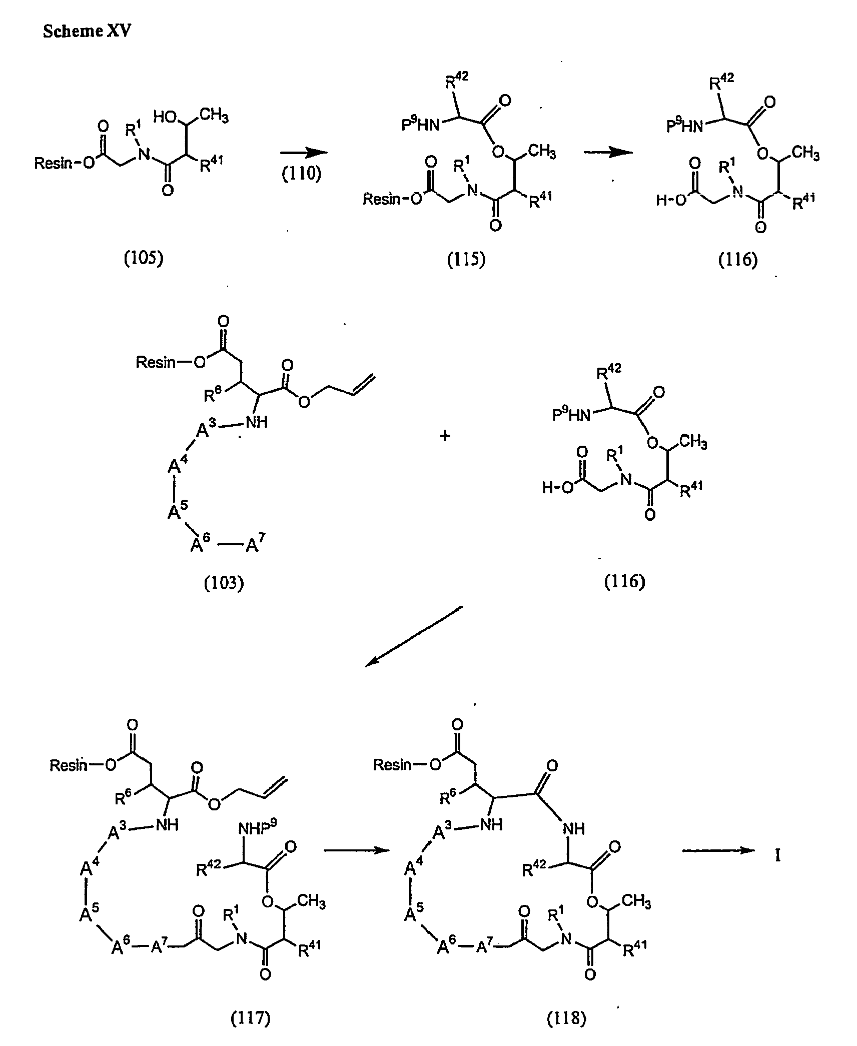

When compounds of Formula I are desired in which there is one exocyclic amino acids, compound 106 can be used in place of compound 108 (Scheme XVII). Compound 106 is coupled with Compound 110 to give Compound 119; removed from the resin to give Compound 120; coupled with Compound 103 to give Compound 121; then deprotected and cyclized to yield a resin bound depsipeptide (122) and cleaved from the resin as described previously in Schemes XIII and XIV (*vide supra*)*.*

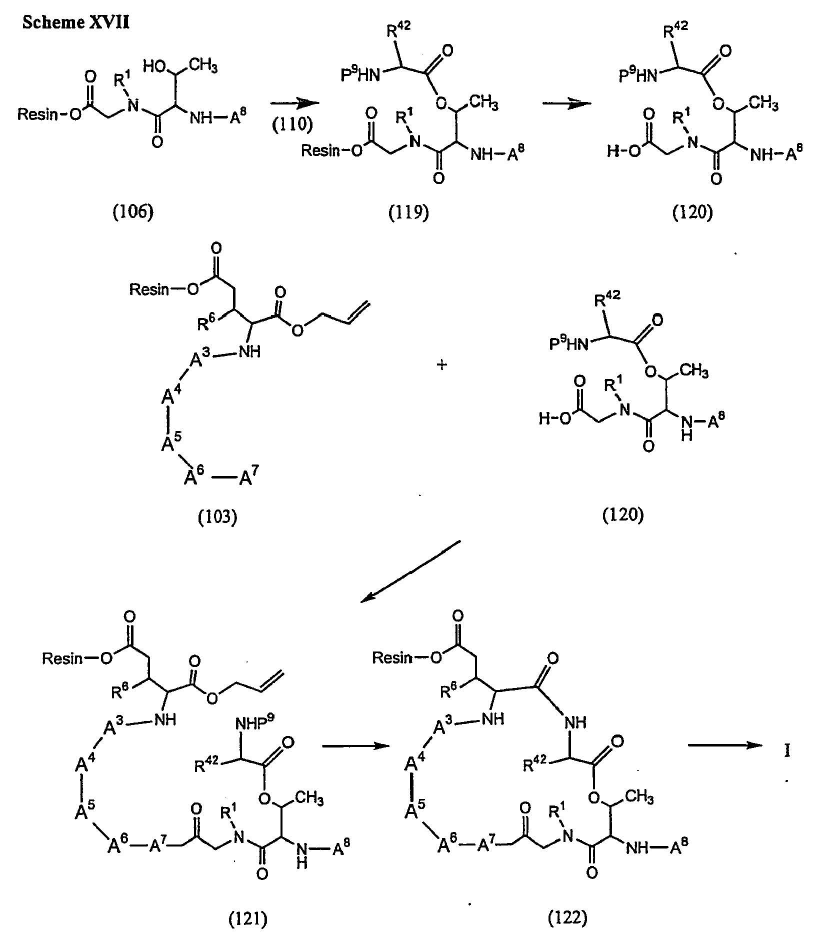

When compounds of Formula I are desired in which there are two exocyclic amino acids, compound 107 can be used in place of compound 108 (Scheme XVIII). Compound 106 is coupled with Compound 110 to give Compound 123; removed from the resin to give Compound 124; coupled with Compound 103 to give Compound 125; then deprotected and cyclized to yield a resin bound depsipeptide (126) and cleaved from the resin as described previously in Schemes XIII and XIV (*vide supra*)*.*

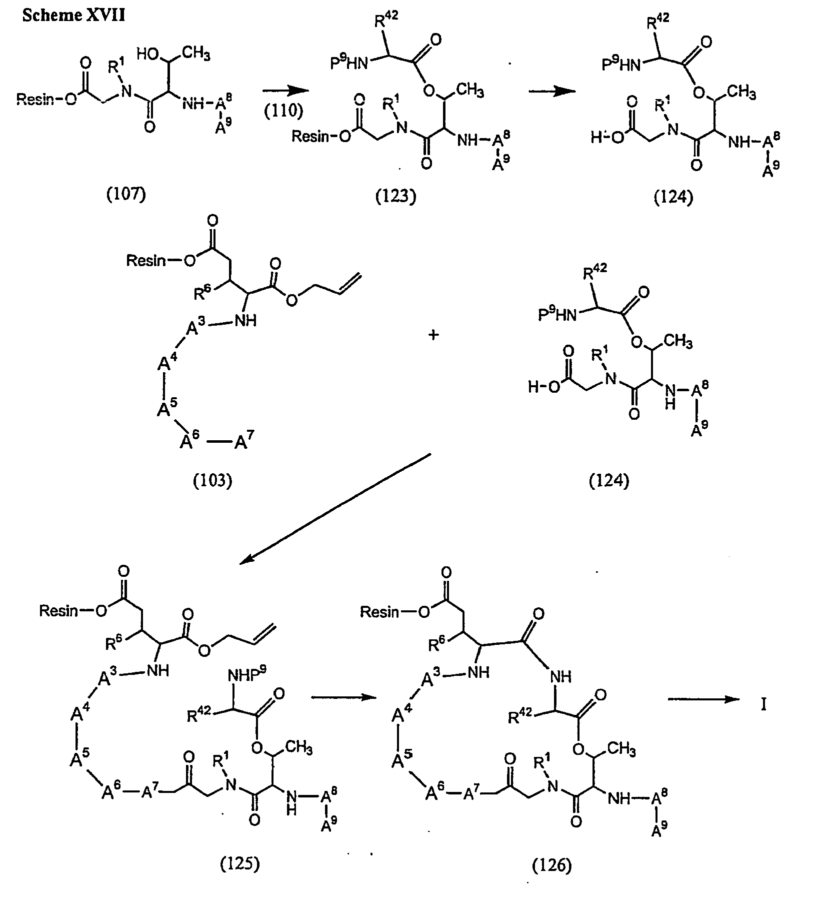

Following the synthetic schemes above (Schemes XII-XVII), it is understood that both the amino acid amino group and the amino acid side chain functional groups must be orthogonally protected prior to attaching them to the growing peptide chain. Suitable protecting groups can be any amino protecting group useful in peptide synthesis. Such pairings of protecting groups are well known. See, e.g., "Synthesis Notes" in the Novabiochem Catalog and Peptide Synthesis Handbook (1999), pages S1-S93 and references cited therein.

It will also be understood by those skilled in the art that the choice of protecting group on the amino acid side chain functional groups will either result or not result in the protecting group being cleaved concomitantly with the peptide's final cleavage from the resin, which will give the natural amino acid functionality or a protected derivative thereof, respectively. When the protecting groups are not concomitantly cleaved when the depsipeptide is cleaved from the resin, additional deprotection may be necessary

### 3. Biosynthetic Process

The compounds of the present invention can also be prepared by recombinant methods. In this process a modified non-ribosomal peptide synthetase of daptomycin is introduced into a cell capable of producing a compound of Formula I and the cell is cultured to form a compound of Formula I. The non-ribosomal peptide synthetases of daptomycin as well as modifications of these synthetases have been reported (see International Patent Application Serial Number 02/059,322).

In order that this invention may be more fully understood, the following examples are set forth. These examples are for the purpose of illustration only and are not to be construed as limiting the scope of the invention in any way.

### Example 1: Synthesis of Peptide Resin Compound 1:

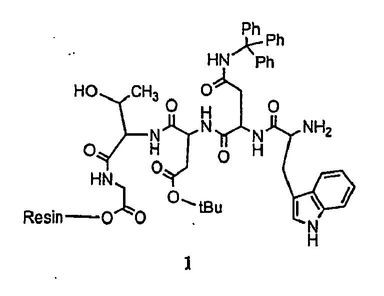

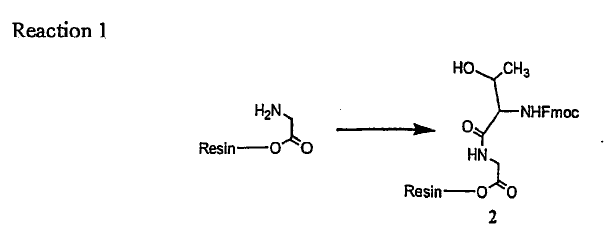

The commercially available Nα-(9-Fluorenylmethoxycarbonyl)-L-threonine (2 mL of a 0.5 molar solution in N-methylpyrolidine), 1,3-diisopropylcarbodiimide (2 mL of a 0.5 molar solution in N-methylpyrolidine), and 1-hydroxy-benzotriazole (2 mL of a 0.5 molar solution in N-methylpyrolidine) was added to commercially available glycine 2-chlorotrityl resin (334 mg). The mixture was shaken for one hour, filtered and a few beads were tested for the presence of a free amine using the standard Kaiser test (see E. Kaiser, et al (1970) Anal.Biochem., 34, 595 ; and Advanced Chemtech Handbook of Combinatorial, Organic and Peptide Chemistry 2003-2004 page 208). The Kaiser test gave a blue color so the coupling conditions above were repeated. After filtration the product bearing resin was washed with N-methylpyrolidine (3 x 6 mL), methanol (3 x 6 mL), and again with N-methylpyrolidine (3 x 6 mL) to give compound 2. 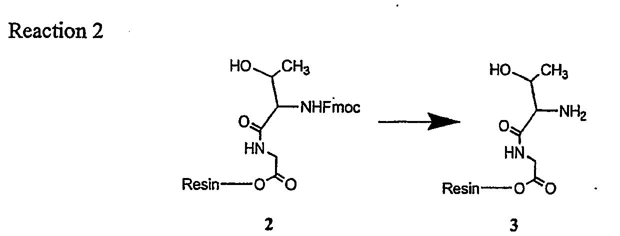 Compound 2 was agitated in 20% piperidine in N-methylpyrolidine (6 mL) for 30 minutes. The resin was filtered and re-suspended in 20% piperidine in N-methylpyrolidine (6 mL) and agitated for 30 minutes. The reaction mixture was filtered then the solid washed with N-methylpyrolidine (3 x 6 mL), methanol (3 x 6 mL), and again with N-methylpyrolidine (3 x 6 mL) to give compound 3. 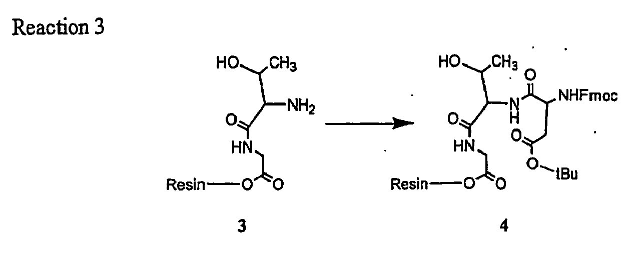 The commercially available Nα-(9-Fluorenylmethoxycarbonyl)-L-aspartic acid β-*tert*-butyl ester (2 mL of a 0.5 molar solution in N-methylpyrolidine), 1,3-diisopropylcarbodiimide (2 mL of a 0.5 molar solution in N-methylpyrolidine), and 1-hydroxy-benzotriazole (2 mL of a 0.5 molar solution in N-methylpyrolidine) were added to compound 3. The mixture was shaken for one hour, filtered and the coupling was repeated. The reaction mixture was filtered then the solid was washed with N-methylpyrolidine (3 x 6 mL), methanol (3 x 6 mL), and again with N-methylpyrolidine (3 x 6 mL) to give compound. 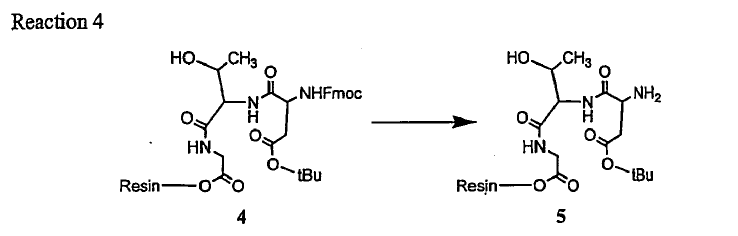 Compound 4 was agitated in 20% piperidine in N-methylpyrolidine (6 mL) for 30 minutes. The resin was filtered and re-suspended in 20% piperidine in N-methylpyrolidine (6 mL) and agitated for 30 minutes. The reaction mixture was filtered then washed with N-methylpyrolidine (3 x 6 mL), methanol (3 x 6 mL), and again with N-methylpyrolidine (3 x 6 mL) to give compound 5. 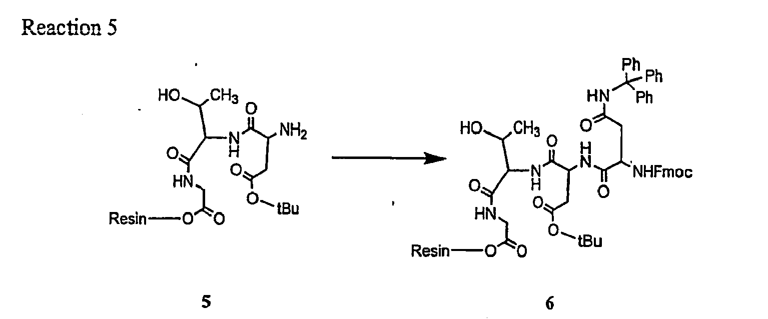 The commercially available Nα-(9-Fluorenylmethoxycarbonyl)-L-asparagine δ-N-trityl (2 mL of a 0.5 molar solution in N-methylpyrolidine), 1,3-diisopropylcarbodiimide (2 mL of a 0.5 molar solution in N-methylpyrolidine), and 1-hydroxy-benzotriazole (2 mL of a 0.5 molar solution in N-methylpyrolidine) was added to resin 5. The reaction mixture was shaken for one hour, filtered and the coupling was repeated. The reaction mixture was filtered then the solid was washed with N-methylpyrolidine (3 x 6 mL), methanol (3 x 6 mL), and again with N-methylpyrolidine (3 x 6 mL) to give compound 6. 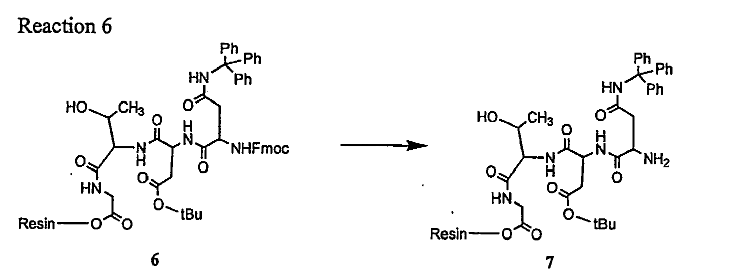 Compound 6 was agitated in 20% piperidine in N-methylpyrolidine (6 mL) for 30 minutes. The resin was filtered and re-suspended in 20% piperidine in N-methylpyrolidine (6 mL) and agitated for 30 minutes. The reaction mixture was filtered then the solid was washed with N-methylpyrolidine (3 x 6 mL), methanol (3 x 6 mL ), and again with N-methylpyrolidine (3 x 6 mL) to give compound 7. 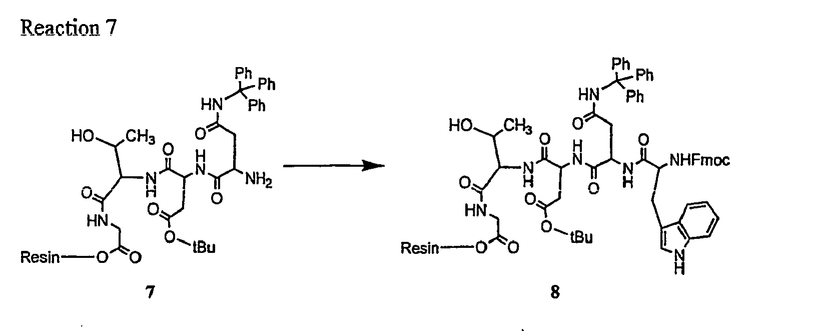 The commercially available Nα-(9-Fluorenylmethoxycarbonyl)-L-tryptophan (2 mL of a 0.5 molar solution in N-methylpyrolidine), 1,3-diisopropylcarbodiimide (2 mL of a 0.5 molar solution in N-methylpyrolidine), and 1-hydroxy-benzotriazole (2 mL of a 0.5 molar solution in N-methylpyrolidine) was added to resin. The reaction mixture was shaken for one hour, then filtered and the coupling was repeated. The reaction mixture was filtered then the solid was washed with N-methylpyrolidine (3 x 6 mL), methanol (3 x 6 mL), and again with N-methylpyrolidine (3 x 6 mL) to give compound 8. 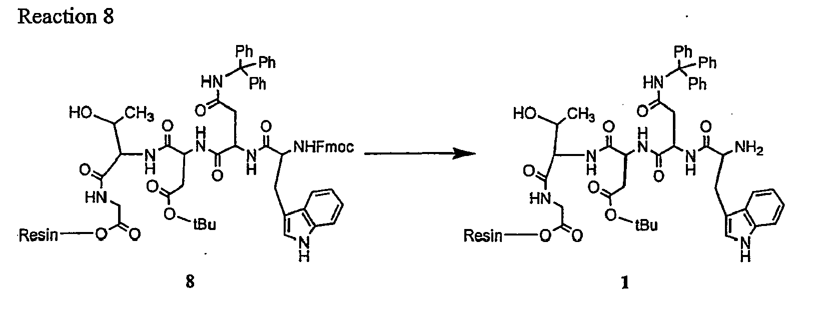 Compound 8 was agitated in 20% piperidine in N-methylpyrolidine (6 mL) for 30 minutes. The resin was filtered and re-suspended in 20% piperidine in N-methylpyrolidine (6 mL) and agitated for 30 minutes. The reaction mixture was filtered then the solid was washed with N-methylpyrolidine (3 x 6 mL), methanol (3 x 6 mL), and again with N-methylpyrolidine (3 x 6 mL) to give resin peptide compound 1.

### Example 2: Synthesis of Peptide Resin Compound 9:

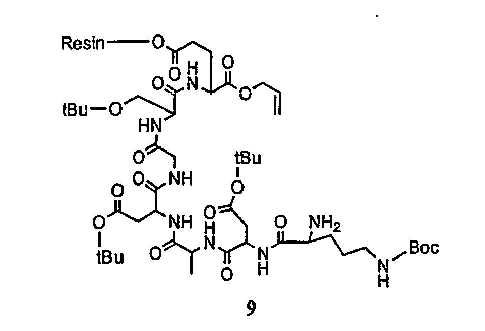

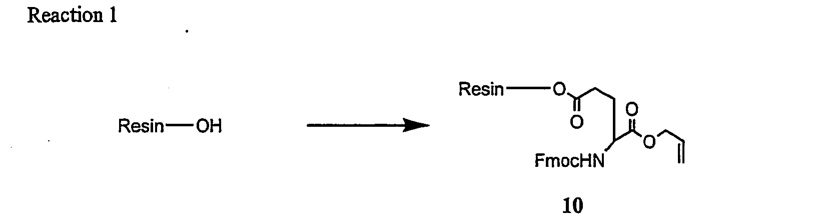

To a suspension of commercially available 4-hydroxymethylphenoxy resin (Wang resin) (5 g, 0.4 mmol/g) in dichloromethane (60 mL) was added 1,3-diisopropylcarbodiimide (0.940 mL), 4-dimethylaminopyridine (24 mg in N-methylpyrolidine (1 mL)), and commercially available Nα-(9-Fluorenylmethoxycarbonyl)-L-glutamic acid α-allyl ester (2.46 g in N-methylpyrolidine (9 mL)). The reaction mixture was stirred for 16 hours, filtered, and the solid was washed with N-methylpyrolidine and dichloromethane and dried to give compound 10.

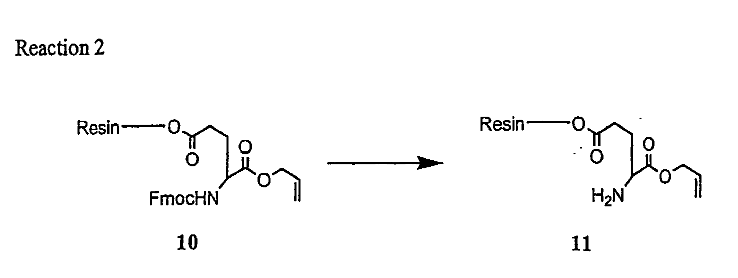

Compound 10 (526 mg) was agitated in 20% piperidine in N-methylpyrolidine (6 mL) for 30 minutes. The resin was filtered and re-suspended in 20% piperidine in N-methylpyrolidine (6 mL) and agitated for 30 minutes. The reaction mixture was filtered then the solid was washed with N-methylpyrolidine (3 x 6 mL), methanol (3 x 6 mL), and again with N-methylpyrolidine (3 x 6 mL) to give compound 11.

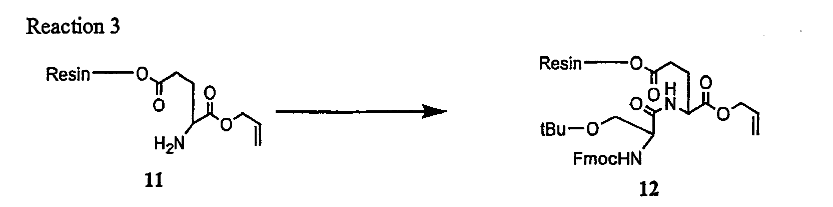

Commercially available Nα-(9-Fluorenylmethoxycarbonyl)-L-serine-*tert*-butyl ether (2 mL of a 0.5 molar solution in N-methylpyrolidine), 1,3-diisopropylcarbodiimide (2 mL of a 0.5 molar solution in N-methylpyrolidine), and 1-hydroxy-benzotriazole (2 mL of a 0.5 molar solution in N-methylpyrolidine) were added to resin 11. The reaction mixture was shaken for one hour, then filtered and the coupling was repeated. The reaction mixture was filtered then the solid was washed with N-methylpyrolidine (3 x 6 mL), methanol (3 x 6 mL), and again with N-methylpyrolidine (3 x 6 mL) to give compound 12.

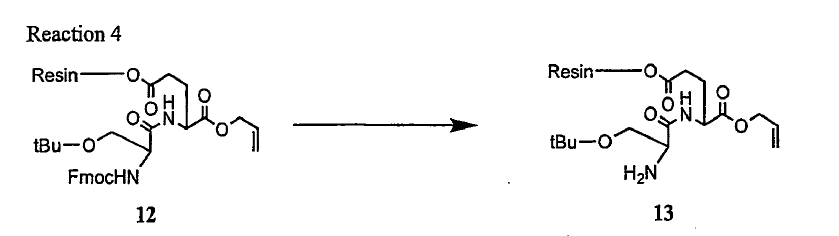

Compound 12 was agitated in 20% piperidine in N-methylpyrolidine (6 mL) for 30 minutes. The resin was filtered and re-suspended in 20% piperidine in N-methylpyrolidine (6 mL) and agitated for 30 minutes. The reaction mixture was filtered then the solid was washed with N-methylpyrolidine (3 x 6 mL), methanol (3 x 6 mL), and again with N-methylpyrolidine (3 x 6 mL) to give compound 13.

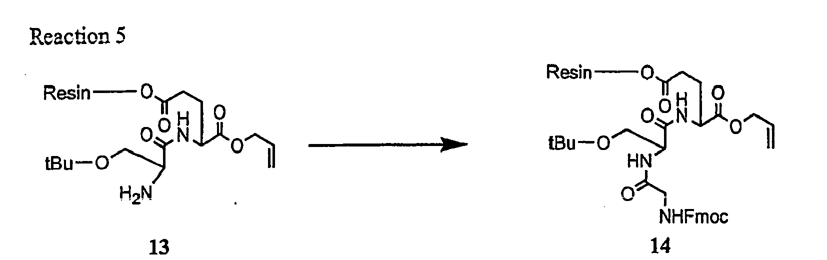

Commercially available Nα-(9-Fluorenylmethoxycarbonyl)-L-glycine ((2 mL of a 0.5 molar solution in N-methylpyrolidine), 1,3-diisopropylcarbodiimide (2 mL of a 0.5 molar solution in N-methylpyrolidine), and 1-hydroxy-benzotriazole (2 mL of a 0.5 molar solution in N-methylpyrolidine) were added to resin 13. The reaction mixture was shaken for one hour, then filtered and the coupling was repeated. The reaction mixture was filtered then the solid was washed with N-methylpyrolidine (3 x 6 mL), methanol (3 x 6 mL), and again with N-methylpyrolidine (3 x 6 mL) to give compound 14.

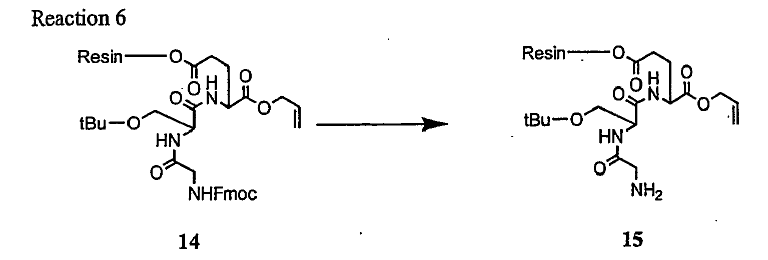

Compound 14 was agitated in 20% piperidine in N-methylpyrolidine (6 mL) for 30 minutes. The resin was filtered and re-suspended in 20% piperidine in N-methylpyrolidine (6 mL) and agitated for 30 minutes. The reaction mixture was filtered then the solid was washed with N-methylpyrolidine (3 x 6 mL), methanol (3 x 6 mL), and again with N-methylpyrolidine (3 x 6 mL) to give compound 15. 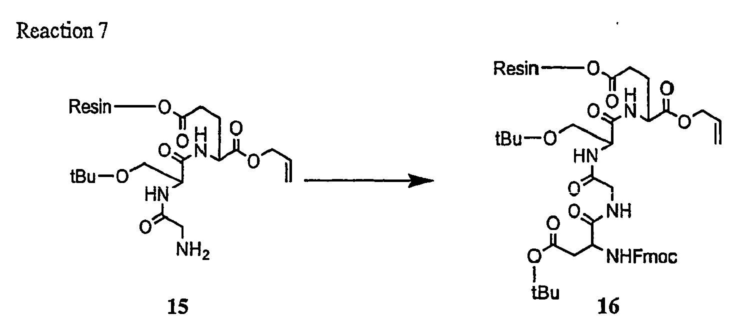 Commercially available Nα-(9-Fluorenylmethoxycarbonyl)-L-aspartic acid β-*tert-*butyl ester ((2 mL of a 0.5 molar solution in N-methylpyrolidine), 1,3-diisopropylcarbodiimide (2 mL of a 0.5 molar solution in N-methylpyrolidine), and 1-hydroxy-benzotriazole (2 mL of a 0.5 molar solution in N-methylpyrolidine) were added to resin 15. The reaction mixture was shaken for one hour, filtered and the coupling was repeated. The reaction mixture was filtered then the solid was washed with N-methylpyrolidine (3 x 6 mL), methanol (3 x 6 mL), and again with N-methylpyrolidine (3 x 6 mL) to give compound 16. 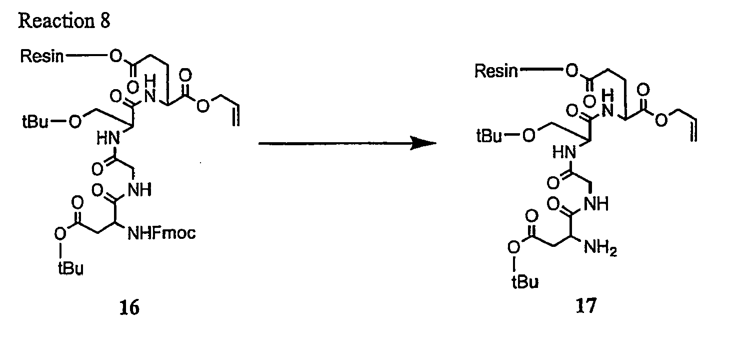 Compound 16 was agitated in 20% piperidine in N-methylpyrolidine (6 mL) for 30 minutes. The resin was filtered and re-suspended in 20% piperidine in N-methylpyrolidine (6 mL) and agitated for 30 minutes. The reaction mixture was filtered then the solid was washed with N-methylpyrolidine (3 x 6 mL), methanol (3 x 6 mL), and again with N-methylpyrolidine (3 x 6 mL) to give compound 17. 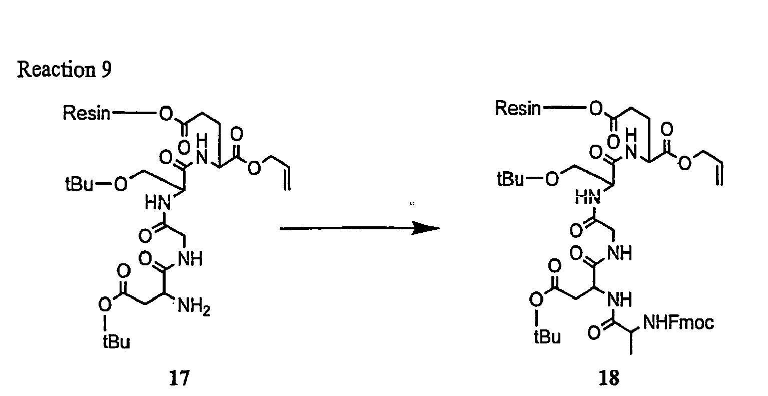 Commercially available Nα-(9-Fluorenylmethoxycarbonyl)-L-alanine ((2 mL of a 0.5 molar solution in N-methylpyrolidine), 13-diisopropylcarbodiimide (2 mL of a 0.5 molar solution in N-methylpyrolidine), and 1-hydroxy-benzotriazole (2 mL of a 0.5 molar solution in N-methylpyrolidine) was added to resin 17. The reaction mixture was shaken for one hour, filtered and the coupling was repeated. The reaction mixture was filtered then the solid was washed with N-methylpyrolidine (3 x 6 mL), methanol (3 x 6 mL), and again with N-methylpyrolidine (3 x 6 mL) to give compound 18. 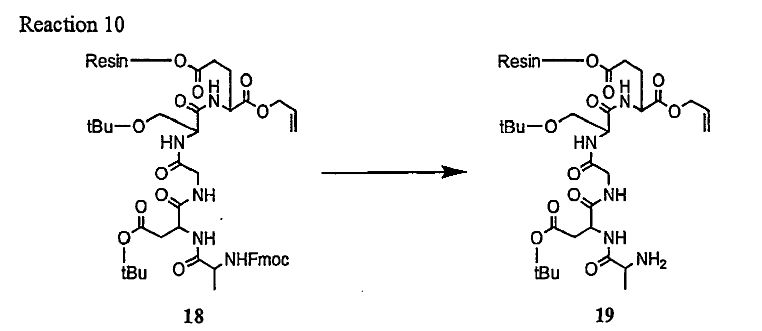 Compound 18 was agitated in 20% piperidine in N-methylpyrolidine (6 mL) for 30 minutes. The resin was filtered and re-suspended in 20% piperidine in N-methylpyrolidine (6 mL) and agitated for 30 minutes. The reaction mixture was filtered then the solid was washed with N-methylpyrolidine (3 x 6 mL), methanol (3 x 6 mL), and again with N-methylpyrolidine (3 x 6 mL) to give compound 19.

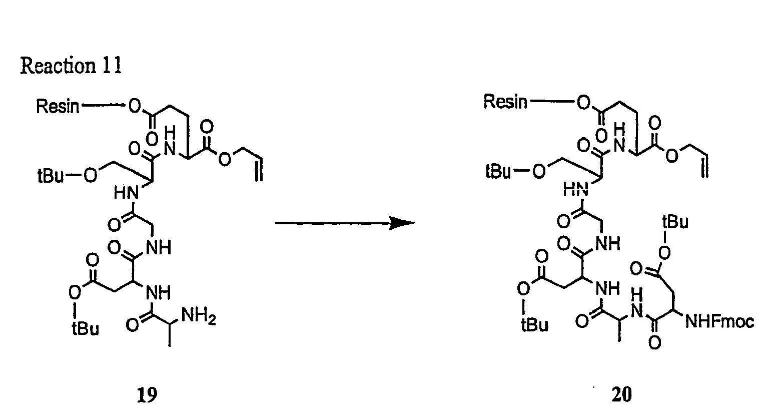

Commercially available Nα-(9-Fluorenylmethoxycarbonyl)-L-aspartic acid β-tertbutyl ester ((2 mL of a 0.5 molar solution in N-methylpyrolidine), 1,3-diisopropylcarbodiimide (2 mL of a 0.5 molar solution in N-methylpyrolidine), and 1-hydroxy-benzotriazole (2 mL of a 0.5 molar solution in N-methylpyrolidine) was added to resin 19. The reaction mixture was shaken for one hour, filtered and the coupling was repeated. The reaction mixture was filtered then the solid was washed with N-methylpyrolidine (3 x 6 mL), methanol (3 x 6 mL), and again with N-methylpyrolidine (3 x 6 mL) to give compound 20.

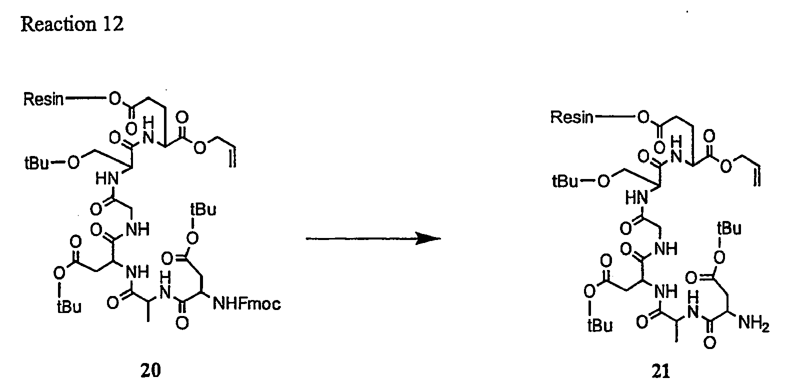

Compound 20 was agitated in 20% piperidine in N-methylpyrolidine (6 mL) for 30 minutes. The resin was filtered and re-suspended in 20% piperidine in N-methylpyrolidine (6 mL) and agitated for 30 minutes. The reaction mixture was filtered then the solid was washed with N-methylpyrolidine (3 x 6 mL), methanol (3 x 6 mL), and again with N-methylpyrolidine (3 x 6 mL) to give compound 21.

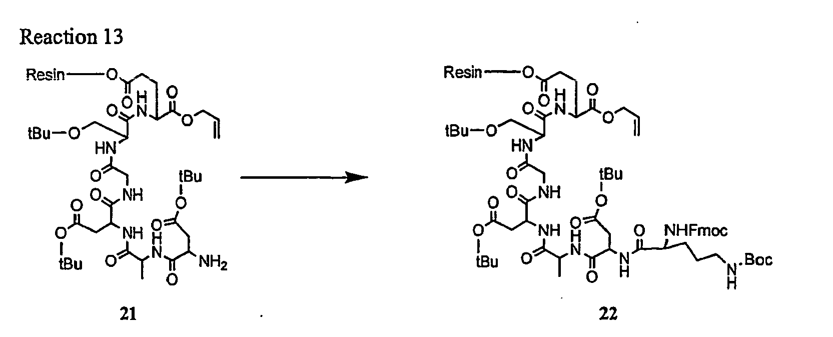

Commercially available Nα-(9-Fluorenylmethoxycarbonyl)-Nδ-(tertbutoxycarbonyl) -L-ornithine (2 mL of a 0.5 molar solution in N-methylpyrolidine), 1,3-diisopropylcarbodiimide (2 mL of a 0.5 molar solution in N-methylpyrolidine), and 1-hydroxy-benzotriazole (2 mL of a 0.5 molar solution in N-methylpyrolidine) was added to resin 21. The reaction mixture was shaken for one hour, then filtered and the coupling was repeated. The reaction mixture was filtered then the solid was washed with N-methylpyrolidine (3 x 6 mL), methanol (3 x 6 mL), and again with N-methylpyrolidine (3 x 6 mL) to give compound 22.

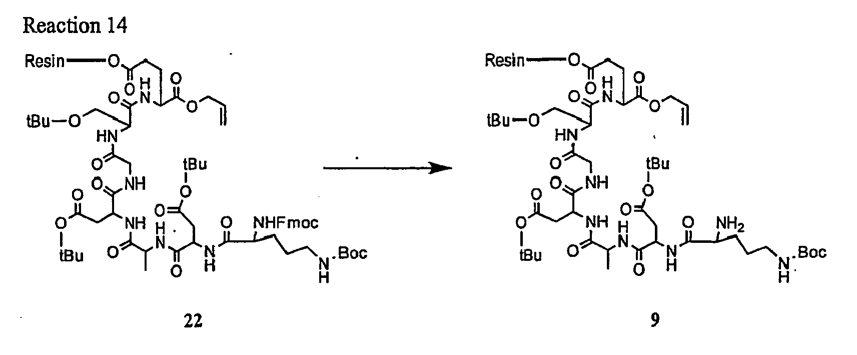

Compound 22 was agitated in 20% piperidine in N-methylpyrolidine (6 mL) for 30 minutes. The resin was filtered and re-suspended in 20% piperidine in N-methylpyrolidine (6 mL) and agitated for 30 minutes. The reaction mixture was filtered then the solid was washed with N-methylpyrolidine (3 x 6 mL), methanol (3 x 6 mL), and again with N-methylpyrolidine (3 x 6 mL) to give compound 9.

### Example 3: Synthesis of Peptide Resin Compound 23:

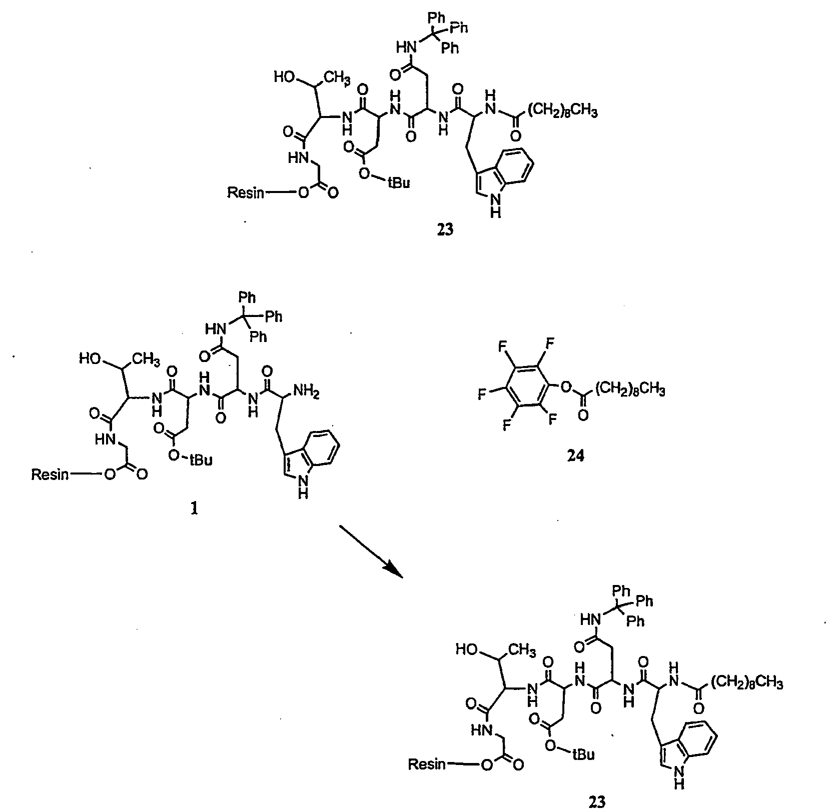

Resin peptide 1 (2 g) was added to a solution of the pentafluorophenyl ester of decanoic acid 24 (440 mg, for the preparation of compound 24 see Example 6, Reaction 1) in dichloromethane. The mixture was shaken for 17 hours, filtered, and the reaction was judged to be incomplete using the Kaiser Test (*vide supra*)*.* Decanoic acid (517 mg), 1-hydroxy-benzotriazole (446 mg), and 1,3-diisopropylcarbodiimide (438 µL) were dissolved in N-methylpyrolidine (8 mL) and stirred for one hour. The resin was then added to the mixture then stirred for 8 hours, filtered and washed with N-methylpyrolidine (3 x 6 mL), methanol (3 x 6 mL), and again with N-methylpyrolidine (3 x 6 mL). The reaction was found to be complete using the Kaiser Test, yielding the resin bound lipopetide 23.

### Example 4: Synthesis of Compound 29:

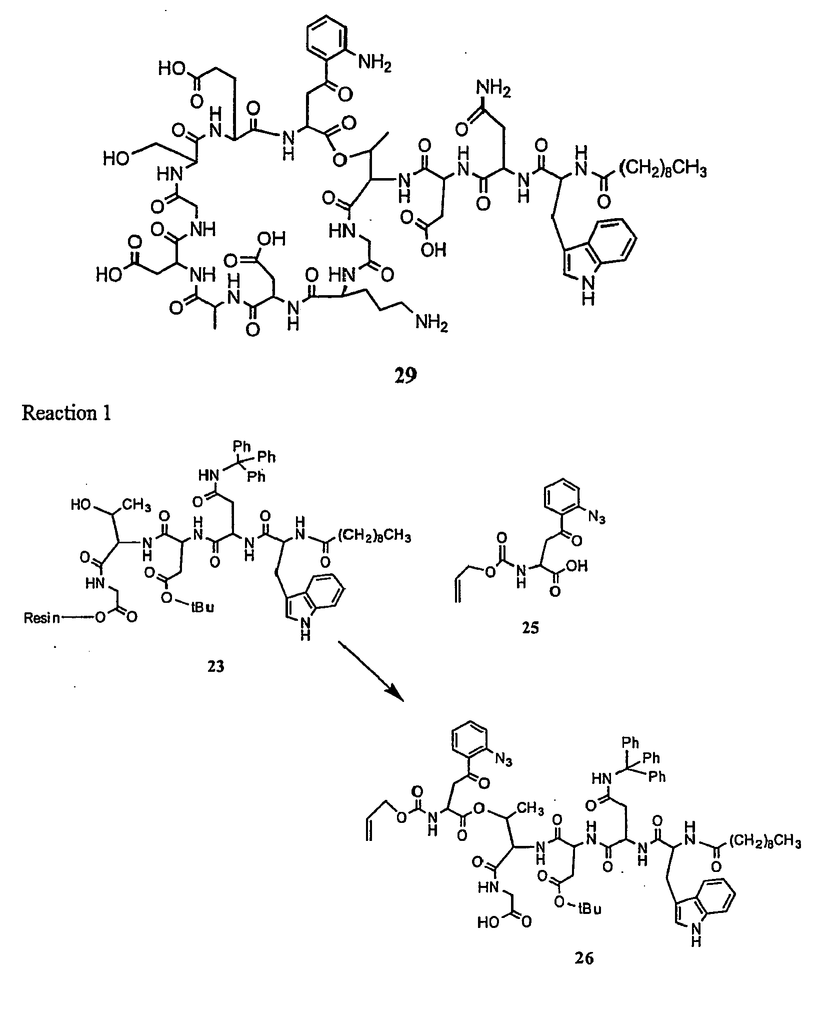

L-2-N-(allyloxycarbonyl)-4-(2-azidophenyl)-4-oxobutanoic acid 25 (636 mg, see example 15 (*vide infra*)), 4-dimethylaminopyridine (25 mg), and N-methyl-2-chloropyridinium iodide (511 mg) were flushed well with argon, then suspended in dichloromethane (10 mL). Triethylamine (560 µL) was added and the reaction mixture was stirred to give a homogeneous solution. Resin lipopeptide 23 (667 mg) was added to the solution and the flask was flushed again with argon and shaken for 17 hours. A 20 mg sample of the resin was removed to test the reaction for completion (20mgs of resin in dichloromethane (0.6ml) was treated with 2,2,2-trifluoroethanol, (0.2ml) and acetic acid (0.2ml) and stirred for 3 hours. The reaction mixture was filtered, and the solvent was evaporated to give a residue. Liquid Chromatography/Mass Spectral analysis of the residue indicated the reaction was incomplete). Coupling was judged to be incomplete so the resin was dried under reduced pressure for 5 days, and the above coupling was repeated over another 17 hours. The reaction mixture was filtered and the solid was washed well with dichloromethane. The solid was then suspended in dichloromethane (6 mL), 2,2,2-trifluoroethanol (2 mL), acetic acid (2 mL), and shaken for 5 hours. The reaction mixture was filtered and evaporation of the filtrate gave the crude desired peptide 26 (44 mg). The crude product was purified by reverse phase HPLC (C18 10 uM Jupiter column 250 x 21.2mm) eluting with a gradient from 20% acetonitrile 0.5% formic acid: 80 % water 0.5% formic acid to 80% acetonitrile 0.5% formic acid: 20 % water 0.5% formic acid over 25 minutes. The product bearing fractions were freeze-dried to give the pure product 26 (10.6 mg).

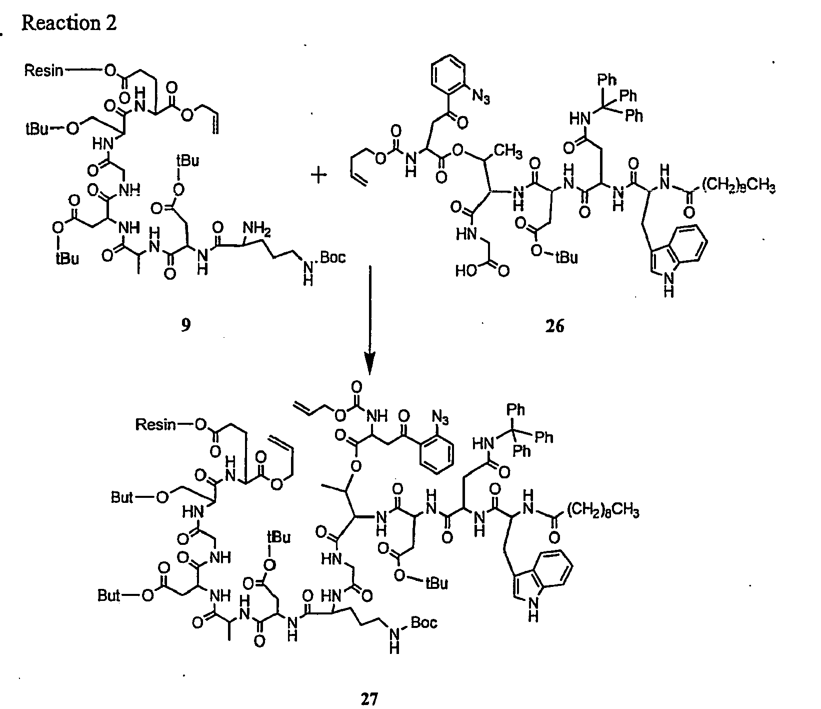

Hydroxy-benzotriazole (5 mgs), 1,3-diisopropylcarbodiimide (6 µL), and peptide resin 9 (12.3 mg) were added to a solution of compound 26 (10.6mg) in N-methylpyrolidine (0.7 mL) then shaken for 22 hours. The resin was filtered and the coupling was judged to be complete using the Kaiser Test, yielding resin bound lipopeptide 27.

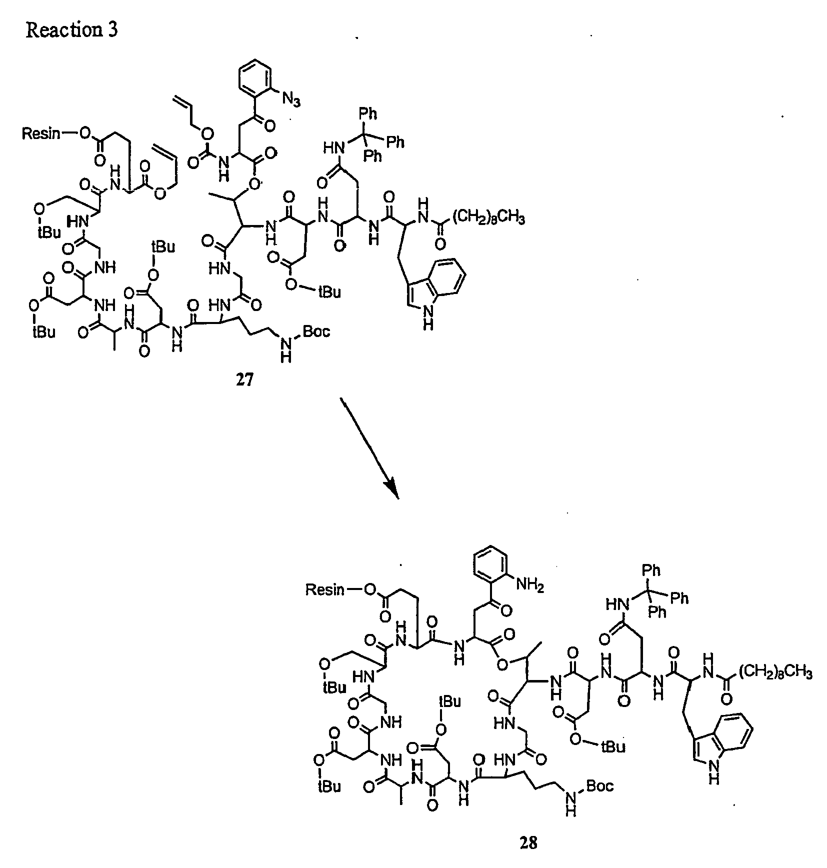

The dried resin 27 was placed under an argon atmosphere, and treated with a solution of tetrakis-(triphenylphosphine)palladium(0) (19 mg) in dichloromethane (1.47 mL), acetic acid (74 µL), and N-methylmorpholine (37 µL). The mixture was shaken for 4 hours at ambient temperature, filtered, and the solid was washed with two times with N-methylmorpholine, two times with methanol, and again two times with N-methylmorpholine . 1-Hydroxy-benzotriazole (0.5 mL of a 0.5 molar solution in N-methylmorpholine) and 1,3-diisopropylcarbodiimide (0.5 mL of a 0.5 molar solution in N-methylmorpholine) were added to the resin. The reaction was shaken for 17 hours, filtered, and washed well with N-methylmorpholine to give the resin bound cyclized depsipeptide 28.

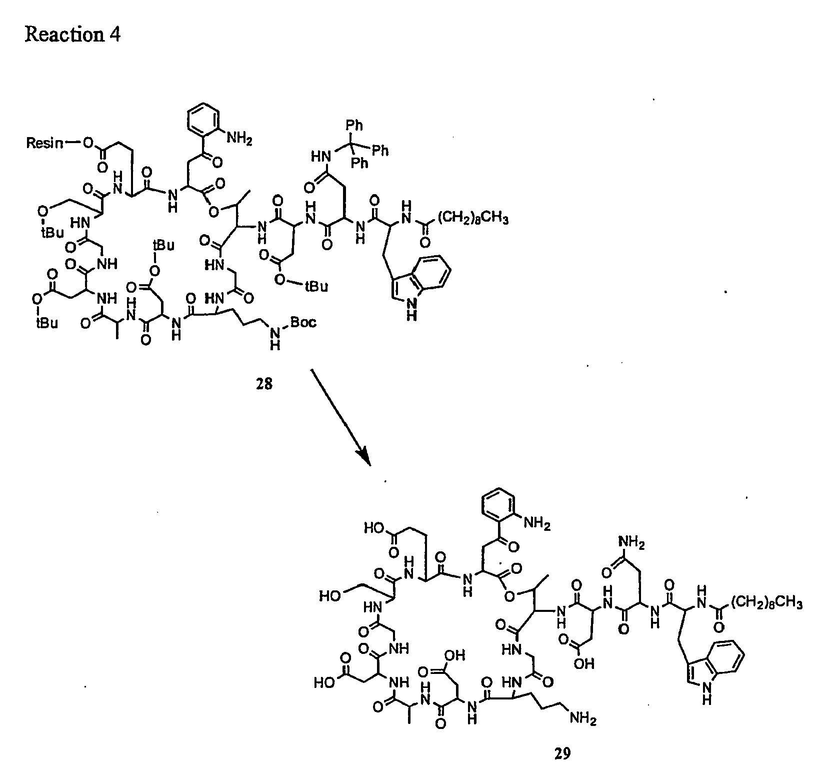

The dried resin 28 was suspended in dichloromethane, (4 mL) trifluoroacetic acid, (6 mL) ethanedithiol (250 µl), and triisopropylsilane(250 µl), and the reaction mixture was stirred for 3 hours at ambient temperature. The resin was filtered and the combined filtrates were evaporated under reduced pressure. Crude product was then partitioned between diethyl ether (6 mL), and water (3 mL). The aqueous layer was freeze-dried to give crude product. The crude product was purified by reverse phase HPLC (C18 10 uM Jupiter column 250 x 21.2mm) eluting with a gradient from 20% acetonitrile 0.5% formic acid: 80 % water 0.5% formic acid to 80% acetonitrile 0.5% formic acid: 20 % water 0.5% formic acid over 25 minutes. The product bearing fractions were combined and freeze-dried to give the pure product 29 (1.0 mg).

### Example 5: Synthesis of Compound 33:

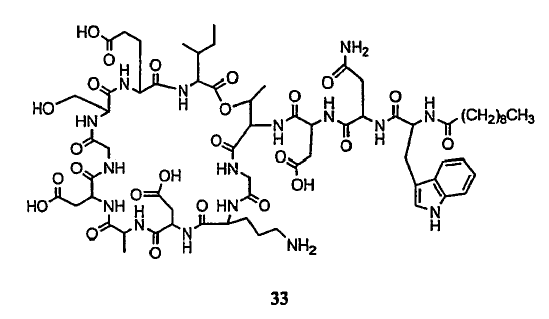

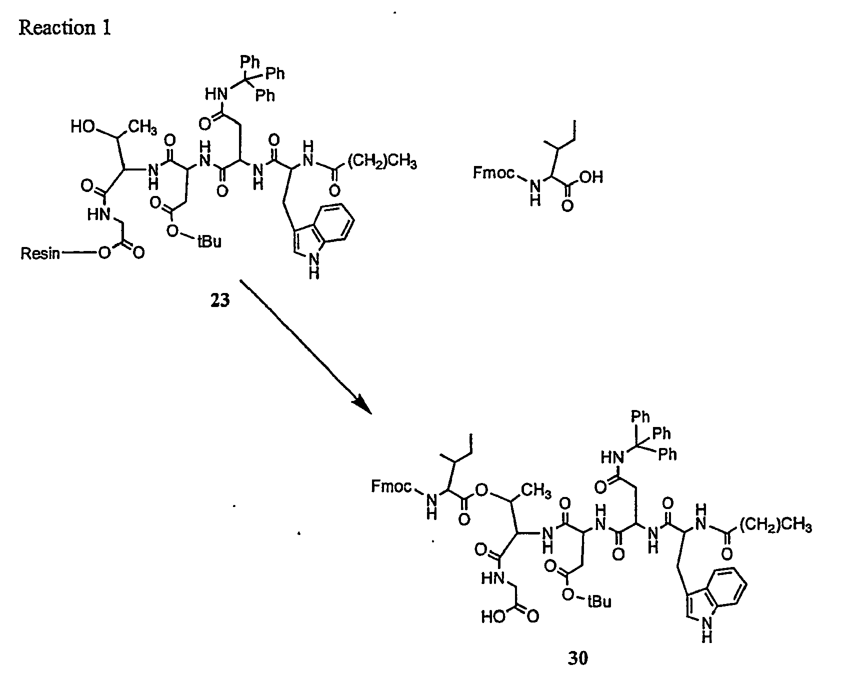

Commercially available Nα-(9-Fluorenylmethoxycarbonyl)-L-isoleucine (95 mg), 4-dimethylaminopyridine (6 mg), and N-methyl-2-chloropyridinium iodide (69 mg) were flushed well with argon then suspended in dichloromethane (2.7 mL). Triethylamine (76 µL) was added and the reaction mixture was stirred to give a homogeneous solution. Resin lipopeptide 23 (200 mg) was added to the solution, the flask was flushed again with argon and then shaken for 14 hours. The resulting resin was then filtered and washed well with dichloromethane. The solid was suspended in dichloromethane (6 mL), 2,2,2-trifluoroethanol (2 mL), and acetic acid (2 mL), and shaken for 3 hours. The resin was filtered and evaporation of the filtrate gave the desired peptide 30 (54 mg) as a white solid.

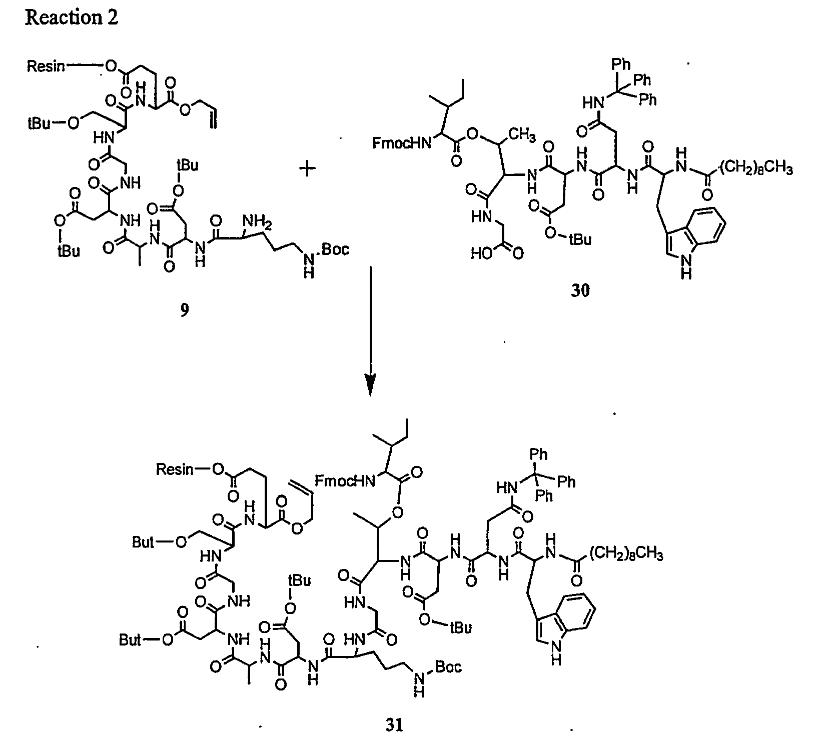

1-Hydroxy-benzotriazole (26 mg), 1,3-diisopropylcarbodiimide (30 µl), and peptide resin 9 (64 mg) were added to a solution of the depsipeptide 30 (54 mg) in N-methylmorpholine (3.8 mL), and the resulting mixture was shaken for 22 hours. The resin was filtered, and the coupling was judged to be complete using the Kaiser Test, yielding the resin bound depsipeptide 31.

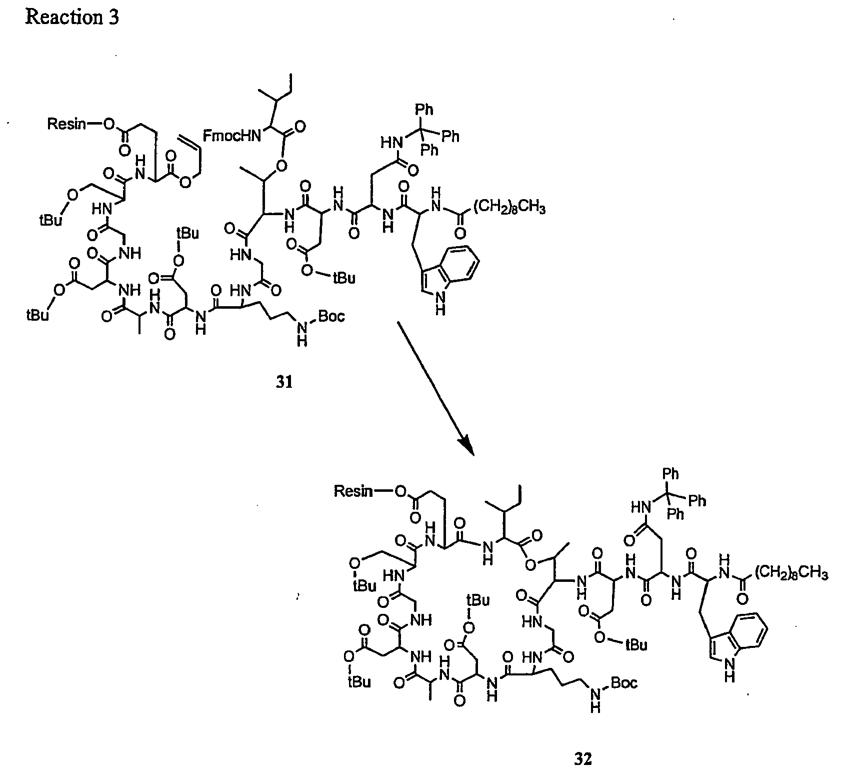

The dried resin 31 was placed under an argon atmosphere, and treated with a solution of tetrakis-(triphenylphosphine)palladium(0) (48 mg in dichloromethane (7.63mL)), acetic acid (0.38 mL), and N-methylmorpholine (0.19 mL). The mixture was shaken for 4 hours at ambient temperature, filtered, and the solid was washed two times with N-methylmorpholine, two times with methanol, and again two times with N-methylmorpholine. The solid resin was suspended in 20% piperidine in N-methylmorpholine (7 mL) for 105 minutes, filtered and the solid was washed well with N-methylmorpholine. 1-Hydroxy-benzotriazole (0.3 mL of a 0.5 molar solution in N-methylmorpholine) and 1,3-diisopropylcarbodiimide (0.3 mL of a 0.5 molar solution in N-methylmorpholine) were added to the resin. The reaction was shaken for 17 hours, filtered, and the precipiate was washed well with N-methylmorpholine to give the resin bound cyclized depsipeptide 32.

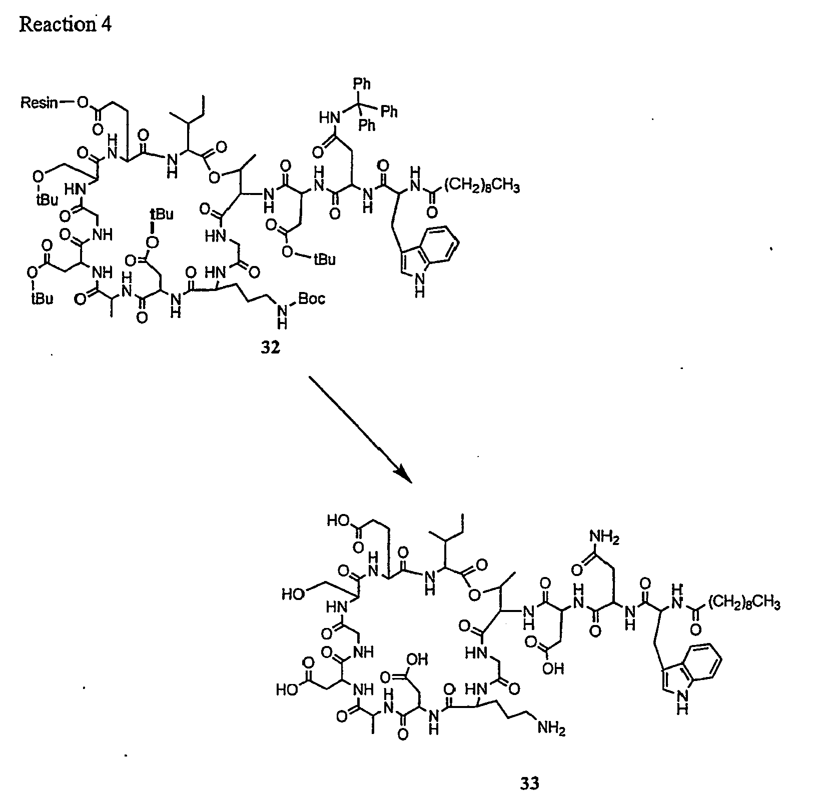

The dried resin 32 was suspended in dichloromethane (4 mL), trifluoroacetic acid (6 mL), ethanedithiol (250 µl), and triisopropylsilane (250 µl), and stirred for 3 hours at ambient temperature. The reaction mixture was filtered an washed with dichloromethane (2 x 2 mL) and the combined filtrates were evaporated under reduced pressure. Crude product was then partitioned between diethyl ether (6 mL) and water (3 mL). The aqueous layer was separated and freeze dried to give the crude product 33 (21.5 mgs). The crude product was then purified by reverse phase HPLC (C18 10 uM Jupiter column 250 x 21.2mm) eluting with a gradient from 20% acetonitrile 0.5% formic acid: 80 % water 0.5% formic acid to 80% acetonitrile 0.5% formic acid : 20 % water 0.5% formic acid over 25 minutes. The product bearing fractions were combined and freeze-dried to give the pure product 33 (1.8 mg).

### Example 6: Synthesis of Compound 34:

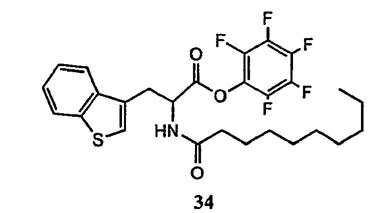

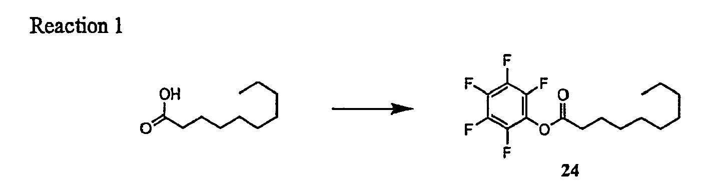

To a solution of commercially available decanoic acid (13.78 g) in tetrahydrofuran (40 mL), was added 1,3-diisopropylcarbodiimide (13.76 mL). After 5 minutes a solution of pentafluorophenol (16.20 g) in tetrahydrofuran (40 mL) was added and the reaction mixture was stirred for 72 hours. The resulting mixture was filtered; the residue was washed with tetrahydrofuran (40 mL) and the tetrahydrofuran filtrates were combined. The solvent was evaporated and the residue was purified on silica gel using 9:1 hexane: ethyl acetate as an eluent to give the desired product 24 as an oil (23.49 g)

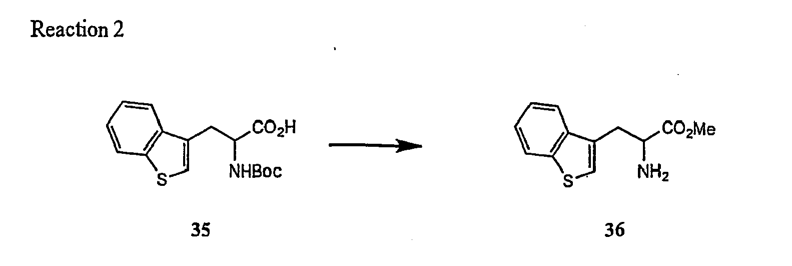

A solution of commercially available Boc-L-3-Benzothienylalanine 35 (1.0 g) in dry methanol (20 mL) was cooled to 0 °C in an ice bath. Thionyl chloride (1.63 mL) was added dropwise, and the reaction mixture was allowed to warm to ambient temperature over 16 hours. Evaporation of the solvent gave a crude product that was partitioned between ethyl acetate and aqueous potassium bicarbonate. The organic layer was washed with aqueous potassium bicarbonate, and saturated sodium chloride, dried with anhydrous sodium sulphate and evaporated to give the product 36 (0.66g).

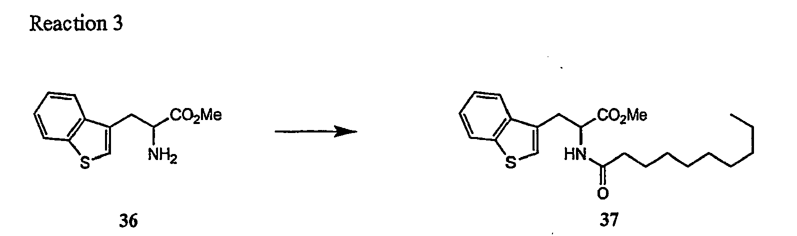

To a solution of the Benzothienylalanine-methyl ester 36 (0.66 g) in tetrahydrofuran (10 mL) was added a solution of the decanoyl pentafluorophenolester 24 (1.04 g) in tetrahydrofuran (10 mL). The resulting solution was stirred for 24 hours. The reaction mixture was poured into ethyl acetate and washed successively with 1N hydrochloric acid, 10% aqueous potassium carbonate, and saturated sodium chloride. The combined organic layer was then dried with anhydrous sodium sulphate and evaporated to give product 37 (1.40 g) which was used as crude material in the next reaction

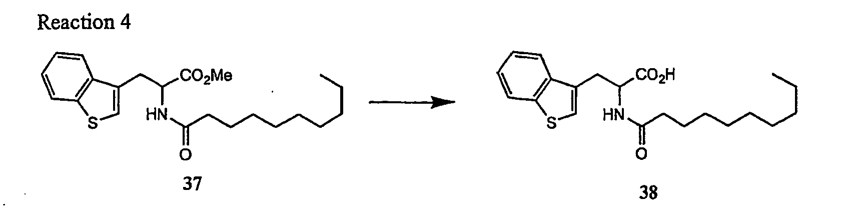

Lithium hydroxide mono hydrate (0.59 g) was added to a solution of compound 37 (1.40 g) methanol (15 mL) and water (3 mL) and the reaction mixture was stirred for one hour. The methanol was removed by evaporation and the aqueous solution was acidified to pH 1 with 1 N hydrochloric acid and extracted with ethyl acetate. The organic layers were combined and dried with anhydrous sodium sulphate and evaporated to give the product 38.

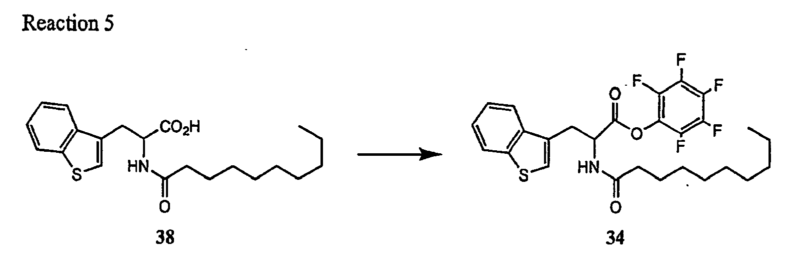

1,3-Diisopropylcarbodiimide (121 mg) was added to a solution of the compound 38 (200 mg), and pentafluorophenol (108 mg) in tetrahydrofuran (5mL) and the reaction mixture was stirred for one hour. The resulting mixture was filtered, the solvent was evaporated and the residue was purified on silica gel. Using 9:1 hexane: ethyl acetate as an eluent gave the faster running impurity and 4:1 hexane : ethyl acetate as an eluent gave the product 34 (80 mg).

### Example 7: Synthesis of Compound 39:

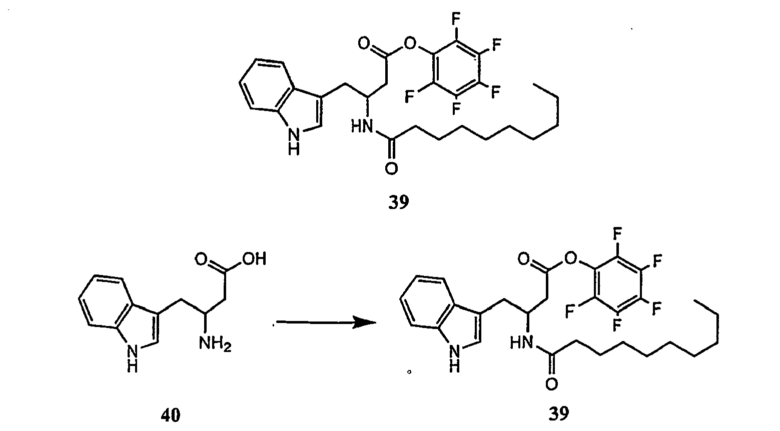

Compound 39 was prepared from commercially available 40 via the conditions described above as follows: Compound 39 was converted to the corresponding methyl ester under the conditions described in Example 6, Reaction 2. The methyl ester was acylated under the conditions described in Example 6, Reaction 3. The acylated compound was then saponified under the reaction conditions described in Example 6, Reaction 4. The saponified material was then converted to compound 39 via the conditions described in Example 6, Reaction 5.

### Example 8: Synthesis of Compound 41:

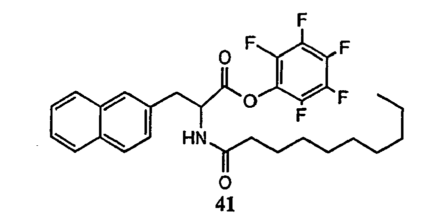

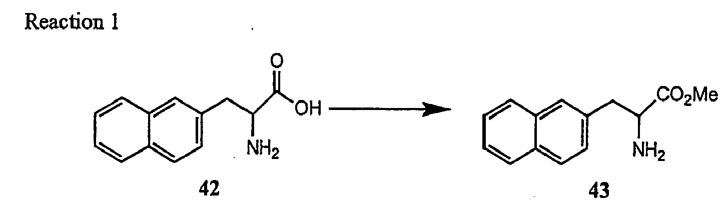

Commercially available compound 42 was converted to compound 43 under the conditions described in Example 6, Reaction 2

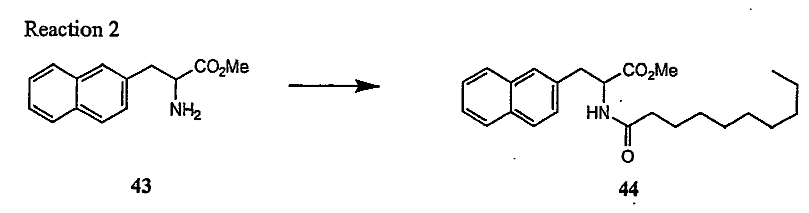

Decanoyl chloride (0.51 mL) was added to a solution of amino acid methyl ester 43(440 mg) and triethylamine (0.43 mL) cooled to 0 °C in an ice bath. The reaction was allowed to warm to ambient temperature over three hours. The reaction was then poured into ethyl acetate and washed successively with water, and saturated sodium chloride. The organic layers were then dried with anhydrous sodium sulphate and evaporated to give the product 44.

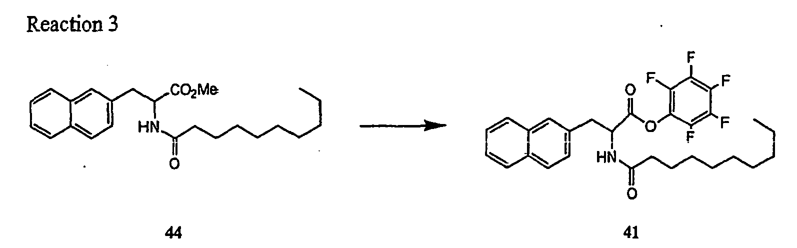

Compound 44 was converted to compound 41 via the conditions described in the as follows. Compound 44 was saponified under the reaction conditions described in Example 6, Reaction 4. The saponified material was then converted to compound 41 via the conditions described in Example 6, Reaction 5.

### Example 9: Synthesis of Compound 45:

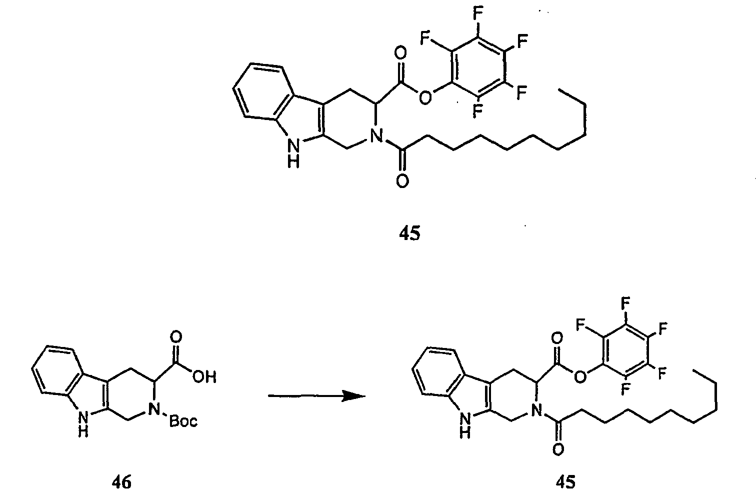

Compound 45 was prepared from commercially available 46 via the conditions described above as follows: Compound 46 was converted to the corresponding methyl ester under the conditions described in Example 6, Reaction 2. The methyl ester was acylated under the conditions described in Example 8, Reaction 2. The acylated compound was then saponified under the reaction conditions described in

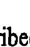

Example 6, Reaction 4. The saponified material was then converted to compound 45 via the conditions described in Example 6, Reaction 5.

### Example 10 : Synthesis of Compound 47:

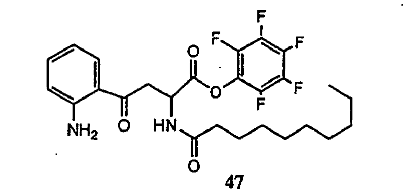

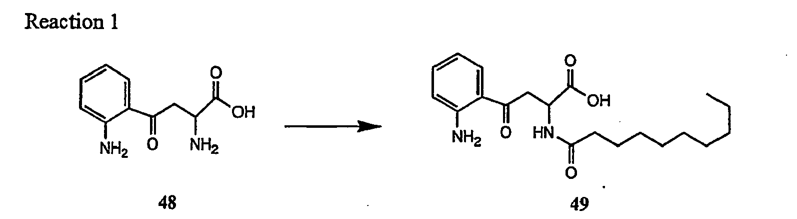

Commercially available 48 was treated under the conditions described in Example 6, Reaction 3 above to afford compound 49. Treatment of compound 49 as described in Example 6, Reaction 5 gave compound 47.

### Example 11: Synthesis of Compound 50:

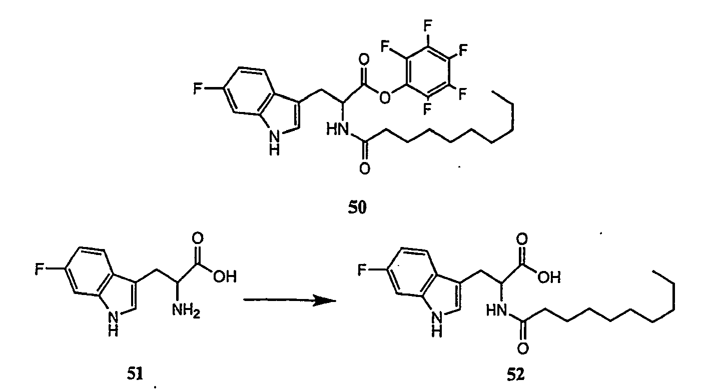

Commercially available 51 was treated under the conditions described in Example 6, Reaction 3 above, with the single modification being the use of N,N'-dimethylformamide solvent in place of tetrahydrofuran solvent, to afford compound 52. Treatment of compound 52 as described in Example 6, Reaction 5 above, with the single modification being the use of N,N'-dimethylformamide solvent in place of tetrahydrofuran solvent, gave compound 50.

### Example 12: Synthesis of Compound 53:

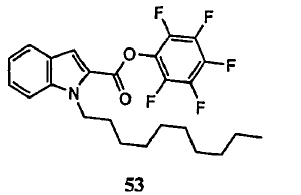

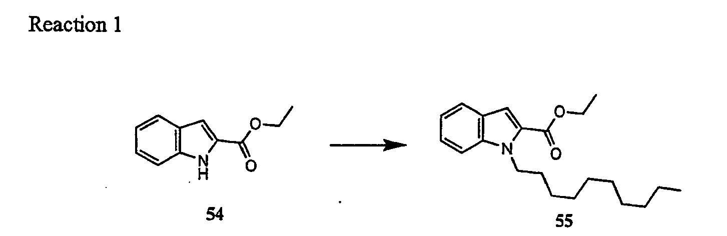

A solution of commercially available ethyl indole-2-carboxylate 54 (2 g), iododecane (2.25 mL), and potassium carbonate (2.92 g) in dry dimethylformamide (30 mL) was stirred for 24 hours. The reaction was poured into ethyl acetate (200 mL) and washed with water (300 mL). The organic layer was then dried with anhydrous magnesium sulphate and evaporated to give the product 55 (2.604 g).

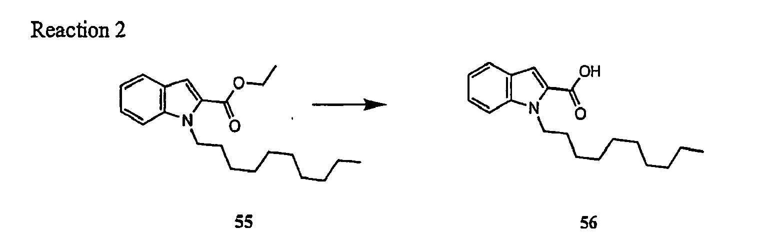

Lithium hydroxide/water (1.65 g) in water (16 mL) was added to a solution of compound 55 (2.6 g) in tetrahydrofuran (16 mL) and stirred for 4 days. Ethyl acetate (20 mL) and water (10 mL) were added and the aqueous layer was adjusted to pH 1 and extracted with ethyl acetate (3 x 5 mL). The organic layers were combined, dried and evaporated to give an oil. The hydrolysis was incomplete so the oil was dissolved in methanol/water 2:1 and potassium hydroxide (0.88 g) was added. The reaction mixture was stirred for 15 hours. Ethyl acetate (20 mL) and water (10 mL) were added the layers were separated, and the aqueous layer was adjusted to pH 1 and extracted with ethyl acetate (3 x 5 mL). The organic layers were combined, dried and evaporated to give the crude product as an oil which was purified on silica gel using 10:1 1 hexane: ethyl acetate as an eluent to give the product 56 (0.35g).

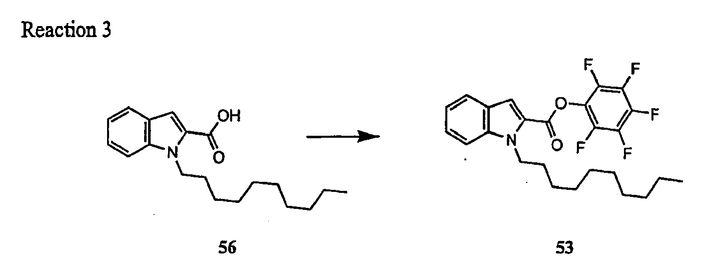

1,3-Diisopropylcarbodiimide (206 mg) was added to a solution of the compound 56 (340 mg), and pentafluorophenol (227 mg) in dichloromethane (5 mL) and the reaction mixture was stirred for 15 hours. The mixture was quenched with hexane, filtered, and the solvent was evaporated. Purification of the residue on silica gel with 20:1 hexane: ethyl acetate gave the product 53 (490 mg).

### Example 13: Synthesis of Compound 57:

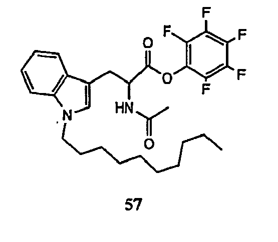

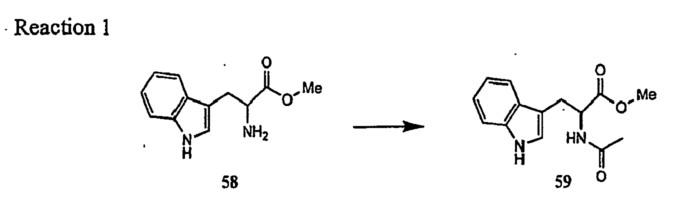

To a solution of commercially available tryptophan methyl ester 58 (2.54 g) and triethylamine (2.9 mL) in dichloromethane (10 mL) was added 4-dimethylaminopyridine (0.12 g) and acetic anhydride (1.03 mL). After 18 hours the reaction mixture was quenched with 1N hydrochloric acid (10 mL), and the aqueous layer was extracted with ethyl acetate (3 x 10 mL). The combined organic layers were washed with saturated sodium bicarbonate and saturated sodium chloride then dried with anhydrous sodium sulphate and evaporated to a crude product Purification on silica gel with 2:1 hexane: ethyl acetate gave the product 59 (1.99 g).

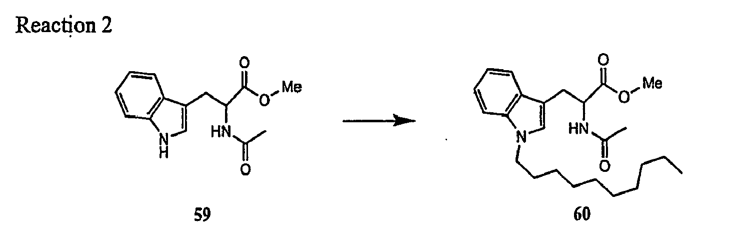

Compound 59 was alkylated under the conditions described in Example 8, Reaction 2 above, substituting potassium hydroxide for potassium carbonate, to afford compound 60.

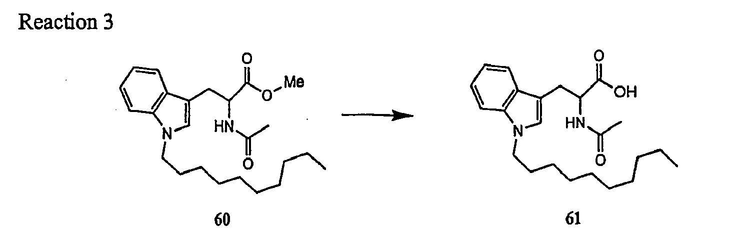

Hydrolysis of compound 60 utilizing the procedure described in Example 6, Reaction 4 gave compound 61.

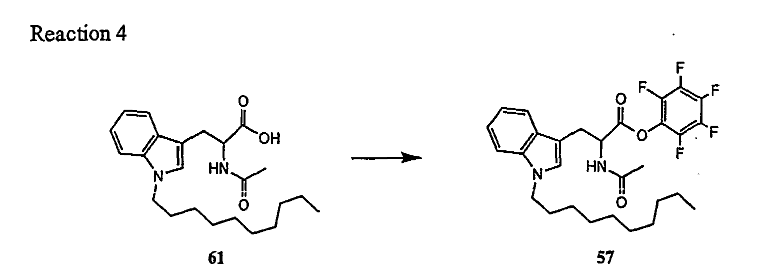

Compound 61 was converted to compound 57 utilizing the conditions described in Example 6, Reaction 4.

### Example 14: Synthesis of Compound 62:

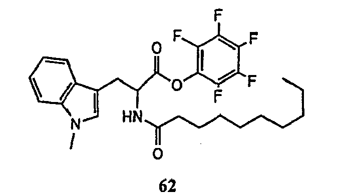

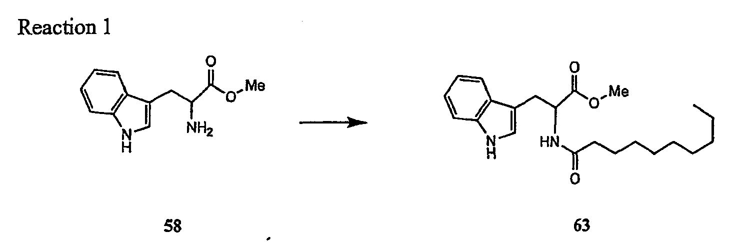

Commercially available tryptophan methyl ester 58 is converted to Compound 63 via the conditions described in Example 8, Reaction 2.

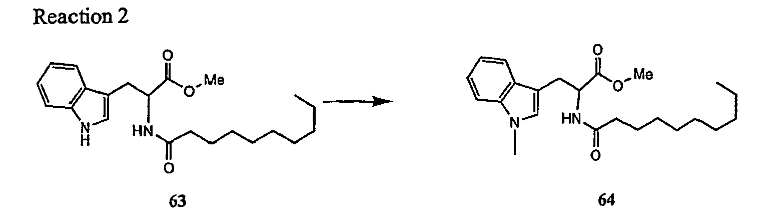

Compound 63 is then converted to Compound 64 utilizing the conditions described in Example 12, Reaction 1 using methyl iodide in place of iododecane.

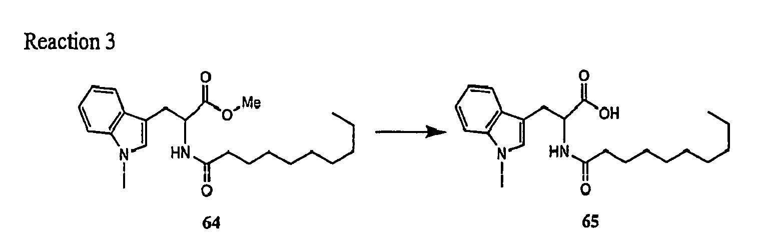

Compound 64 was hydrolyzed using the conditions described in Example 13, Reaction 3 to afford Compound 65.

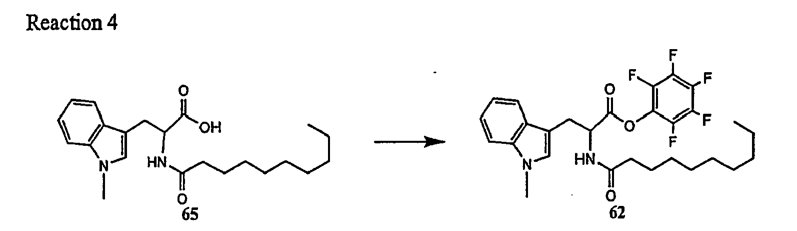

Compound 65 was converted to Compound 62 utilizing the conditions described in Example 13, Reaction 4.

### Example 15: Synthesis of Compound 25:

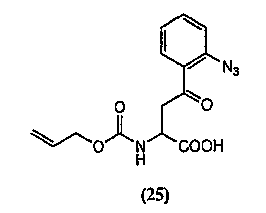

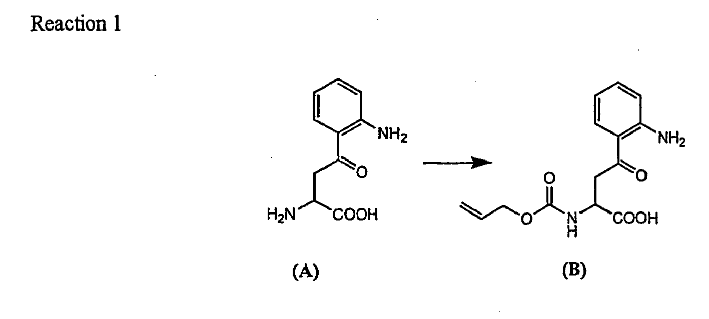

To a suspension of the commercially available L-2-Amino-4-(2-aminophenyl)-4-oxobutanoic acid A (3.93 g) and triethylamine (5.79 mL) in tetrahydrofuran (181 mL), was added sufficient water to obtain a homogenous solution. The solution was then cooled to 0 °C in an ice bath, and diallyl pyrocarbonate (3.36 mL) was added dropwise. The reaction mixture was allowed to warm to ambient temperature over 16 hours, then was concentrated to one third the original volume. The mixture was poured into dichloromethane (350 mL), washed with 1N hydrochloric acid (3 x 100 mL) and saturated sodium chloride (1 x 100 mL). The combined aqueous layers were back extracted with ethyl acetate (4 x 100 mL), and the combined organic fraction was dried with anhydrous sodium sulphate. Evaporation of the solvent gave product B as a yellow solid (5.14 g)

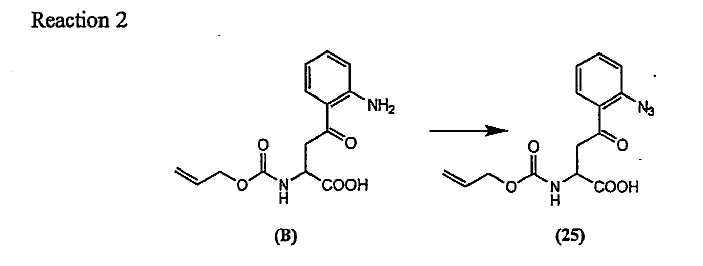

To a -2 °C solution of compound B (5.00 g) in water (130 mL) and concentrated hydrochloric acid (43 mL), was added a solution of sodium nitrite (1.30 g) in water (3 mL) via dropwise addition, so that the temperature remained below 0 °C. The reaction mixture was stirred at -4°C for 135 minutes and a solution of sodium azide (3.34 g) in water (3 mL) was added. The reaction mixture was allowed to warm to ambient temperature over 16 hours. The mixture was then poured into water (130 mL), and the aqueous phase was extracted with dichloromethane (4 x 80 mL). The combined organic layer was then dried with anhydrous sodium sulphate. Evaporation of the solvent gave the product 25 as an orange foam (5.14 g)

### Example 16 : Synthesis of Compound 77:

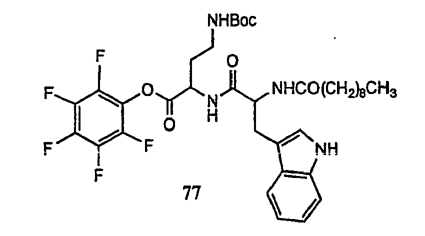

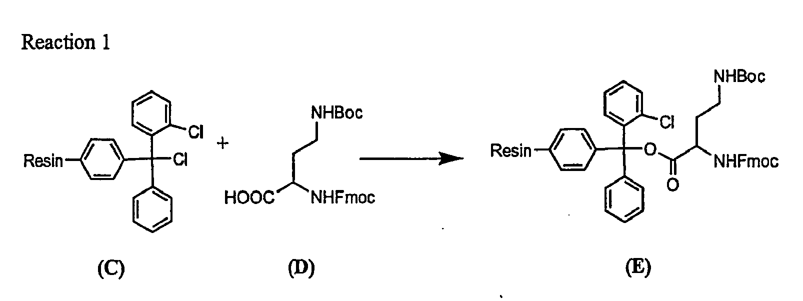

To a suspension of commercially available 2-chlorotritylresin C (1 g), and commercially available 2-N-(9-Fluorenylmethoxycarbonyl)-4-N(*tert-*butoxycarbonyl)-L-2,4-diaminobutyric acid in dichloromethane D (10 mL), was added diisopropylethylamine (1.65 mL). The mixture was shaken for 3 hours, filtered, and the solid was washed with dichloromethane (10 mL), then dried to give E.

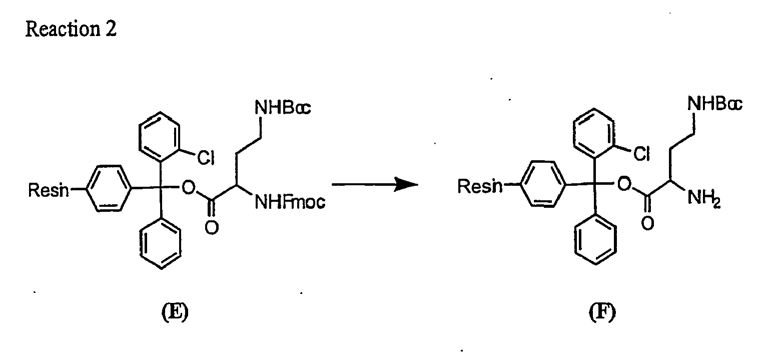

Compound E was agitated in 20% piperidine in N-methylpyrolidine (20 mL) for 60 minutes. The reaction mixture was filtered then the solid washed with N-methylpyrolidine (20 mL) to give compound F.

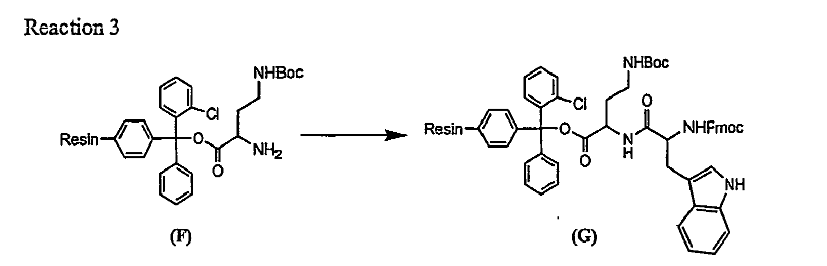

Diisopropylethylamine (0.5 mL) was added to a solution of commercially available Nα-(9-Fluorenylmethoxycarbonyl)-L-tryptophan (1.23 g), TBTU (0.92 g) and N,N-dimethylformamide (10 mL) and the resulting mixture was shaken for 10 minutes. The solution was then added to resin F, shaken for one hour, filtered, and the solid was washed with N-methylpyrolidine (10 mL) to give compound G.

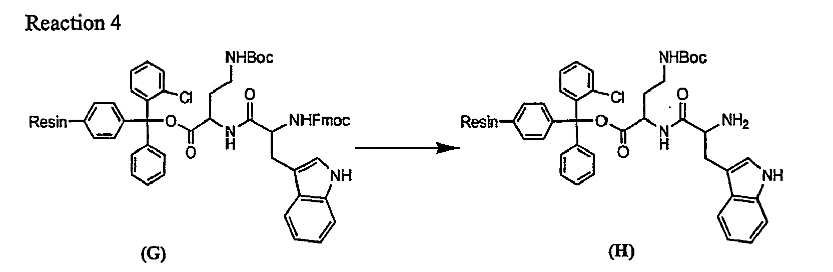

Compound G was agitated in 20% piperidine in N-methylpyrolidine (20 mL) for 60 minutes. The reaction mixture was filtered then the solid was washed with N-methylpyrolidine (20 mL) to give compound H.

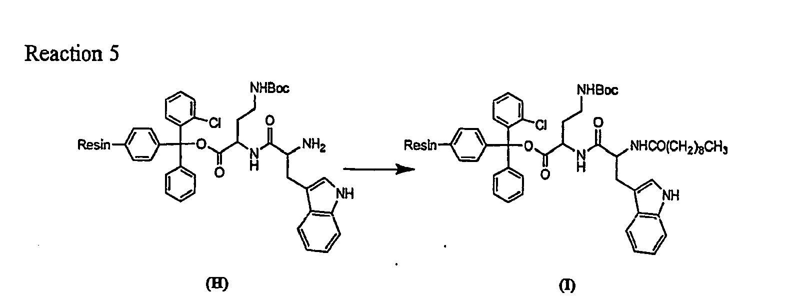

Diisopropylethylamine (0.5 mL) was added to a solution of commercially available decanoic acid (0.50 g), TBTU (0.92 g) and N,N-dimethylformamide (15 mL) and the resulting mixture was shaken for 10 minutes. The solution was then added to the resin H, shaken for one hour, filtered, and the solid was washed with N-methylpyrolidine (10 mL) to give compound I.

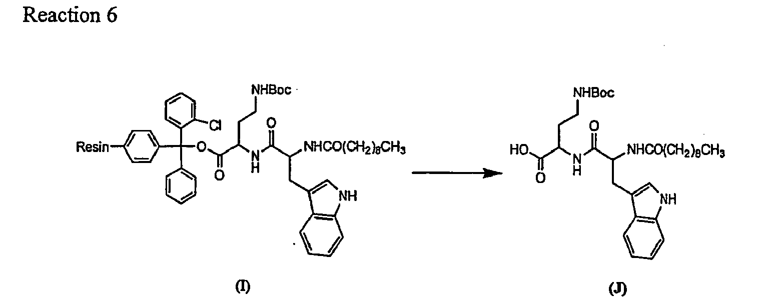

Compound I was treated with dichloromethane:2,2,2,-trifluoroethanol:acetic acid (3:1:1) and the resulting mixture was shaken for 3 hours. The reaction mixture was then filtered and the filtrate was evaporated to give compound J as a white solid (180 mg).

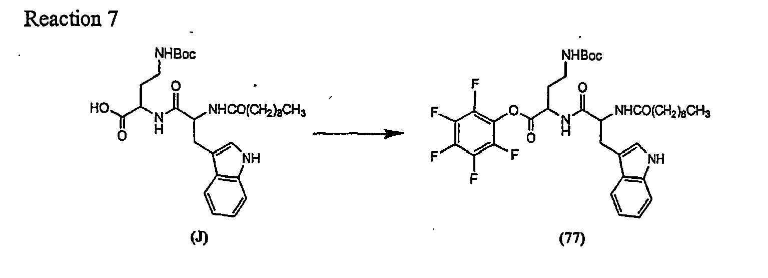

Compound J was converted to compound 77 utilizing the conditions described in Example 6, Reaction 4.

### Example 17: Synthesis of Alloc Protected Daptomycin: Compound 67:

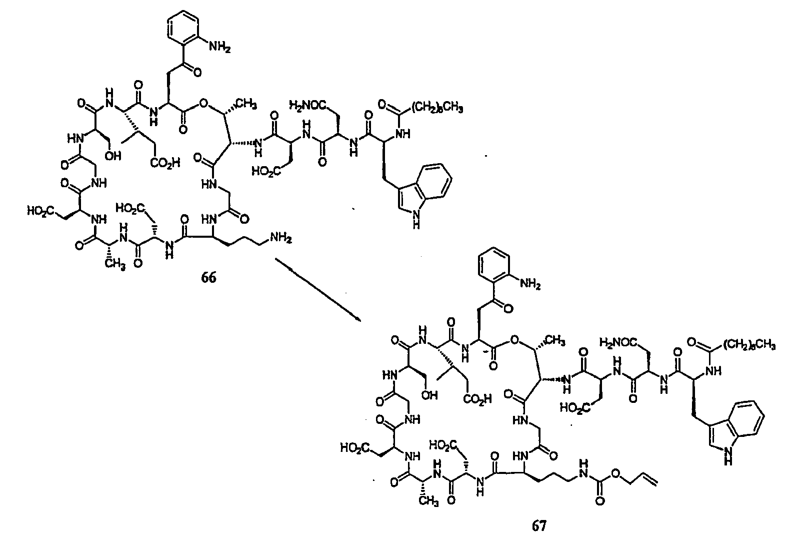

To a solution of daptomycin 66 (10 g) in dry N, N'-dimethylformamide (40 mL) at 0 °C was added allyl-1-benzotriazolylcarbonate (13.5 g). The reaction mixture was allowed to warm up to room temperature and stirred for 18 hours. The mixture was diluted with water (200 mL) then loaded on Bondesil 40µM C8 resin (400 g) that had been prewashed with methanol (1 L) and water (1 L). The resin was washed with water (1 L) and the product was eluted with methanol (1 L). Evaporation of the methanol gave compound 67 as a yellow solid (1 g).

### Example 18: Preparation of Compound 68:

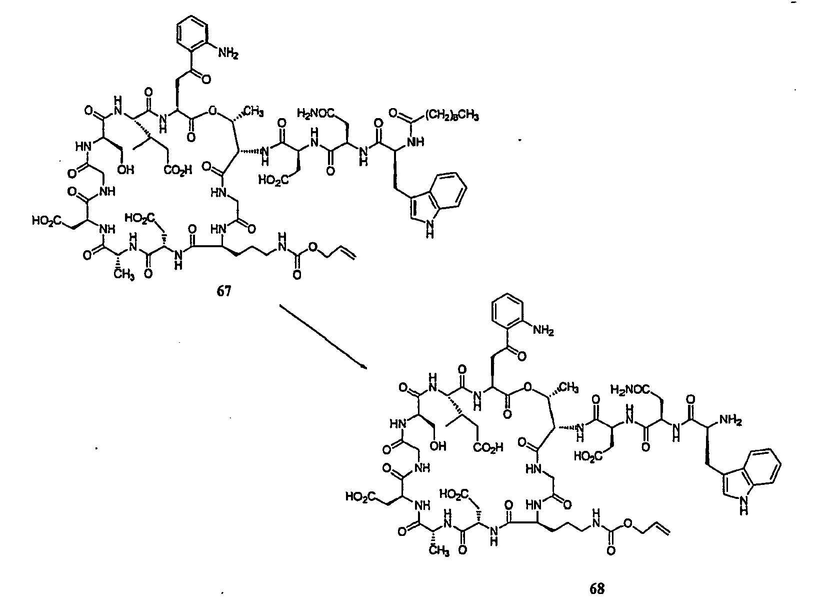

A preparation of deacylase enzyme was produced from recombinant *Streptomyces lividans,* which expresses the *Actinoplanes utahensis* deacylase enzyme. The enzyme in aqueous ethylene glycol (10 mL) was added to a solution of compound 67 (15 g in water, 1.9 L) at pH 8. The reaction mixture was stirred at room temperature for 18 hours and the pH was adjusted to 8 using 1 M sodium hydroxide. The reaction mixture was poured on to Bondesil 40µM C8 resin (400 g) that had been prewashed with methanol (1 L) and water (1 L). The product was eluted with 20% acetonitrile in water (1 L) and freeze-dried to give compound 68 as a yellow solid (9.1 g).

### Example 19: Edman Degradation to Remove Tryptophan

### Preparation of Compound 70:

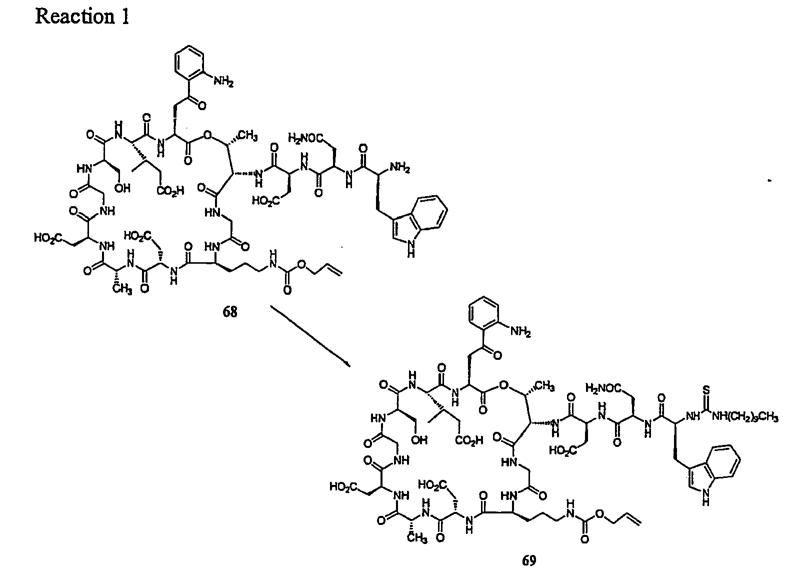

To a suspension of compound 68 (9.1 g) in dry N, N'-dimethylformamide (15 mL) was added n-decylisothiocyanate (1.2 mL). The reaction mixture was stirred at room temperature for 18 hours. The reaction mixture was poured on to Bondesil 40µM C8 resin (400 g) that had been pre-washed with methanol (1L) and water (1 L). The product was eluted with methanol (800 mL) after being first washed with water (800 mL) followed by 20% acetonitrile in water (800 mL). Evaporation of the methanol gave compound 69 as a yellow solid (7.3 g).

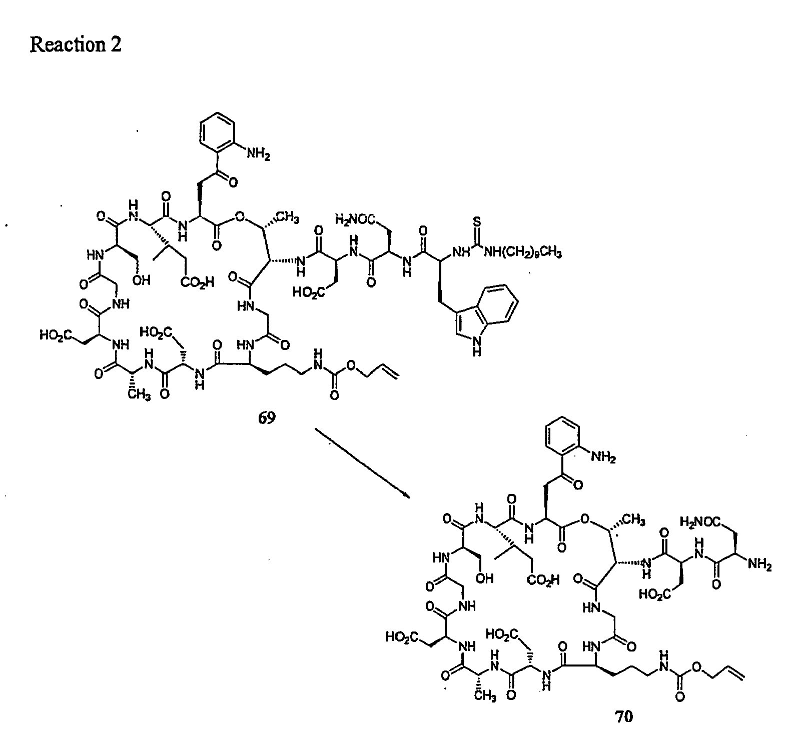

Compound 69 (7.3 g) was stirred at room temperature in 25% trifluoroacetic acid in dry dichloromethane (30 mL) for 2 hours before being evaporated to dryness. The residue was dissolved in water (50 mL) poured on to Bondesil 40µM C8 resin (400 g) that had been pre-washed with methanol (1 L) and water (1 L). The product was eluted with a 20 to 40% acetonitrile in water gradient and freeze-dried to give compound 70 as a yellow solid (1.05 g).

### Example 20: Edman Degradation to Remove Asparagine

### Preparation of Desasparagine Compound 72:

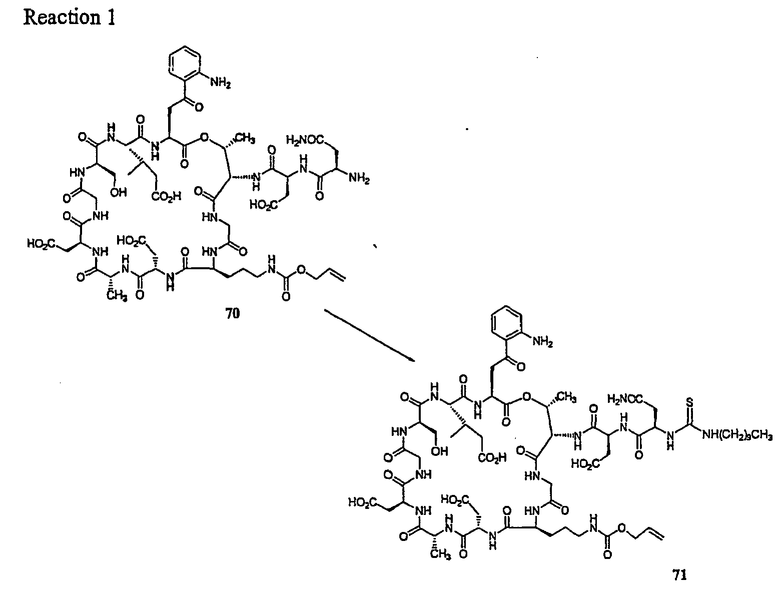

To a suspension of compound 70 (0.57 g) in dry N, N'-dimethylformamide (5 mL) was added n-decylisothiocyanate (0.16 mL). The reaction mixture was stirred at room temperature for 18 hours before evaporation to dryness. The residue was tritrated with diethylether (5 mL) to give compound 71 as a yellow solid (0.54 g).

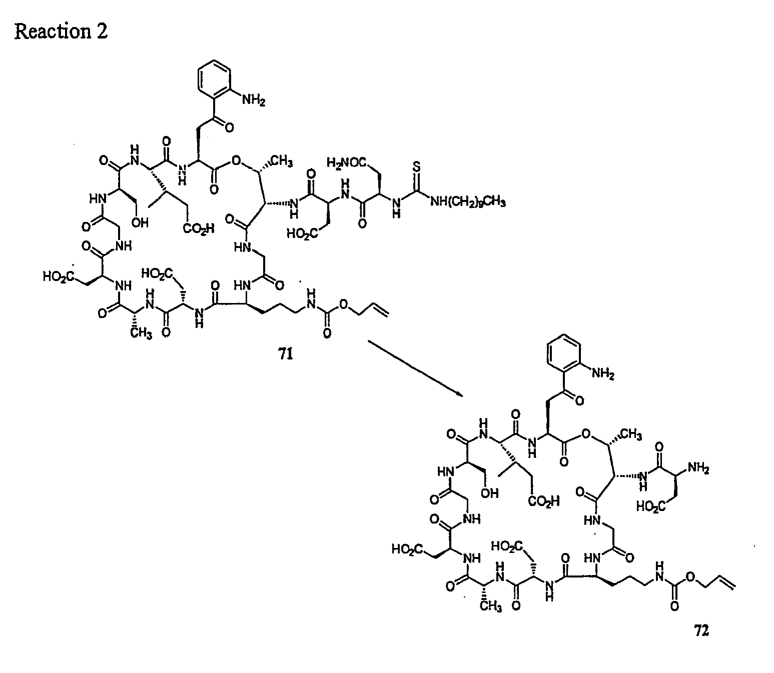

Compound 71 (0.54 g) was stirred in 50% trifluoroacetic acid in dry dichloromethane (4 mL) for 2 hours before evaporation to dryness. The residue was dissolved in water (25 mL) poured on to Bondesil 40µM C8 resin (50 g) that had been pre-washed with methanol (100 mL) and water (100 mL). The product was eluted with 20% acetonitrile in water after first being washed with water (100 mL). The eluent was evaporated to dryness to give compound 72 as a yellow solid (0.40 g).

### Example 21: Edman Degradation to Remove Aspatic Acid

### Preparation of Core Compound 74:

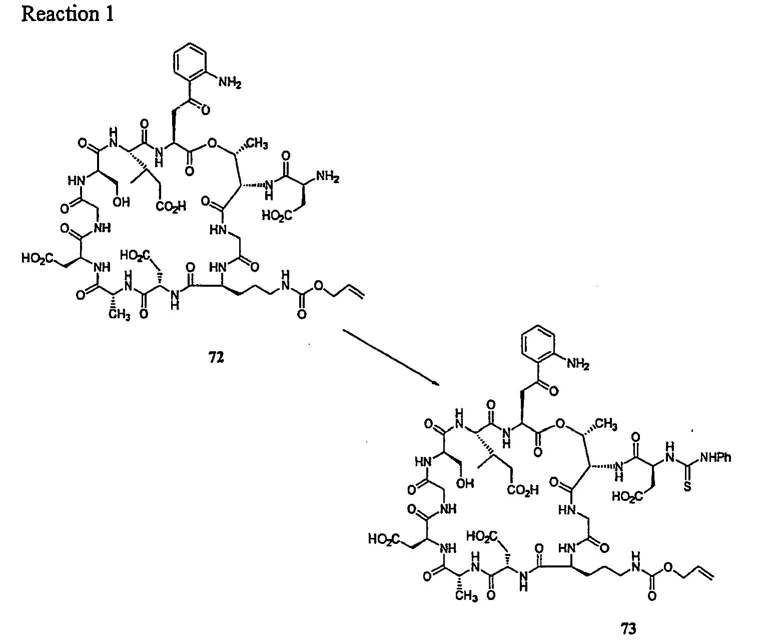

Phenylisothiocyanate (1 mmol) is added to a suspension of compound 72 (1.0 mmol) in dry N, N'-dimethylformamide (5 mL). The reaction is stirred at ambient temperature for 18 hours, then evaporated to dryness. The residue is titurated with diethyl ether (5 mL) to give the product 73.

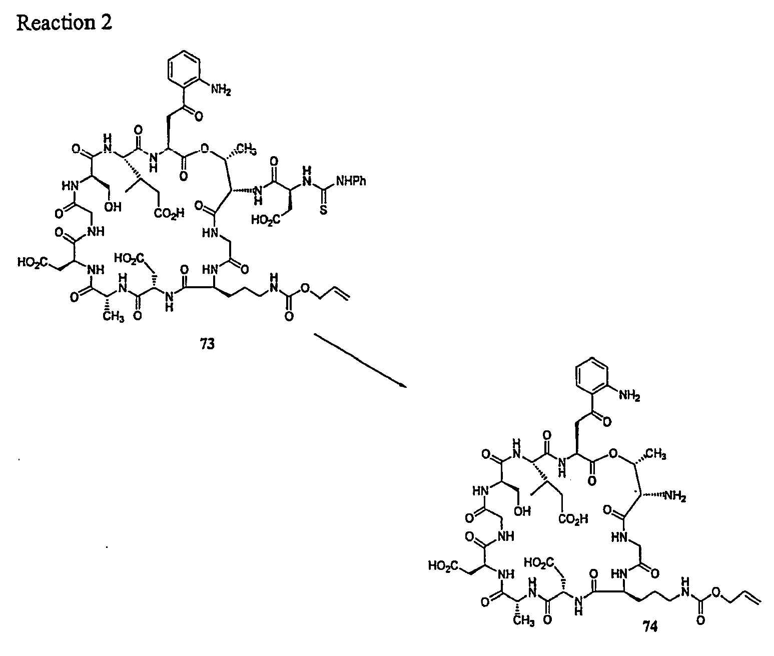

Compound 73 is stirred in dichloromethane (2 mL) and trifluoroacetic acid (2 mL) for 2 hours, before being evaporated to dryness to give the product as the triflouroacetic acid salt 74.

### Example 22: Preparation of Compound 76:

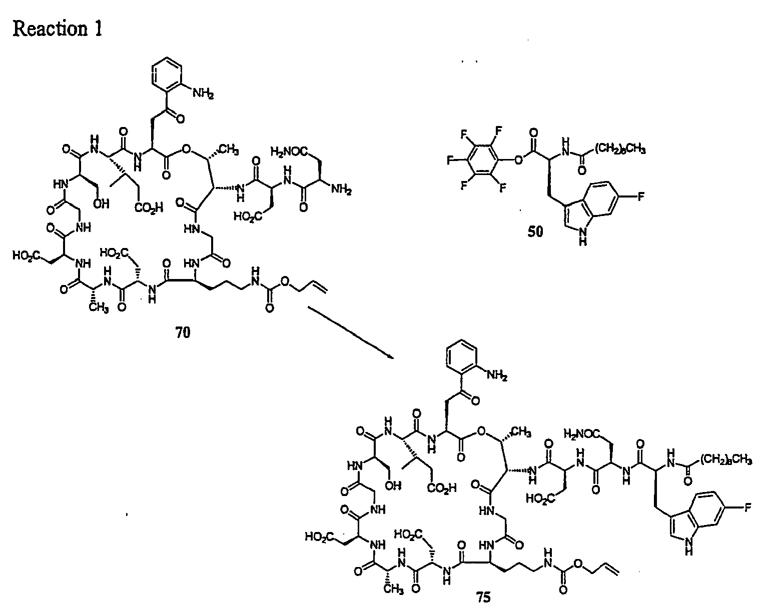

Compound 50 (397 mg) was added to a solution of the deacylated destrptophan daptomycin 70 (500 mg), and diisopropylethylamine (2-3 drops) in N,N'-dimethylformamide (5 mL). The reaction was monitored by analytical reverse phase HPLC (eluting with a gradient from 5% to 95% acetonitrile 0.1% formic acid in water 0.1%formic acid using a luna C18 5uM 150 x 3 mm) and complete consumption of compound 70 was observed after stirring for 16 hours. The solvent was removed by evaporation and the crude product 75 (631 mg) was carried forward in the next step.

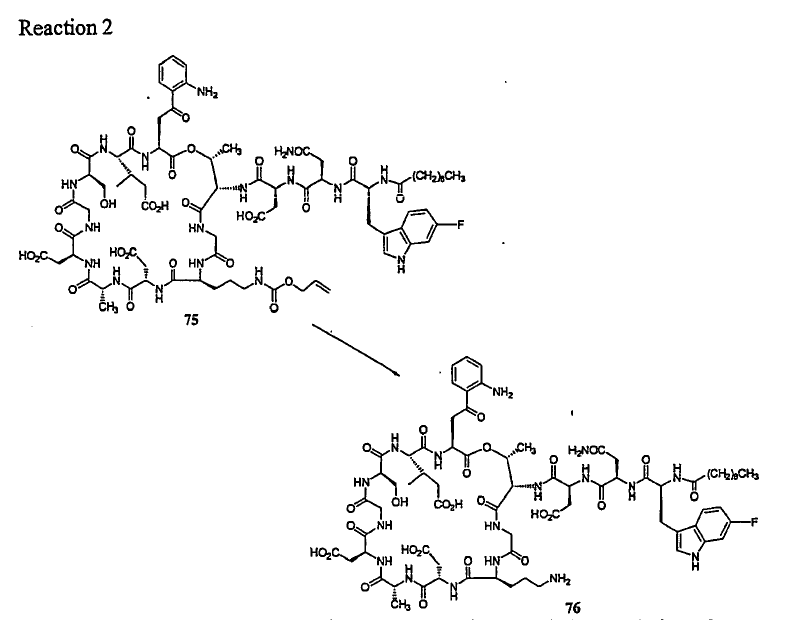

Tetrakis-(triphenylphosphine)palladium(0), (631 mg) was added to a solution of Compound 75 (631 mg); N-methyl morpholine (631 µl) in dioxane (10 mL) and 1 N hydrochloric acid (6.31 mL). After stirring for 16 hours the reaction was filtered, and purified by reverse phase HPLC using C1810 uM Jupiter column 250 x 21.2mm, eluting with a gradient from acetonitrile:water:formic acid 30:70:0.1 to acetonitrile:water:formic acid 90:10:0.1 over 25 minutes. Evaporation of the product bearing fractions gave the product 76 (44 mg).

### Example 23: Preparation of Compound 79:

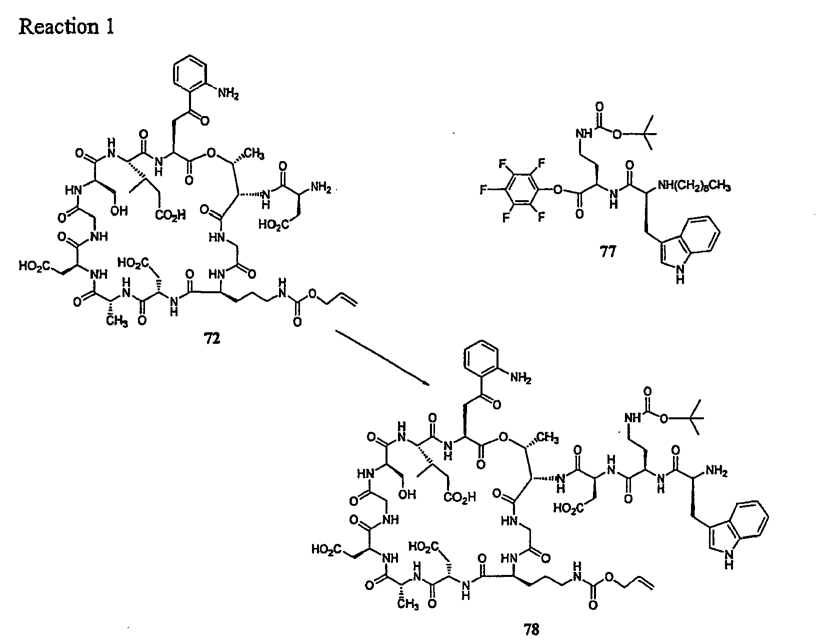

Compound 77 (1.2 mmol, see example 16 (*vide supra*)) is added to a solution of the deacylated destryptophan-desasparagine daptomycin 72 (1.0 mmol) and diisopropylethylamine (2-3 drops) in dry dimethylformamide (5 mL). The reaction is stirred at ambient temperature until all the starting material is consumed as determined by analytical reverse phase HPLC (eluting with a gradient from 5% to 95% acetonitrile 0.1% formic acid in water 0.1% formic acid using a luna C18 5uM 150 x 3 mm) The mixture is evaporated to dryness and the residue is titurated with ether (5 mL) to give the desired product 78.

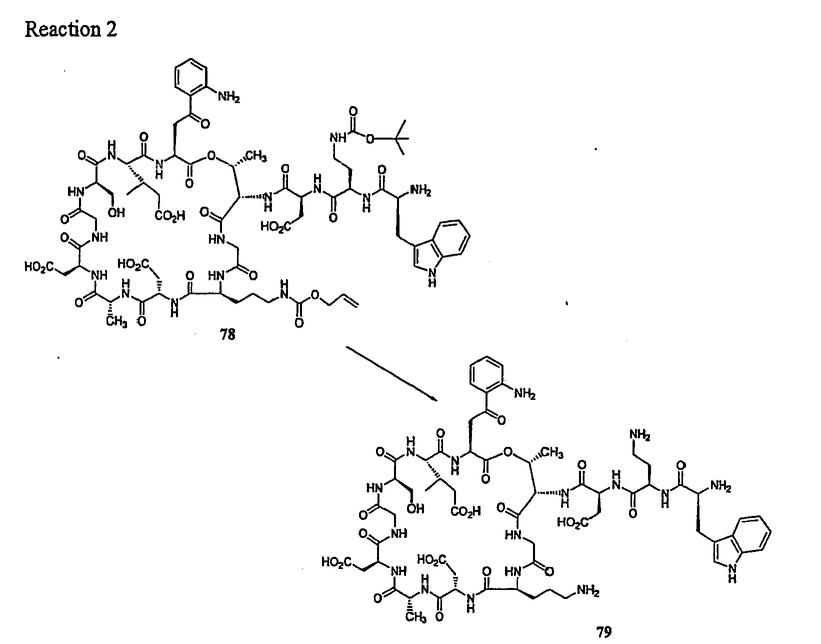

To a solution of compound 78 (1 mmol) in 0.5 M hydrochloric acid (1 mL) and dioxane (3 mL), is added N-methylmorpholine (0.1 mL) and tetrakis-(triphenylphosphine)-palladium(0) (100 mg). The reaction is stirred under argon for 24 hours, filtered and concentrated to give a crude semi solid. The residue is dissolved in dry dichloromethane (4 mL). Triisopropylsilane (0.1 mL) and trifluoroacetic acid (1 mL) are added and the reaction is stirred for 2 hours before being evaporated to dryness. The residue is then purified by preparative HPLC with a 250 x 21.2mm Jupiter 10µm C8 column using a 30-80% acetonitrile gradient in 0.1 % aqueous formic acid as eluent Evaporation of the solvent from the product bearing fractions gives the desired product 79.

### Example 24

### Biological Activity

Compounds according to Formula I were tested for antimicrobial activity against a panel of organisms according to standard procedures described by the National Committee for Clinical Laboratory Standards (NCCLS document M7-A5, Vol. 20, No. 2, 2000) except that all testing was performed at 37 °C. Compounds were dissolved in 100% dimethyl sulfoxide and were diluted to the final reaction concentration (0.1 µg/mL-100 µg/mL) in microbial growth media. In all cases the final concentration of dimethyl sulfoxide incubated with cells is less than or equal to 1%. For minimum inhibitory concentration (MIC) calculations, 2-fold dilutions of compounds were added to wells of a microtiter plate containing 5x10⁴ bacteria cells in a final volume of 100 µL of media (Mueller-Hinton Broth supplemented with 50 mg/L Ca²). The optical densities (OD) of the bacterial cells, which measures bacterial cell growth and proliferation, were measured using a commercial plate reader. The MIC value is defined as the lowest compound concentration inhibiting growth of the test organism. The MIC (in µg/ml) value of representative compounds of the present invention are listed in Table III.

**Table III: Biological Activity of Compounds of Formula I**

| | | | | |
|---|---|---|---|---|
| 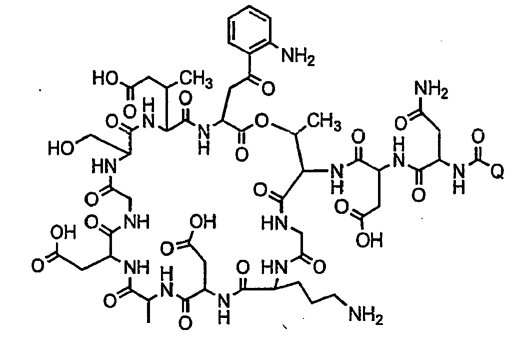 | | | | |

| Q | SA 399 | SA 42 | Efm 14 | Efs 201 |
|---|---|---|---|---|
| 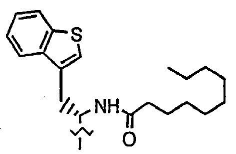 | +++ | +++ | ++ | ++ |
| 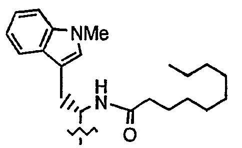 | ++ | ++ | + | ++ |
| 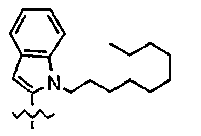 | ++ | ++ | ++ | ++ |
| 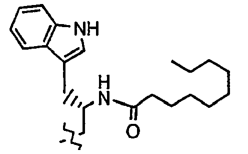 | ++ | ++ | + | + |
| 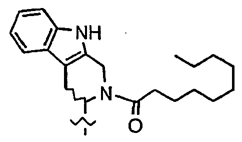 | ++ | ++ | ++ | ++ |
| 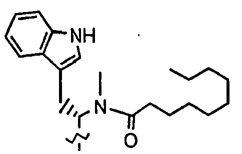 | +++ | +++ | ++ | ++ |
| 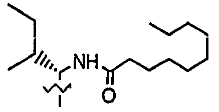 | | ++ | + | + |
| 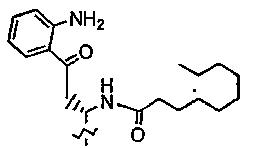 | ++ | ++ | + | ++ |
| 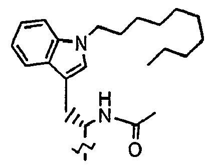 | ++ | ++ | ++ | ++ |
| 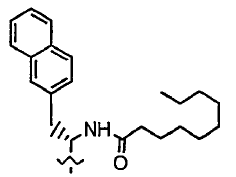 | ++ | ++ | + | ++ |
| 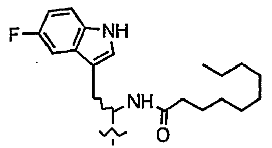 | +++ | +++ | ++ | ++ |
| 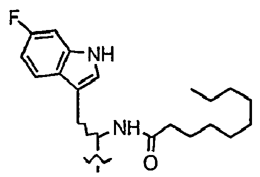 | +++ | +++ | ++ | ++ |
| 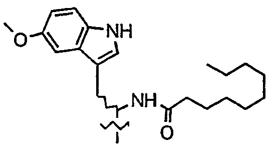 | | ++ | + | + |

| | | | | |
|---|---|---|---|---|
| SA399 is Methicillin Resistant *Staphylococcus aureus.* SA42 is Wild Type *Staphylococcus aureus.* EF14 is Wild Type *Enterococcus faecium*. EF201 is Wild Type *Enterococcus faecalis.* | | | | |

Wherein "+++" indicates that the compound has an MIC (µg/ml) of 1 µg/ml or less or an ED₅₀ of 1mg/kg or less;

"++" indicates that the compound has an MIC µg/ml) or ED₅₀ of greater than 1 µg/ml or 1 mg/kg, respectively but less than or equal to 10 µg/ml or ED₅₀ of 10 mg/kg, respectively; and

"+" indicates that the compound has an MIC (µg/ml) of greater than 10 µg/ml or an ED₅₀ of greater than 10 mg/kg.

### Example 25

### In Vivo Activity

The mouse protection test is an industry standard for measuring the efficacy of a test compound *in vivo* [for examples of this model see J. J. Clement, et al., Antimicrobial Agents and Chemotherapy, 38 (5), 1071-1078, (1994)]. As exemplified below, this test is used to demonstrate the *in vivo* efficacy of the compounds of the present invention against bacteria.

The *in vivo* antibacterial activity is established by infecting female CD-1 mice (Charles River Lab, MA) weighing 19-23 g intraperitoneally with Methicillin Resistant *S. aureus* (MRSA) inoculum. The inoculum is prepared from Methicillin Resistant *S. aureus* (ATCC 43300). The MRSA inoculum is cultured in Mueller-Hinton (MH) broth at 37°C for 18 hours. The optical density at 600 nm (OD₆₀₀) is determined for a 1:10 dilution of the overnight culture. Bacteria (8 x 10⁸ cfu) is added to 20 ml of phosphate buffered saline (Sigma P-0261) containing 5 % hog gastric mucin (Sigma M-2378). All animals are injected with 0.5 ml of the inoculum, equivalent to 2 x 10⁷ cfu/mouse, which is the dose causing ~100% death of the animals without treatment.

The test compound is dissolved in 10.0 ml of 50mM phosphate buffer to give a solution of 1 mg/ml (pH = 7.0). This solution is serially diluted with vehicle by 4-fold (1.5 ml to 6.0 ml) to give 025, 0.063 and 0.016 mg/ml solutions. All the solutions are filtered with 0.2 m Nalgene syringe filter. Immediately after the bacterial inoculation, group 1 animals are subcutaneously (sc) injected with buffer (no test compound) and groups 2 to 5 were given test compound sc at 10.0, 2.5, 0.63, and 0.16 mg/kg, respectively. Group 6 animals receive test compound sc at 10 mg/kg (or the highest therapeutic dose of a given compound) only for monitoring acute toxicity. These injections are repeated once at 4 hours after the inoculation for the respective groups. The injection volume at each time is 10 ml per kilogram of body weight The 50% protective dose (PD₅₀) is calculated on the basis of the number of mice surviving 7 days after inoculation.

All publications and patent applications cited in this specification are herein incorporated by reference as if each individual publication or patent application were specifically and individually indicated to be incorporated by reference. Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

## Claims

**1.** A composition comprising a compound of the Formula: 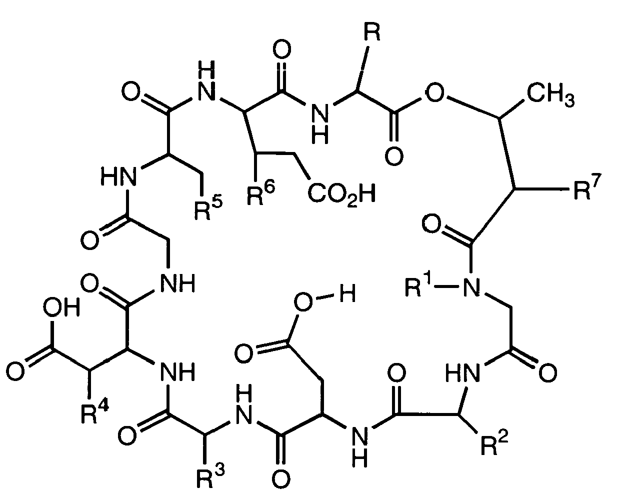 or a salts thereof; wherein:
(a) R is 2-butyl, isopropyl or 2-(2'-aminophenacyl);
(b) each of R¹and R⁶ is independently hydrido or methyl;
(c) R² is methyl or -CH₂CH₂CH₂R⁸;
(d) R³ is methyl or -CH₂CH₂CH₂CH₂R⁹;
(e) R⁴ is hydrido or methoxy;
(f) R⁵ is hydroxy or carboxyamino;
(g) each of R⁷, R⁸ and R⁹ is independently amino, monosubstituted amino, disubstituted amino, acylamino, ureido, guanidino, carbamoyl, sulfonamino, thioacylamino, thioureido, iminoamino, or phosphonamino;
(h) provided that
(1) when R² is -CH₂CH₂CH₂R⁸, R⁷ is other than 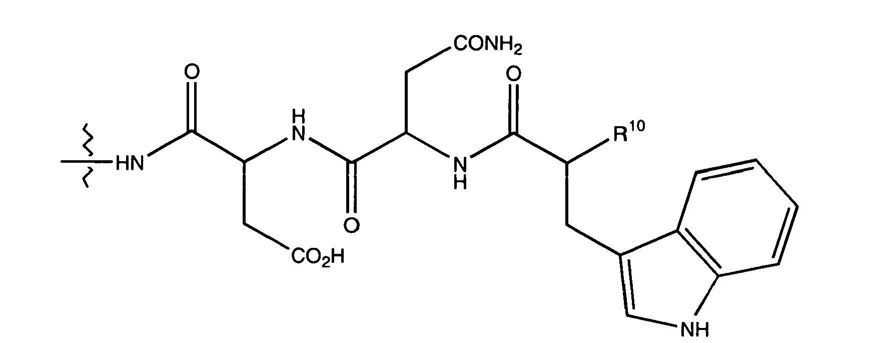 wherein R¹⁰ is amino, monosubstituted amino, disubstituted amino, acylamino, ureido, guanidino, carbamoyl, sulfonamino, thioacylamino, iminoamino, or phosphonamino;
(2) when R² is methyl, R⁷ is other than 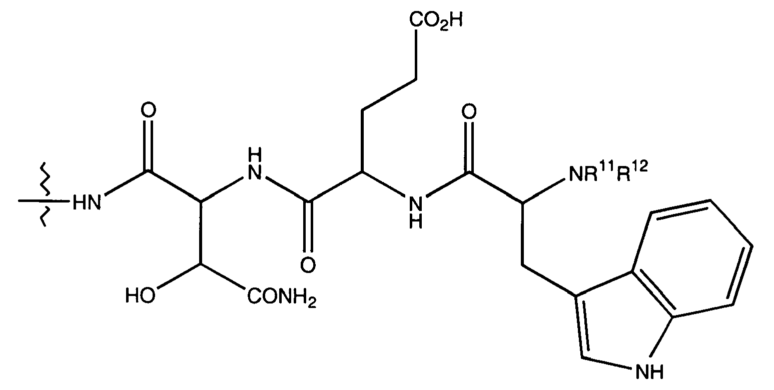 wherein each of R¹¹ and R¹² is hydrido, C₆-C₁₈ unsubstituted alkanoyl, C₈-C₁₈ unsubstituted alkenoyl, C₈-C₁₈ unsubstituted alkyl, or C₈-C₁₈ select substituted alkyl; or alternatively, R¹¹ and R¹² together are C₈-C₁₈ alkylidenyl.

**2.** The composition of Claim 1 wherein R is 2-(2'-aminophenacyl); each of R¹ and R⁴ is hydrido; R² is -CH₂CH₂CH₂R⁸; each of R³ and R⁶ is methyl; and R⁵ is hydroxyl.

**3.** The composition of Claim 1 wherein: R is 2-butyl or isopropyl; each of R¹ and R² is methyl; R³ is -CH₂CH₂CH₂CH₂R⁹; R⁴ is methoxy, and R⁵ is carboxyamino.

**4.** The composition according to any of Claims 1, 2 or 3, wherein R⁷ is 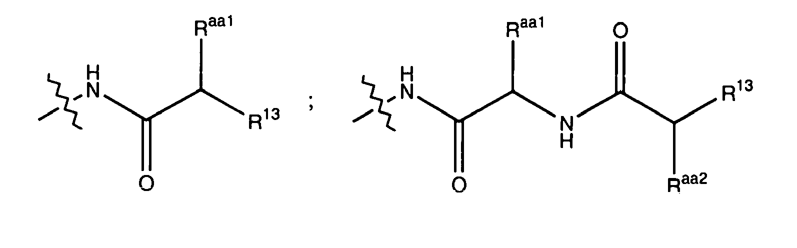 or 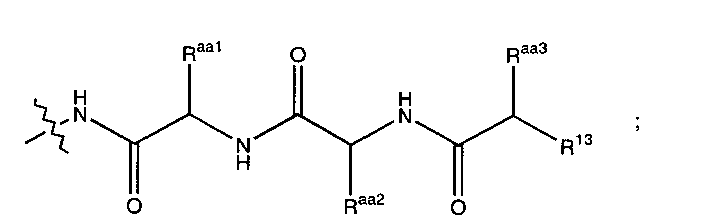 wherein each of R^{aa}, R^{aa2} and R^{aa3} is independently an amino acid side chain and wherein R¹³ is amino, monosubstituted amino, disubstituted amino, acylamino, ureido, guanidino, carbamoyl, sulfonamino, thioacylamino, thioureido, iminoamino, or phosphonamino.

**5.** A composition comprising a compound of the Formula 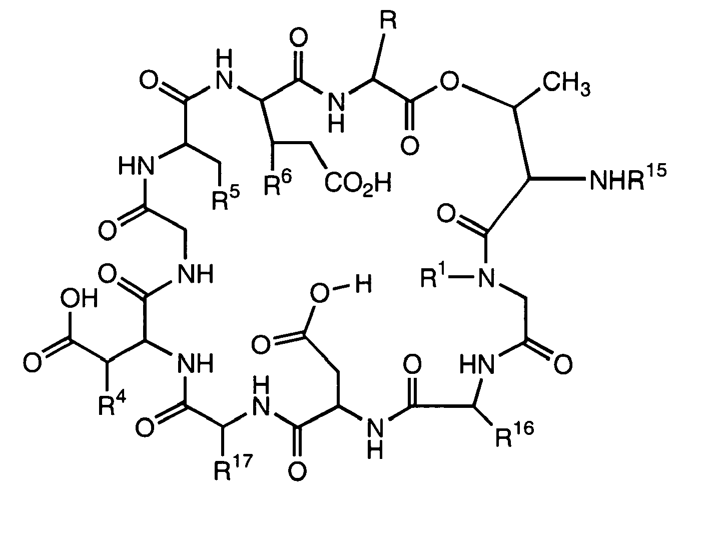 or a salt thereof; wherein:
(a) R is 2-butyl, isopropyl or 2-(2'-aminophenacyl) ;
(b) each of R¹ and R⁶ is independently hydrido or methyl;
(c) R⁴ is hydrido or methoxy;
(d) R⁵ is hydroxy or carboxyamino;
(e) R¹⁵ is hydrido, 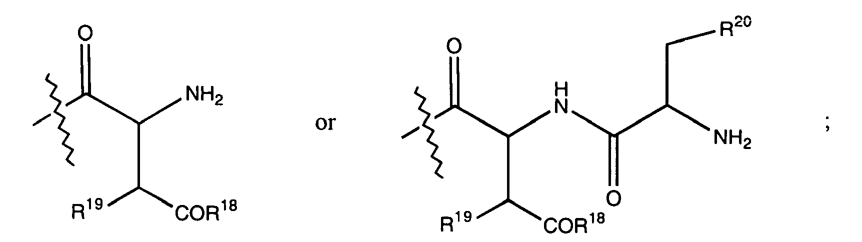 wherein: R¹⁸ is amino or hydroxy; R¹⁹ is hydrido or hydroxy; and R²⁰ is carboxyamino or carboxymethyl;
(f) R¹⁶ is methyl or -CH₂CH₂CH₂R²¹;
(g) R¹⁷ is methyl or-CH₂CH₂CH₂CH₂R²²;
wherein each of R²¹ and R²² is independently amino, monosubstituted amino, disubstituted amino, acylamino, ureido, guanidino, carbamoyl, sulfonamino, thioacylamino, thioureido, iminoamino, or phosphonamino.

**6.** The composition of Claim 5 wherein the compound of Formula I is 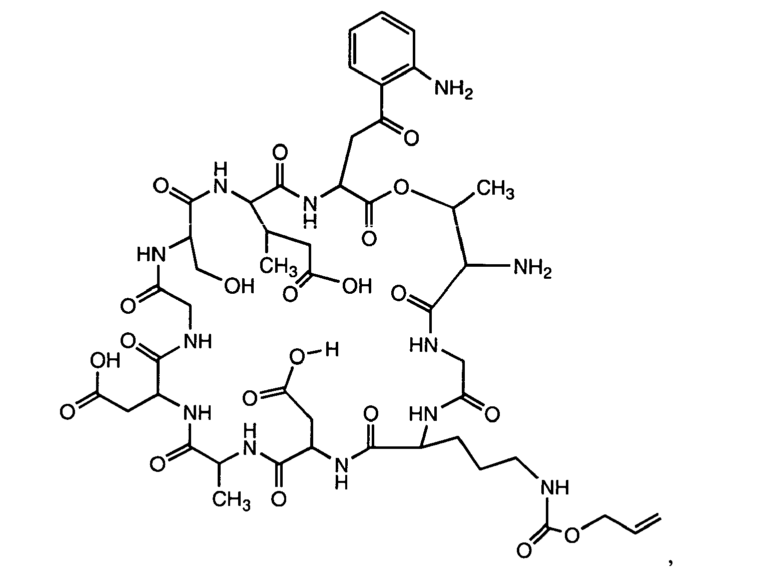 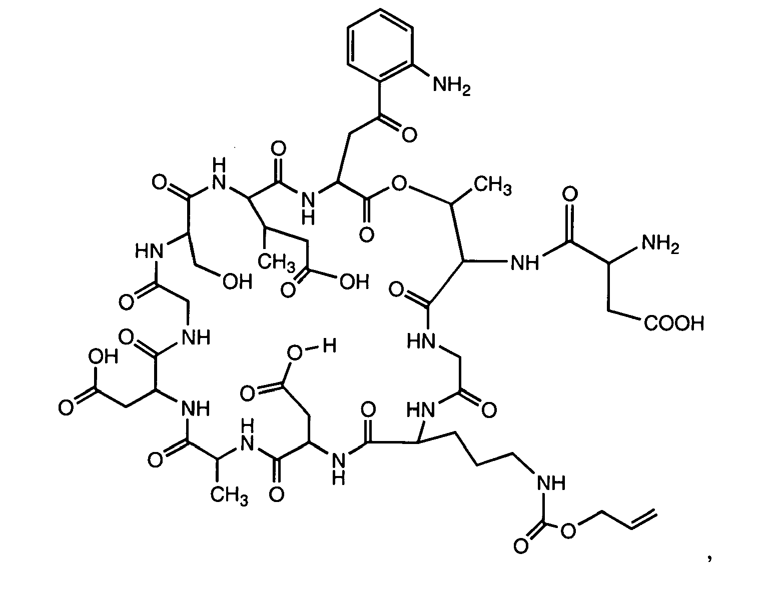 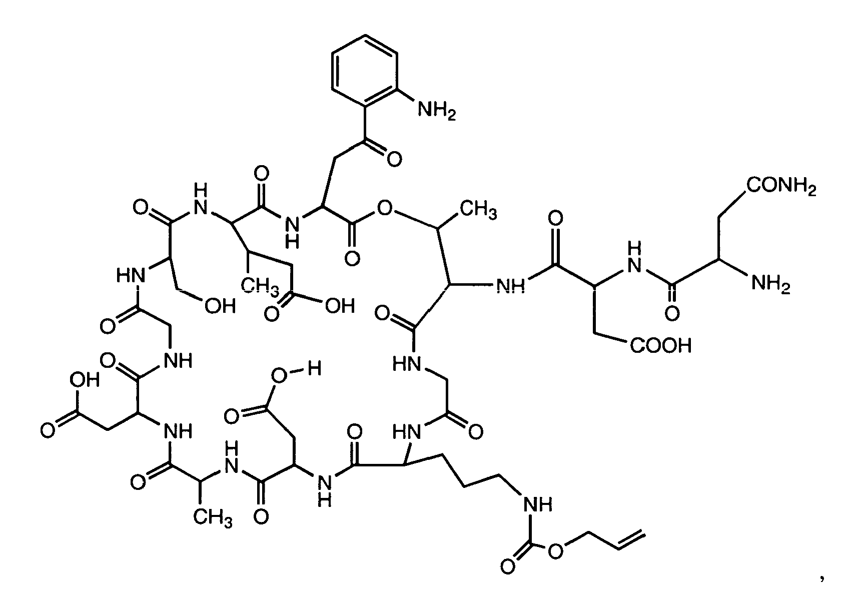 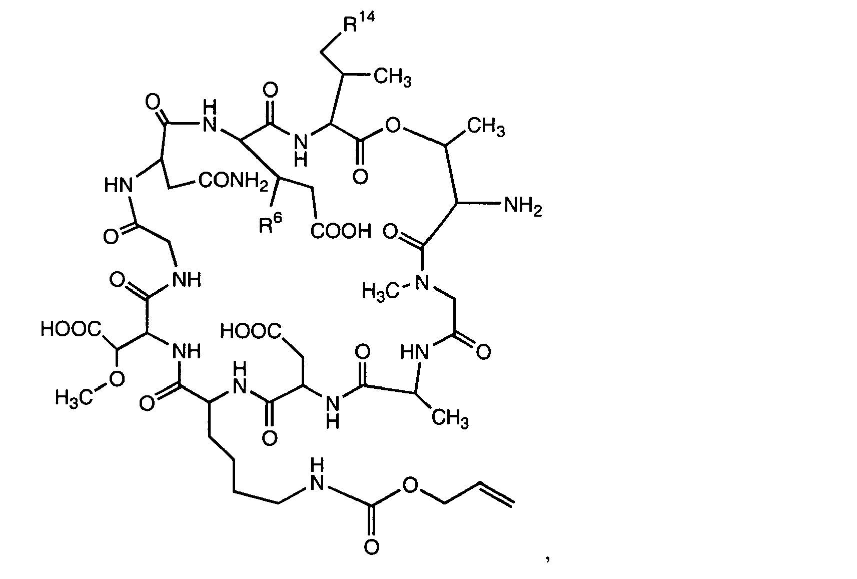 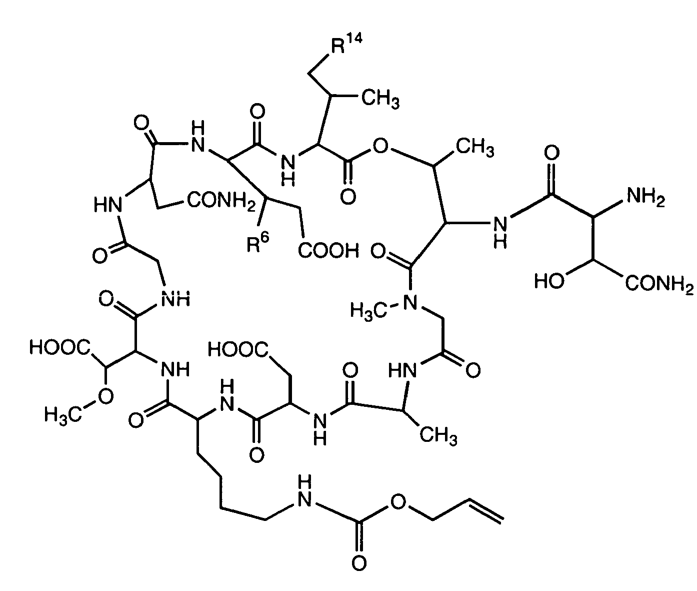 or 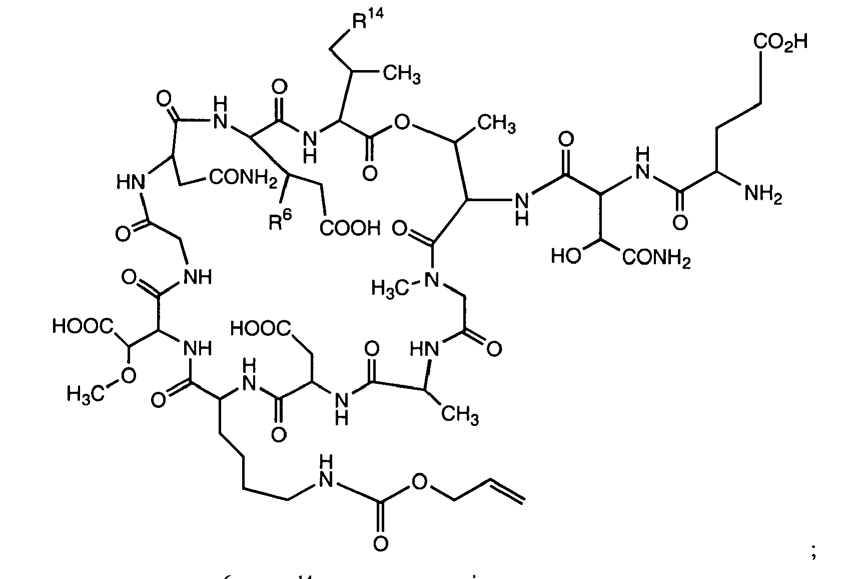 wherein each of R⁶ and R¹⁴ is independently hydrido or methyl.

**7.** Use of the composition of any of Claims 1, 2, 3 or 4 for the manufacture of a medicament for the treatment of a bacterial infection in a subject.

**8.** A process for preparing a compound of Formula I: 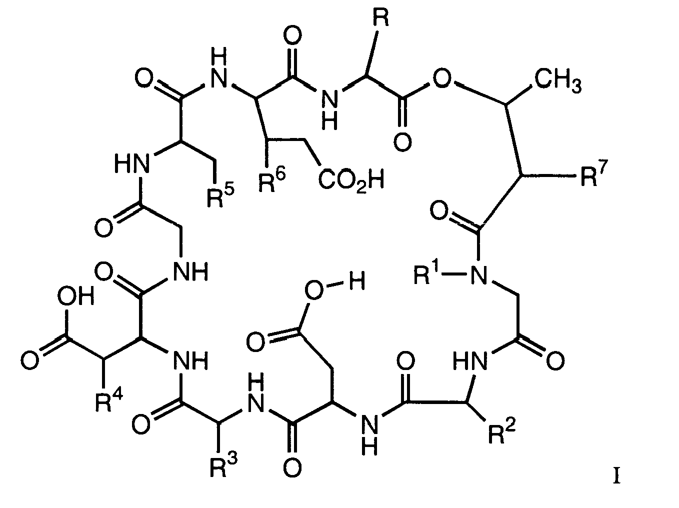 or a salt thereof; wherein:
(a) R is 2-butyl, isopropyl or 2-(2'-aminophenacyl);
(b) each of R¹ and R⁶ is independently hydrido or methyl;
(c) R² is methyl or -CH₂CH₂CH₂R⁸;
(d) R³ is methyl or -CH₂CH₂CH₂CH₂R⁹;
(e) R⁴ is hydrido or methoxy;
(f) R⁵ is hydroxy or carboxyamino;
(g) each of R⁷, R⁸ and R⁹ is independently amino, monosubstituted amino, disubstituted amino, acylamino, ureido, guanidino, carbamoyl, sulfonamino, thioacylamino, thioureido, iminoamino, or phosphonamino;
(h) provided that
(1) when R² is -CH₂CH₂CH₂R⁸, R⁷ is other than 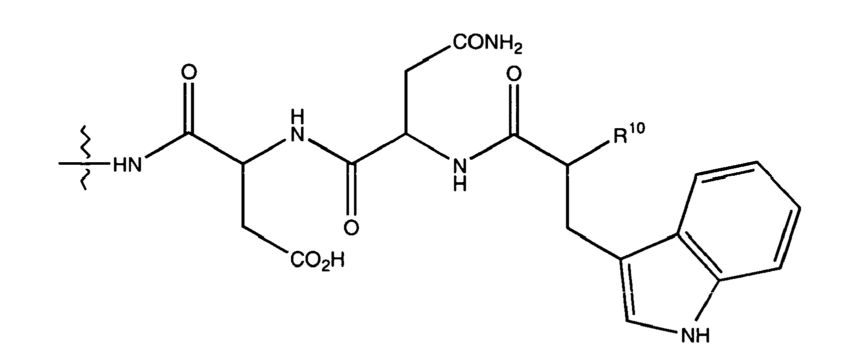 wherein R¹⁰ is amino, monosubstituted amino, disubstituted amino, acylamino, ureido, guanidino, carbamoyl, sulfonamino, thioacylamino, iminoamino, or phosphonamino;
(2) when R² is methyl, R⁷ is other than 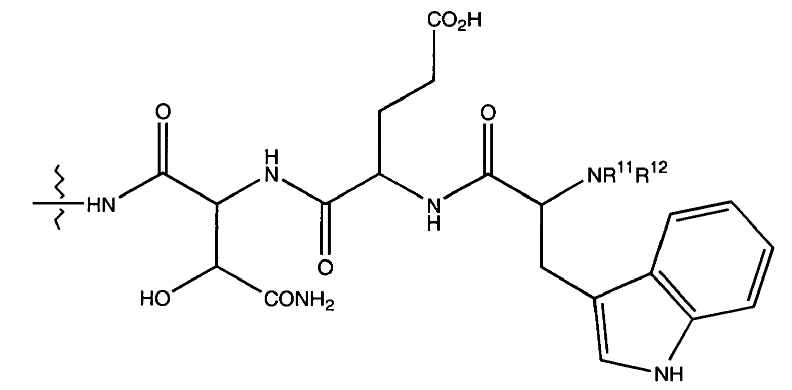
wherein each of R¹¹ and R¹² is hydrido, C₆-C₁₈ unsubstituted alkanoyl, C₈-C₁₈ unsubstituted alkenoyl, C₈-C₁₈ unsubstituted alkyl, or C₈-C₁₈ select substituted alkyl; or alternatively, R¹¹ and R¹² together are C₈-C₁₈ alkylidenyl ;
comprising removing an amino protecting group from a compound of Formula XVII, 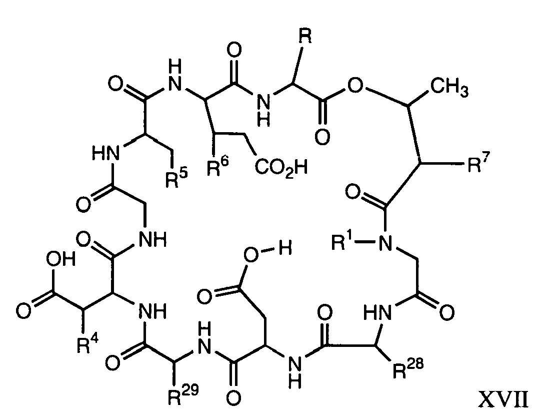 or a salt thereof,
wherein R²⁸ is methyl or -CH₂CH₂CH₂NHP; R²⁹ is methyl or-CH₂CH₂CH₂CH₂NHP; and P is an amino protecting group; provided that at least one of R²⁸ or R²⁹ is other than methyl.

**9.** The process of Claim 8 further comprising treating a compound of the Formula XVI, 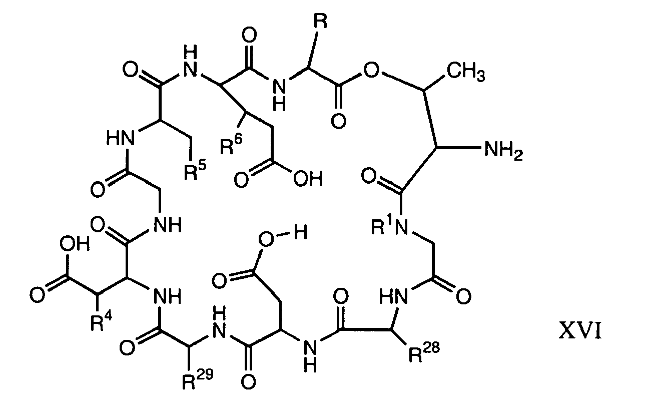 or a salt thereof,
with a modifying agent to obtain the compound of Formula XVII, or a salt thereof.

**10.** The process of Claim 9 further comprising removing the terminal amino acid residue from a compound of Formula XV, 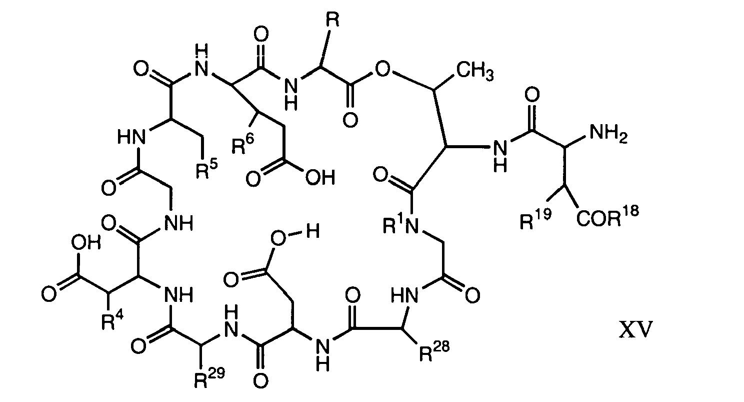 or a salt thereof,
wherein R¹⁸ is amino or hydroxy; R¹⁹ is hydrido or hydroxy;
to obtain the compound of Formula XVI, or a salt thereof.

**11.** The process of Claim 10 further comprising removing the terminal amino acid residue from a compound of Formula XIV 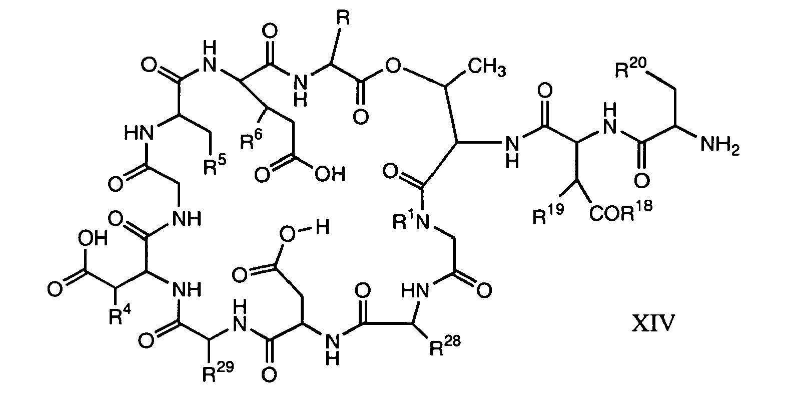 or a salt thereof,
wherein R²⁰ is carboxyamino or carboxymethyl;
to obtain the compound of Formula XV, or a salt thereof.

**12.** The process of Claim 11 further comprising removing the tryptophan amino acid residue from a compound of Formula XIII 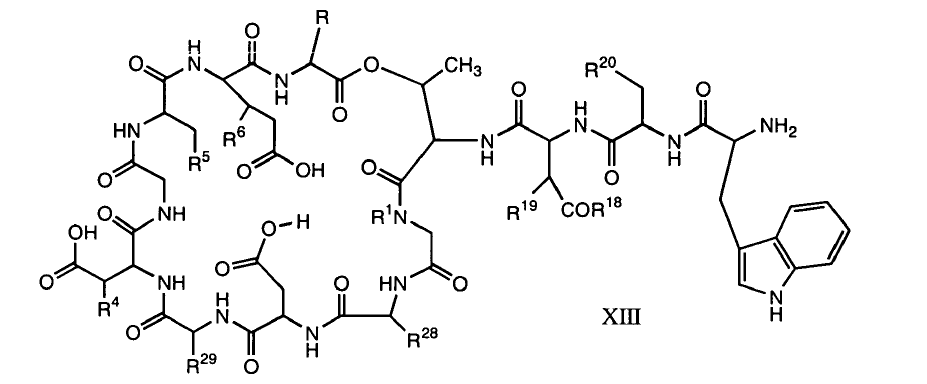 or a salt thereof,
to obtain the compound of Formula XIV, or a salt thereof.

**13.** The process of Claim 12 further comprising deacylating a compound of Formula XII 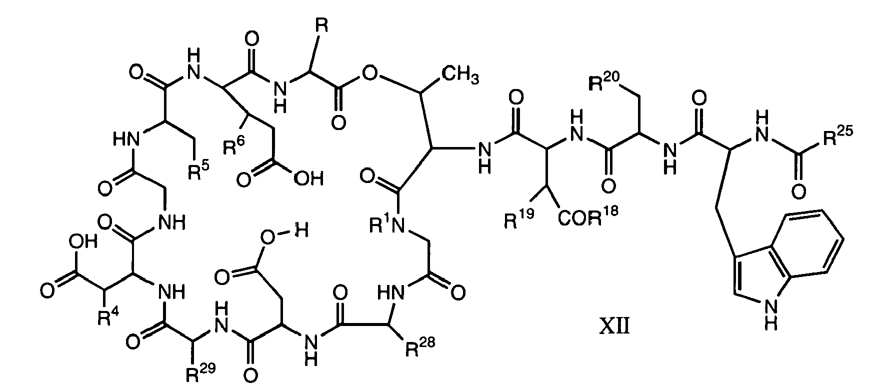 or a salt thereof;
wherein R²⁵ is an alkyl group;
to obtain the compound of Formula XIII, or a salt thereof.

**14.** The process of Claim 13 further comprising protecting the free amine group(s) of a compound of Formula XI 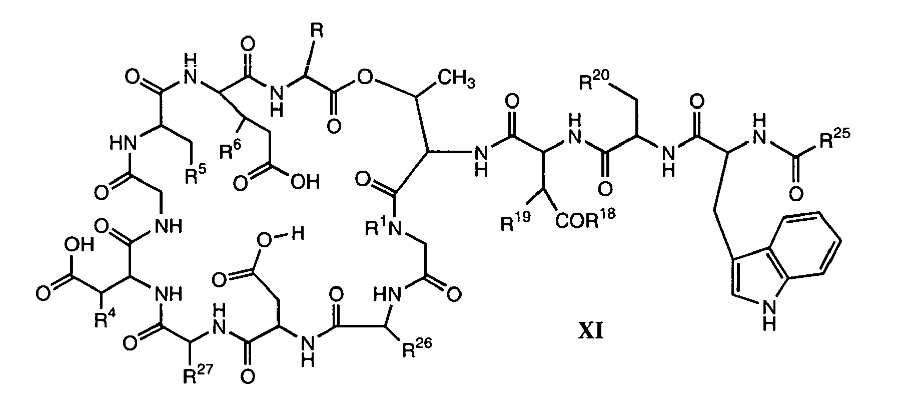 or a salt thereof;
wherein R²⁶ methyl or -CH₂CH₂CH₂NH₂; and R²⁷ is methyl or
-CH₂CH₂CH₂CH₂NH₂;
to obtain the compound of Formula XII, or a salt thereof.

**15.** A process for preparing a compound of Formula I: 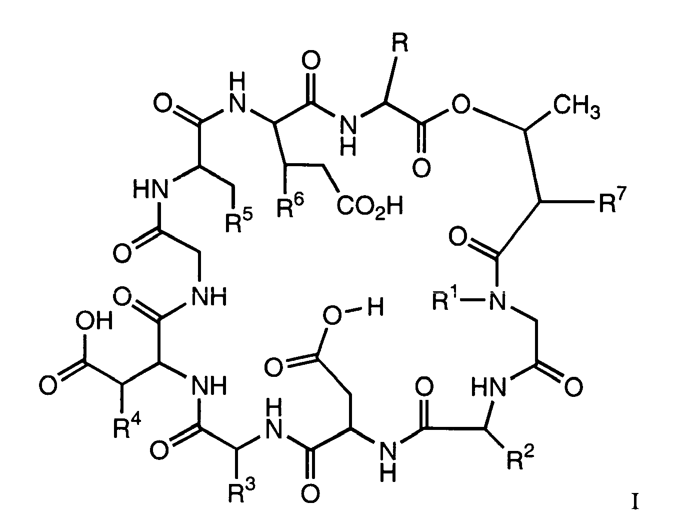 or a salt thereof; wherein:
(a) R is 2-butyl, isopropyl or 2-(2'-aminophenacyl);
(b) each of R¹ and R⁶ is independently hydrido or methyl;
(c) R² is methyl or -CH₂CH₂CH₂R⁸;
(d) R³ is methyl or -CH₂CH₂CH₂CH₂R⁹;
(e) R⁴ is hydrido or methoxy;
(f) R⁵ is hydroxy or carboxyamino;
(g) each of R⁷, R⁸ and R⁹ is independently amino, monosubstituted amino, disubstituted amino, acylamino, ureido, guanidino, carbamoyl, sulfonamino, thioacylamino, thioureido, iminoamino, or phosphonamino;
(h) provided that
(1) when R² is -CH₂CH₂CH₂R⁸, R⁷ is other than 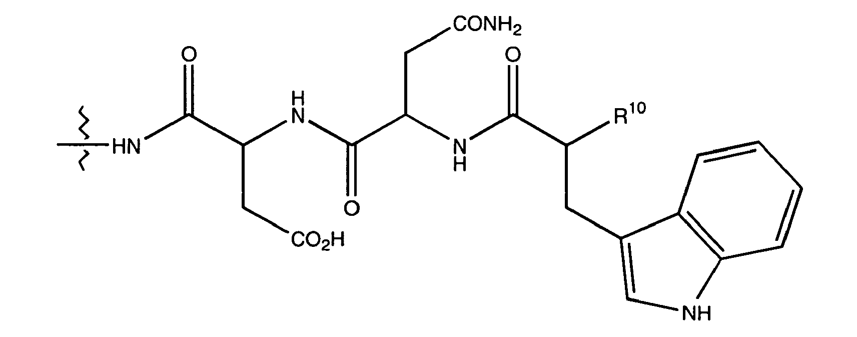 wherein R¹⁰ is amino, monosubstituted amino, disubstituted amino, acylamino, ureido, guanidino, carbamoyl, sulfonamino, thioacylamino, iminoamino, or phosphonamino;
(2) when R² is methyl, R⁷ is other than 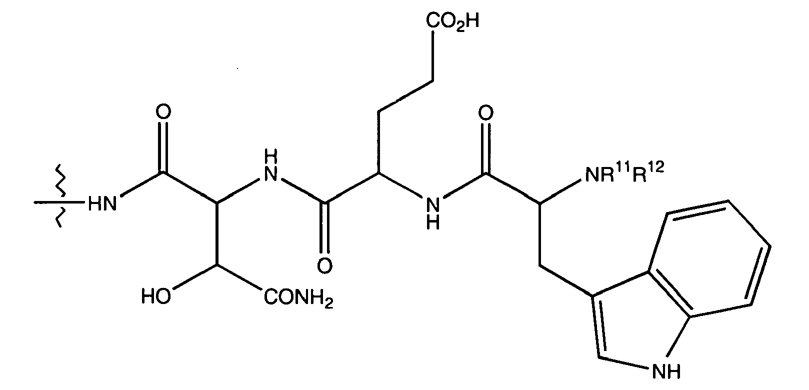
wherein each of R¹¹ and R¹² is hydrido, C₆-C₁₈ unsubstituted alkanoyl, C₈-C₁₈ unsubstituted alkenoyl, C₈-C₁₈ unsubstituted alkyl, or C₈-C₁₈ select substituted alkyl; or alternatively, R¹¹ and R¹² together are C₈-C₁₈ alkylidenyl;
comprising treating a compound of Formula Ia 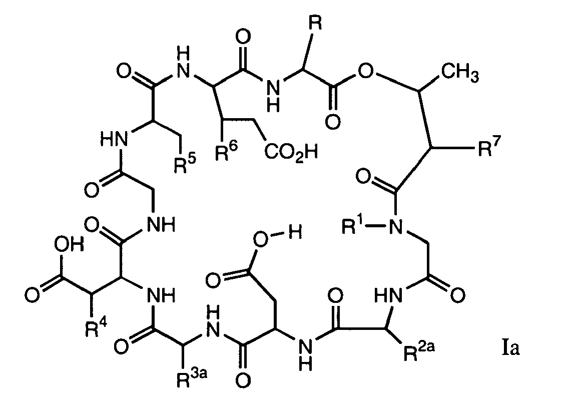 or salt thereof,
wherein R^{2a} is methyl or -CH₂CH₂CH₂NH₂; and R^{3a} is methyl or
-CH₂CH₂CH₂CH₂NH₂; provided at least one of R^{2a} and R^{3a} is other than methyl; with a modifying agent.

**16.** A process for preparing a compound of Formula I: 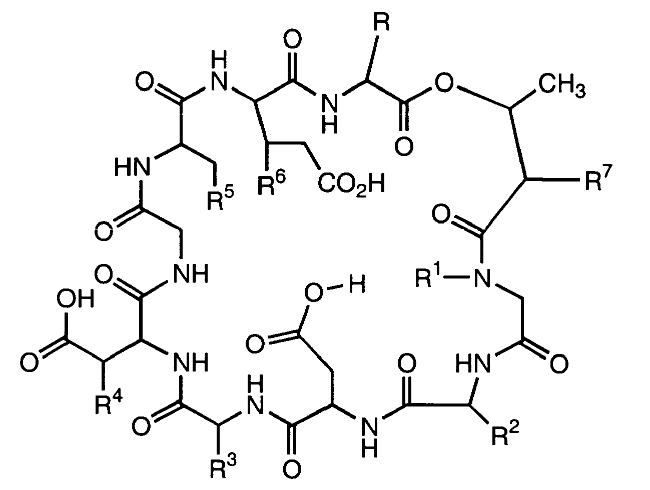 or a salt thereof; wherein:
(a) R is 2-butyl, isopropyl or 2-(2'-aminophenacyl);
(b) each of R¹ and R⁶ is independently hydrido or methyl;
(c) R² is methyl or -CH₂CH₂CH₂R⁸;
(d) R³ is methyl or -CH₂CH₂CH₂CH₂R⁹;
(e) R⁴ is hydrido or methoxy;
(f) R⁵ is hydroxy or carboxyamino;
(g) each of R⁷, R⁸ and R⁹ is independently amino, monosubstituted amino, disubstituted amino, acylamino, ureido, guanidino, carbamoyl, sulfonamino, thioacylamino, thioureido, iminoamino, or phosphonamino;
(h) provided that
(1) when R² is -CH₂CH₂CH₂R⁸, R⁷ is other than 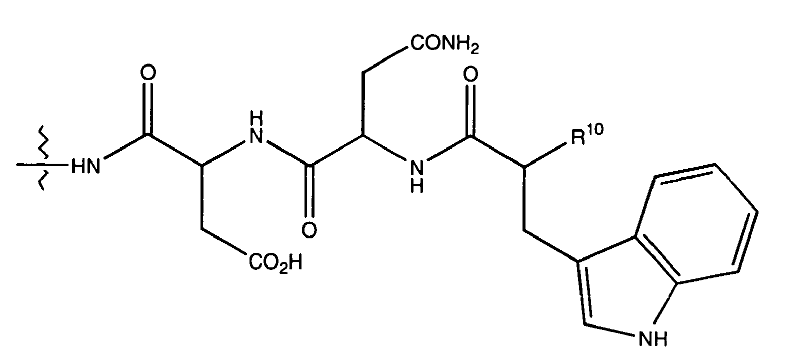 wherein R¹⁰ is amino, monosubstituted amino, disubstituted amino, acylamino, ureido, guanidino, carbamoyl, sulfonamino, thioacylamino, iminoamino, or phosphonamino;
(2) when R² is methyl, R⁷ is other than 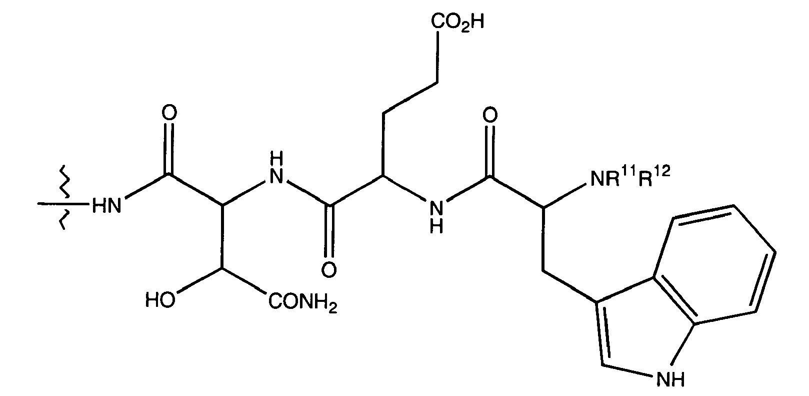
wherein each of R¹¹ and R¹² is hydrido, C₆-C₁₈ unsubstituted alkanoyl, C₈-C₁₈ unsubstituted alkenoyl, C₈-C₁₈ unsubstituted alkyl, or C₈-C₁₈ select substituted alkyl; or alternatively, R¹¹ and R¹² together are C₈-C₁₈ alkylidenyl;
comprising treating a compound of Formula XXVIII 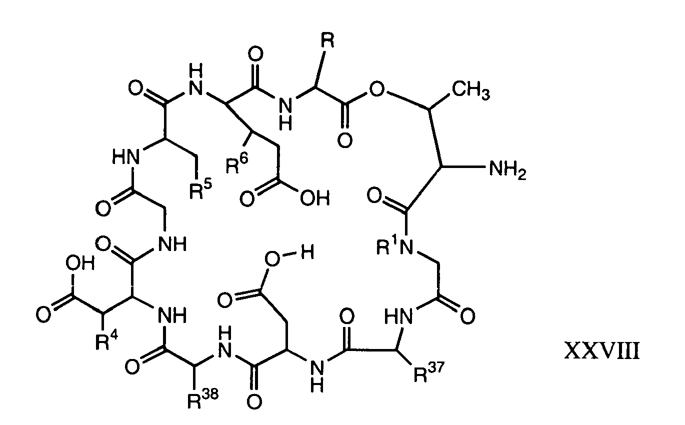 or a salt thereof;
wherein R³⁷ is methyl or -CH₂CH₂CH₂R⁸; and R³⁸ is methyl or-CH₂CH₂CH₂CH₂R⁹; provided that at least one of R³⁷ and R³⁸ is other than methyl and further provided that each of R⁸ and R⁹ is other than amino;
with a modifying agent.

**17.** The process of Claim 16 further comprising removing the terminal amino acid residue from a compound of Formula XXVII 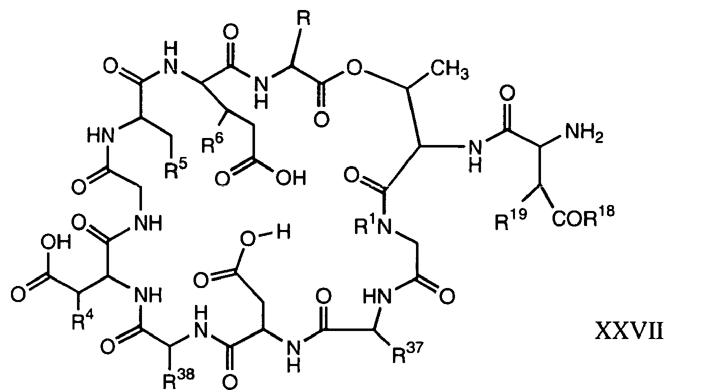 or a salt thereof;
wherein R¹⁸ is amino or hydroxy; and R¹⁹ is hydrido or hydroxy;
to obtain the compound of Formula XXVIII, or a salt thereof.

**18.** The process of Claim 17 further comprising removing the terminal amino acid residue from a compound of Formula XXVI 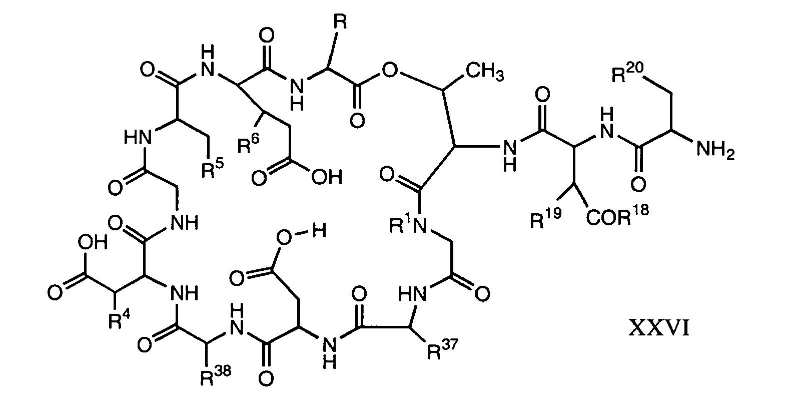 or a salt thereof;
wherein R²⁰ is carboxyamino or carboxymethyl;
to obtain the compound of Formula XXVII, or a salt thereof.

**19.** The process of Claim 18 further comprising removing the tryptophan amino acid residue from a compound of Formula XXV 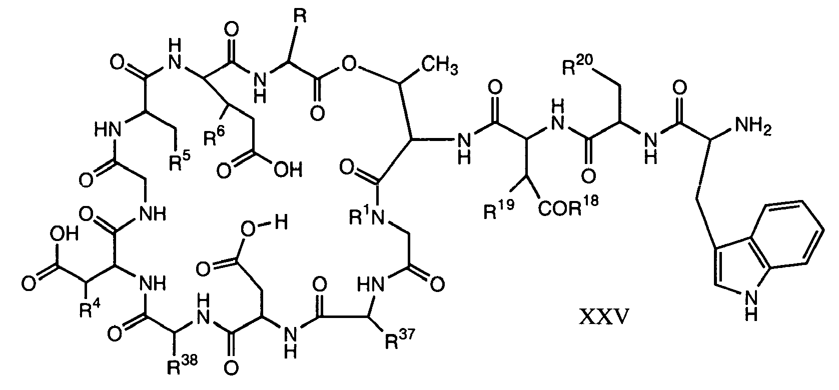 or a salt thereof,
to obtain the compound of Formula XXVI, or a salt thereof.

**20.** The process of Claim 19 further comprising deacylating a compound of Formula XXIV 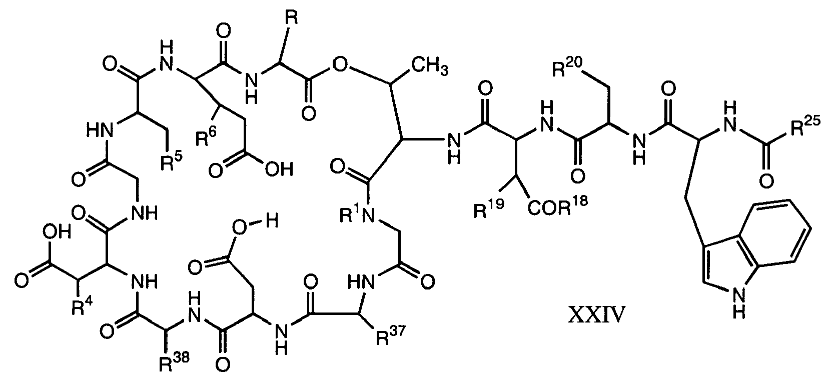 or a salt thereof;
wherein R²⁵ is an alkyl group;
to obtain the compound of Formula XXV, or a salt thereof.

**21.** The process of Claim 20 further comprising treating the free amine group(s) of a compound of Formula XI, 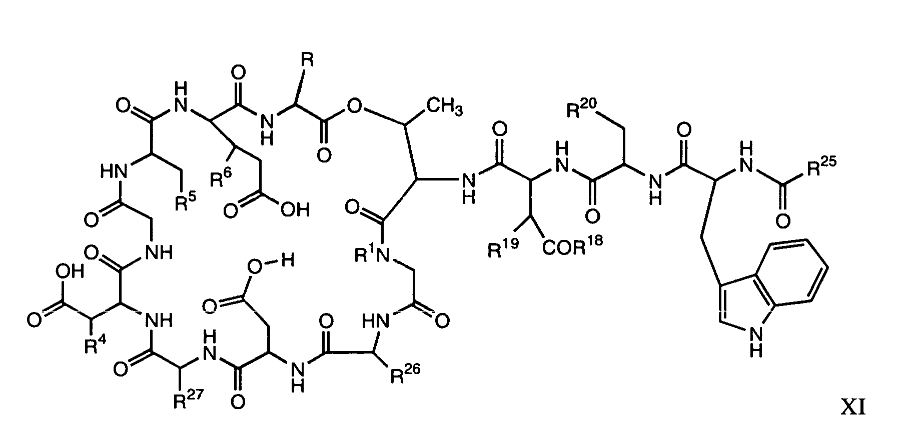 or a salt thereof;
wherein R²⁶ methyl or -CH₂CH₂CH₂NH₂; and R²⁷ is methyl or
-CH₂CH₂CH₂CH₂NH₂;
with a modifying agent to obtain the compound of Formula XXV, or a salt thereof.

**22.** A process for preparing a compound of the Formula XXXII 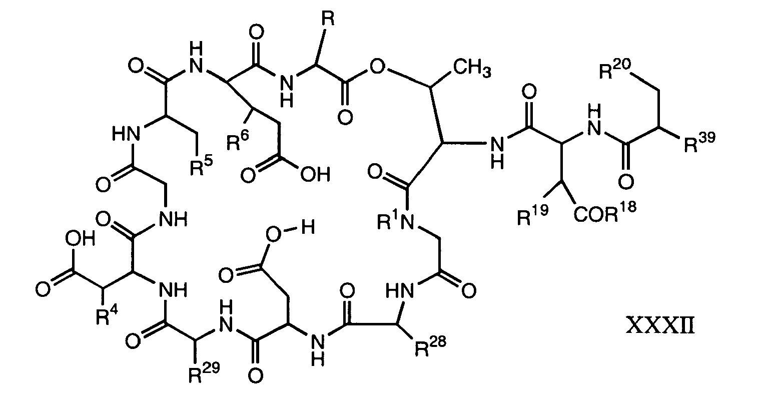 or a salt thereof; wherein:
(a) R is 2-butyl, isopropyl or 2-(2'-aminophenacyl);
(b) each of R¹ and R⁶ is independently hydrido or methyl;
(c) R⁴ is hydrido or methoxy;
(d) R⁵ is hydroxy or carboxyamino;
(e) R¹⁸ is amino or hydroxy;
(f) R¹⁹ is hydrido or hydroxy;
(g) R²⁰ is carboxyamino or carboxymethyl;
(h) R²⁸ is methyl or -CH₂CH₂CH₂NHP;
(i) R²⁹ is methyl or-CH₂CH₂CH₂CH₂NHP;
(j) P is an amino protecting group;
(k) R³⁹ is independently amino, monosubstituted amino, disubstituted amino, acylamino, ureido, guanidino, carbamoyl, sulfonamino, thioacylamino, thioureido, iminoamino, or phosphonamino ;
(l) provided that
(1) when R² is -CH₂CH₂CH₂R⁸ R⁷ is other than 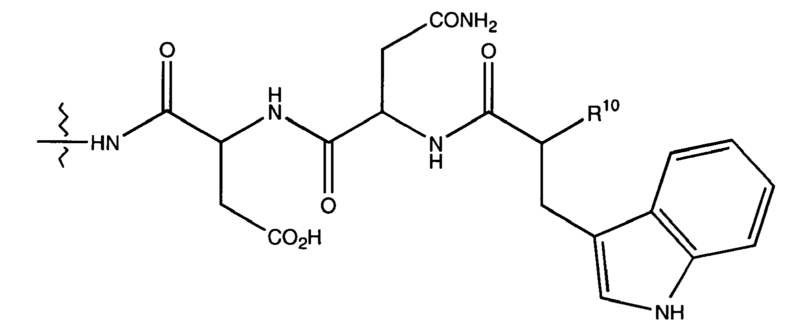 wherein R¹⁰ is amino, monosubstituted amino, disubstituted amino, acylamino, ureido, guanidino, carbamoyl, sulfonamino, thioacylamino, iminoamino, or phosphonamino;
(2) when R² is methyl, R⁷ is other than 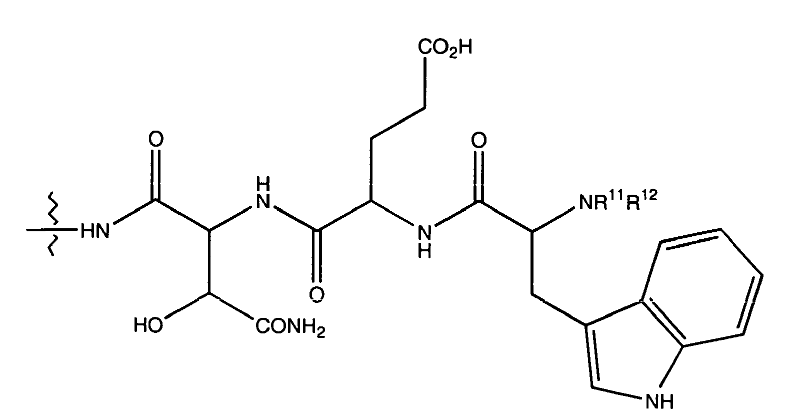 wherein each of R¹¹ and R¹² is hydrido, C₆-C₁₈ unsubstituted alkanoyl, C₈-C₁₈ unsubstituted alkenoyl, C₈-C₁₈ unsubstituted alkyl, or C₈-C₁₈ select substituted alkyl; or alternatively, R¹¹ and R¹² together are C₈-C₁₈ alkylidenyl;
comprising treating a compound of Formula XIV 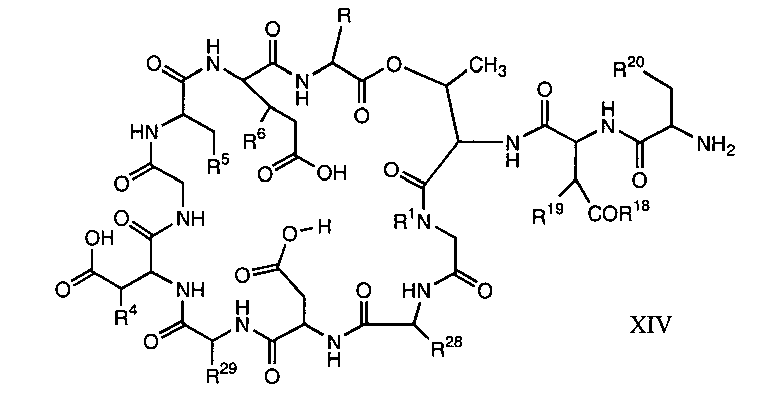 or a salt thereof,
with a modifying agent.

**23.** A process for preparing a compound of the Formula XXXIV 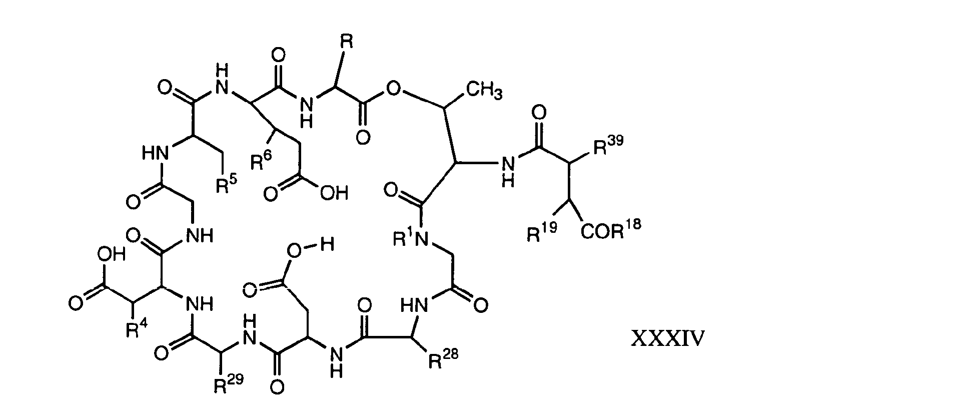 or a salt thereof; wherein:
(a) R is 2-butyl, isopropyl or 2-(2'-aminophenacyl);
(b) each of R¹ and R⁶ is independently hydrido or methyl;
(c) R⁴ is hydrido or methoxy;
(d) R⁵ is hydroxy or carboxyamino;
(e) R¹⁸ is amino or hydroxy;
(f) R¹⁹ is hydrido or hydroxy;
(g) R²⁸ is methyl or -CH₂CH₂CH₂NHP;
(h) R²⁹ is methyl or-CH₂CH₂CH₂CH₂NHP;
(i) P is an amino protecting group;
(j) R³⁹ is independently amino, monosubstituted amino, disubstituted amino, acylamino, ureido, guanidino, carbamoyl, sulfonamino, thioacylamino, thioureido, iminoamino, or phosphonamino;
(k) provided that
(1) when R² is -CH₂CH₂CH₂R⁸, R⁷ is other than 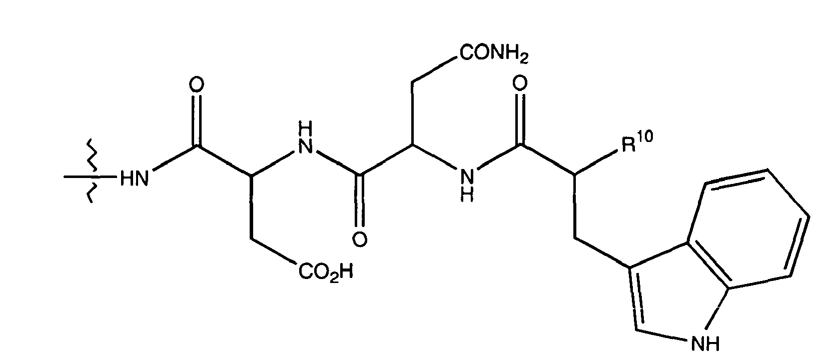 wherein R¹⁰ is amino, monosubstituted amino, disubstituted amino, acylamino, ureido, guanidino, carbamoyl, sulfonamino, thioacylamino, iminoamino, or phosphonamino;
(2) when R² is methyl, R⁷ is other than 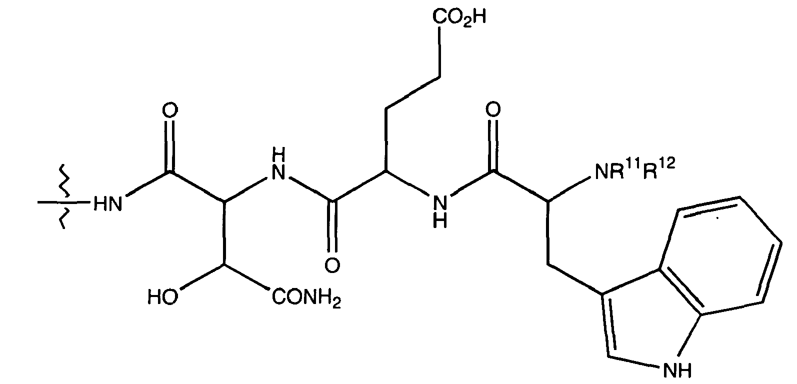 wherein each of R¹¹ and R¹² is hydrido, C₆-C₁₈ unsubstituted alkanoyl, C₈-C₁₈ unsubstituted alkenoyl, C₈-C₁₈ unsubstituted alkyl, or C₈-C₁₈ select substituted alkyl; or alternatively, R¹¹ and R¹² together are C₈-C₁₈ alkylidenyl;
comprising treating a compound of Formula XV 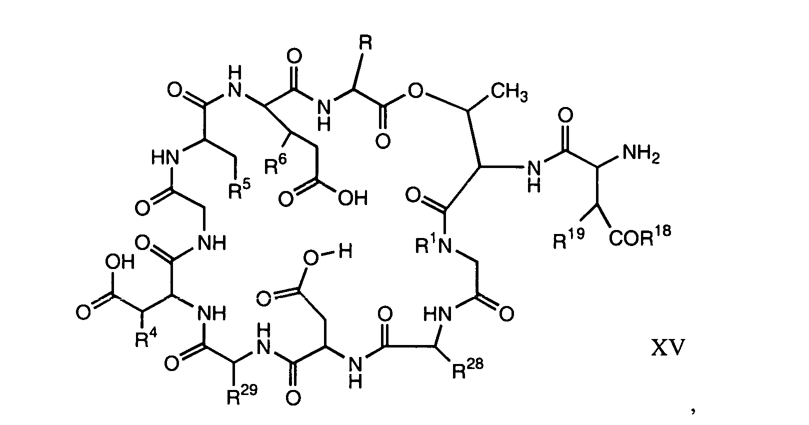 or a salt thereof,
with a modifying agent.
